(19) Europäisches Patentamt
European Patent Office
Office européen des brevets

(11) EP 4 613 358 A1

(12) EUROPEAN PATENT APPLICATION
published in accordance with Art. 153(4) EPC

(43) Date of publication:
**10.09.2025 Bulletin 2025/37**

(21) Application number: **23885871.6**

(22) Date of filing: **02.11.2023**

(51) International Patent Classification (IPC):
***B01D 53/14*** (2006.01) ***B01D 53/52*** (2006.01)
***B01D 53/62*** (2006.01) ***B01D 53/78*** (2006.01)
***C01B 32/50*** (2017.01) ***C07C 217/28*** (2006.01)
***C07D 211/58*** (2006.01) ***C07D 401/12*** (2006.01)
***C07D 405/12*** (2006.01)

(52) Cooperative Patent Classification (CPC):
**B01D 53/14; B01D 53/52; B01D 53/62; B01D 53/78; C01B 32/50; C07C 217/28; C07D 211/58; C07D 401/12; C07D 405/12;**
Y02C 20/40

(86) International application number:
**PCT/JP2023/039696**

(87) International publication number:
**WO 2024/096117 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.11.2022 JP 2022175922**
**05.01.2023 JP 2023000393**
**02.06.2023 JP 2023091519**

(71) Applicants:
- **artience Co., Ltd.**
  **Chuo-ku**
  **Tokyo 104-0031 (JP)**
- **TOYOCOLOR CO., LTD.**
  **Tokyo, 104-0031 (JP)**

(72) Inventors:
- **HAYAKAWA Junpei**
  **Tokyo 104-0031 (JP)**
- **SAKURAI Takahiro**
  **Tokyo 104-0031 (JP)**
- **TAKAI Hideaki**
  **Tokyo 104-0031 (JP)**
- **TSUCHIYA Mizuho**
  **Tokyo 104-0031 (JP)**
- **MACHIDA Kosuke**
  **Tokyo 104-0031 (JP)**

(74) Representative: **Pfenning, Meinig & Partner mbB**
**Patent- und Rechtsanwälte**
**Theresienhöhe 11a**
**80339 München (DE)**

# (54) CARBON DIOXIDE ABSORBING LIQUID, AND CARBON DIOXIDE SEPARATION/RECOVERY METHOD

(57) Provided are: a carbon dioxide absorbing/releasing liquid that has high carbon dioxide absorbing performance and high carbon dioxide releasing performance at low temperature, and that is less likely to degrade when absorbing and releasing cycles are repeated; a carbon dioxide separation/recovery method; and an amine compound production method. The present disclosure relates to an absorbing liquid for separating and recovering carbon dioxide from a gas containing carbon dioxide, the absorbing liquid comprising an amine compound (A) represented by general formula (1), and a liquid medium (B).

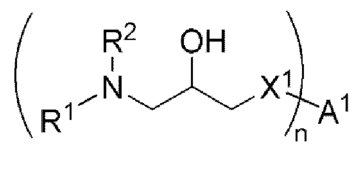

(1)

EP 4 613 358 A1

## Description

### Technical Field

The disclosure relates to an absorbing liquid for efficiently separating and recovering carbon dioxide from a gas containing carbon dioxide, and a method for separating and recovering carbon dioxide using the absorbing liquid.

### Related Art

In recent years, the rapid increase in greenhouse gas emissions, such as carbon dioxide and methane, associated with social activities has been cited as one of the causes of global warming. In particular, carbon dioxide is the most significant among greenhouse gases, and in accordance with the Paris Agreement, which came into effect in 2016, urgent measures to reduce carbon dioxide emissions are required.

As an effort to reduce carbon dioxide emissions, carbon dioxide separation and recovery has been attracting attention, and the development of carbon dioxide absorbing liquids is actively being carried out. Therefore, in recent years, the development of carbon dioxide separation and recovery technology using chemical absorption methods, primarily consisting of aqueous solutions of amine compounds, has been vigorously promoted for gas containing carbon dioxides emitted from power plants and steel mills.

As the above-mentioned amine compounds, the following are known: primary alkanolamines such as monoethanolamine (MEA), diglycolamine (DGA), 2-amino-2-methyl-1-propanol (AMP); secondary alkanolamines such as 2-(methylamino)ethanol (MAE), 2-(ethylamino)ethanol (EAE), 2-(isopropylamino)ethanol (IPAE), 3-(isopropylamino)propanol (IPAP), diethanolamine (DEA), diisopropanolamine (DIPA); tertiary alkanolamines such as N-methyldiethanolamine (MDEA), 2-(dimethylamino)ethanol (DMAE), triethanolamine (TEA); tertiary alkylamines such as N,N,N',N'-tetramethyl-1,6-diaminohexane (TMDAH), N,N,N',N'-tetramethyl-1,4-diaminobutane (TMDAB), bis(2-dimethylaminoethyl)ether (BDER); among these, MEA is widely used.

As a conventional technology for separating and recovering carbon dioxide with less energy, for example, Patent Document 1 describes a method for removing carbon dioxide from combustion exhaust gas by contacting an aqueous solution of secondary alkanolamines having steric hindrance such as alkyl groups around the amino group with combustion exhaust gas at atmospheric pressure to absorb carbon dioxide.

As one of the amine compounds included in the carbon dioxide absorbent used in a method for deacidifying gaseous effluents containing at least one acidic compound selected from the group consisting of hydrogen sulfide ($H_2S$) and carbon dioxide ($CO_2$), descriptions are provided regarding 4-amino-2,2,6,6-tetramethylpiperidine consisting of a hindered amine skeleton and specific solvents. In these documents, after absorbing acidic compounds such as carbon dioxide, a phase-separated state is formed. However, specific examples regarding 4-amino-2,2,6,6-tetramethylpiperidine or specific solvents are not shown, and there are no descriptions related to the absorption performance or release efficiency of carbon dioxide (Patent Document 2 and Patent Document 3).

In addition, reports have been made on carbon dioxide absorbing liquids consisting of 4-amino-2,2,6,6-tetramethylpiperidine, water, and sulfolane. In both cases, the only example of carbon dioxide releasing temperature for the absorbing liquid that has reacted with carbon dioxide is at 120°C, and there are no descriptions regarding the release efficiency or repetitive durability when the carbon dioxide releasing temperature is reduced to 100°C or lower (Patent Document 4 and Patent Document 5).

On the other hand, as a carbon dioxide separation material using compounds with multiple amine structures introduced in a single molecule, triazine derivatives have been reported as gas separation membranes for separating carbon dioxide from other gases. These compounds have multiple amine compounds chemically bonded to the triazine skeleton and introduced using epoxy compounds (Patent Document 6).

### Citation List

#### Patent Document

Patent Document 1: Japanese Patent Application Laid-Open Publication No. H05-301023
Patent Document 2: Japanese Patent Publication No. 2009-529420
Patent Document 3: US Patent Application Publication No. 2006/104877
Patent Document 4: Japanese Patent Application Laid-Open Publication No. 2012-223766.
Patent Document 5: Japanese Patent Application Laid-Open Publication No. 2012-516761.
Patent Document 6: Japanese Patent Application Laid-Open Publication No. 2008-247749.

SUMMARY OF INVENTION

Technical Problem

The disclosure aims to provide a carbon dioxide absorbing/releasing liquid with high carbon dioxide absorption performance, high carbon dioxide release performance at low temperatures, and minimal degradation even after repeated absorption and release cycles, as well as a method for separating and recovering carbon dioxide.

Solution to Problem

The disclosure provides the following carbon dioxide absorbing liquid and method for separating and recovering carbon dioxide.

[1] An absorbing liquid for separating and recovering carbon dioxide from a gas containing carbon dioxide, which includes:

an amine compound (A) represented by the following general formula (1) and a liquid medium (B).

[Chemical formula 1]

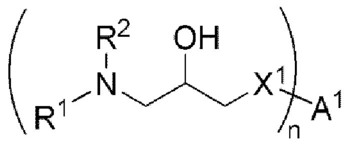

(1)

In the formula,
$R^1$ is a hydrogen atom or a hydrocarbon group which may have a substituent and may have a heteroatom in the carbon chain, and a carbon atom adjacent to N is a primary carbon atom or a carbon atom constituting a ring,
$R^2$ is a hydrogen atom, an alkyl group having 1 to 8 carbon atoms in which a carbon atom adjacent to N is a primary carbon atom, or $-CH_2CH(OH)CH_2X^2A^2$,
$X^1$ is a direct bonding, -O-, -OC(=O)-, -CO(=O)-, or $-NA^3-$,
$A^1$ is a hydrogen atom or an n-valent organic residue (excluding the case where $X^1$ is a direct bonding and $A^1$ is a hydrogen atom),
n is an integer of 1 to 6,
$X^2$ is a direct bonding, -O-, -OC(=O)-, -CO(=O)-, or $-NA^3-$,
$A^2$ is a hydrogen atom or a monovalent organic residue (excluding the case where $X^2$ is a direct bonding and $A^2$ is a hydrogen atom), and
$A^3$ is a hydrogen atom or a monovalent organic residue.

[2] The absorbing liquid according to [1], in which the $R^1$ contains a nitrogen atom.
[3] The absorbing liquid according to [1] or [2], in which the $R^2$ is a group expressed by the following general formula (2), general formula (3), general formula (4), or general formula (5).

[Chemical formula 2]

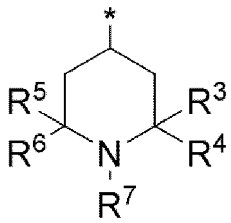
(2)

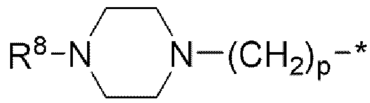
(3)

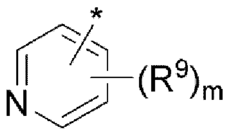
(4)

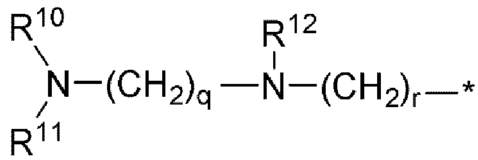
(5)

In the formula,

$R^3$, $R^4$, $R^5$, and $R^6$ are each independently a hydrogen atom or a methyl group,
$R^7$ is a hydrogen atom or a methyl group,
$R^8$ is a hydrogen atom or a methyl group,
p is an integer of 0 to 4,
$R^9$ is an alkyl group having 1 to 8 carbon atoms,
m is an integer of 0 to 4,
$R^{10}$ and $R^{12}$ are each independently a hydrogen atom, an alkyl group having 1 to 8 carbon atoms, or a hydroxyalkyl group,
$R^{11}$ is a hydrogen atom, an alkyl group having 1 to 8 carbon atoms, a hydroxyalkyl group, or - $(CH_2)_s$-$R^{13}$,
$R^{13}$is a hydroxyl group or -N($R^{14}$)$R^{15}$, and $R^{14}$ and $R^{15}$ are each independently a hydrogen atom, a methyl group, or a hydroxyalkyl group,
q is 2 or 3,
r is 2 or 3, and
s is 2 or 3.

[4] The absorbing liquid according to any one of [1] to [3], in which $A^1$ is an n-valent organic residue.
[5] The absorbing liquid according to any one of [1] to [4], in which $A^1$, $A^2$, and $A^3$ are each independently a straight-chain or branched aliphatic hydrocarbon residue, which may have a substituent and may have a heteroatom in the carbon chain; a (meth)acryloyl residue which may have a substituent; an alicyclic hydrocarbon residue which may have a substituent and may have a heteroatom in the carbon chain; an aromatic hydrocarbon residue which may have a substituent; or an aromatic heterocyclic residue which may have a substituent.
[6] The absorbing liquid according to any one of [1] to [5], in which $R^2$ is a hydrogen atom.
[7] The absorbing liquid according to any one of [1] to [6], which further includes at least one amine compound (C) selected from a group consisting of amino alcohols, cyclic polyamines, and chain polyamines.
[8] The absorbing liquid according to any one of [1] to [7], in which the liquid medium (B) has a total Hansen solubility parameter ($\delta$T) of 17 $MPa^{1/2}$ or more.
[9] The absorbing liquid according to any one of [1] to [8], in which a proportion of water in the liquid medium (B) is 50% by mass or less.
[10] The absorbing liquid according to any one of [1] to [9], which includes 5% by mass or more of the amine compound (A) represented by the formula (1).
[11] The absorbing liquid according to any one of [1] to [10], in which the gas further includes hydrogen sulfide, and the absorbing liquid absorbs the hydrogen sulfide.
[12] A method for separating and recovering carbon dioxide from a gas containing carbon dioxide, which includes:

Process A: bringing the absorbing liquid according to any one of [1] to [11] into contact with a gas containing carbon dioxide to obtain an absorbing liquid that has absorbed carbon dioxide from the gas containing carbon dioxide, and
Process B: heating the absorbing liquid that has absorbed carbon dioxide obtained in Process A to desorb and release carbon dioxide from the absorbing liquid and recovering the released carbon dioxide.

[13] The method according to [12], in which a heating temperature in the Process B is 50°C or higher and 160°C or lower.

Effects of Invention

According to the disclosure, the absorbing liquid has a high carbon dioxide recovery amount and absorption rate, possesses the ability to release carbon dioxide with low energy, and the degradation of the material due to repeated carbon dioxide recovery and release is suppressed, enabling carbon dioxide separation and recovery with low energy for the entire system. Furthermore, by improving the absorption efficiency, it becomes possible to design a more compact carbon dioxide separation and recovery facility, thereby reducing initial costs.

DESCRIPTION OF EMBODIMENTS

The following describes the absorbing liquid and the method for separating and recovering carbon dioxide.
Further, in the disclosure, "to" indicating a numerical range includes the values described before and after it as the lower limit value and upper limit value, unless otherwise specified.
Further, in cases where multiple identical symbols appear in a chemical formula, unless otherwise specified, these identical symbols are not limited to representing the same substituent, and may represent different substituents within the range defined for that symbol.

[Absorbing liquid]

The absorbing liquid of the disclosure is an absorbing liquid for separating and recovering carbon dioxide from a gas containing carbon dioxide, and includes an amine compound (A) represented by the following general formula (1) and a liquid medium (B).

[Chemical formula 3]

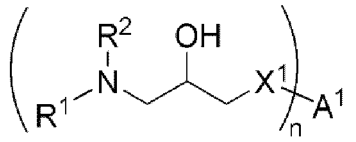

(1)

In the formula,

$R^1$ is a hydrogen atom or a hydrocarbon group which may have a substituent and may have a heteroatom in the carbon chain, and a carbon atom adjacent to N is a primary carbon atom or a carbon atom constituting a ring,
$R^2$ is a hydrogen atom, an alkyl group having 1 to 8 carbon atoms in which a carbon atom adjacent to N is a primary carbon atom, or $-CH_2CH(OH)CH_2X^2A^2$,
$X^1$ is a direct bonding, -O-, -OC(=O)-, -CO(=O)-, or $-NA^3-$,
$A^1$ is a hydrogen atom or an n-valent organic residue (excluding the case where $X^1$ is a direct bonding and $A^1$ is a hydrogen atom),
n is an integer of 1 to 6,
$X^2$ is a direct bonding, -O-, -OC(=O)-, -CO(=O)-, or $-NA^3-$,
$A^2$ is a hydrogen atom or a monovalent organic residue (excluding the case where $X^2$ is a direct bonding and $A^2$ is a hydrogen atom), and
$A^3$ is a hydrogen atom or a monovalent organic residue.

The present inventors have found that by using the specific amine compound (A) described above, a high carbon dioxide recovery amount may be achieved, and the energy consumption required for the carbon dioxide recovery amount may be kept low. The absorbing liquid of the disclosure using the specific amine compound (A) not only efficiently absorbs carbon dioxide and releases it with low energy, enabling high-efficiency recovery of high-purity carbon dioxide, but also is resistant to degradation even when absorption and release are repeated.

<Amine compound (A)>

The amine compound (A) is a compound represented by the following general formula (1).

[Chemical formula 4]

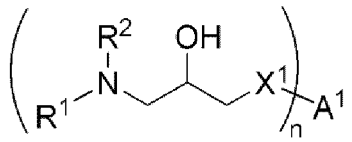

(1)

$R^1$ is a hydrogen atom or a hydrocarbon group which may have a substituent and may have a heteroatom in the carbon chain, and a carbon atom adjacent to N is a primary carbon atom or a carbon atom constituting a ring, Here, N represents the N explicitly shown in formula (1).

As the hydrocarbon group in which the carbon atom adjacent to N is a primary carbon atom, a straight-chain alkyl group may be mentioned. Further, as the hydrocarbon group in which the carbon atom adjacent to N is a carbon atom constituting a ring, a cycloalkyl group and an aryl group may be mentioned.

As the heteroatom that may be present in the carbon chain, O, N, S, Si, etc. may be mentioned, with O or N being preferred. The carbon chain may have two or more heteroatoms.

Furthermore, as the "substituent" that $R^1$ may have, halogen atoms, straight-chain or branched alkyl groups, cycloalkyl groups, alkoxy groups, cyano groups, trifluoromethyl groups, nitro groups, hydroxyl groups, carbamoyl groups, N-substituted carbamoyl groups, sulfamoyl groups, N-substituted sulfamoyl groups, carboxyl groups, sulfo groups, amino groups, imino groups, phenyl groups, sulfanyl groups, etc. may be mentioned, the aforementioned substituents may further have substituents, and such substituents include those mentioned above.

Among these, $R^1$ is preferably a hydrogen atom, an alkyl group which may have a substituent and may have a heteroatom in the carbon chain, a heterocyclic group which may have a substituent, or a cycloalkyl group which may have a substituent.

The alkyl group which may have a substituent and may have a heteroatom in the carbon chain in $R^1$ includes, for example, methyl group, ethyl group, propyl group, butyl group, isobutyl group, neopentyl group, n-hexyl group, n-octyl group, stearyl group, 2-ethylhexyl group, trichloromethyl group, trifluoromethyl group, 2,2,2-trifluoroethyl group, 2,2-dibromoethyl group, 2,2,3,3-tetrafluoropropyl group, 2-ethoxyethyl group, 2-butoxyethyl group, 2-nitropropyl group, benzyl group, 4-methylbenzyl group, 4-tert-butylbenzyl group, 4-methoxybenzyl group, 4-nitrobenzyl group, 2,4-dichlorobenzyl group, methylsulfanyl group, ethylsulfanyl group, propylsulfanyl group, butylsulfanyl group, pentylsulfanyl group, hexylsulfanyl group, octylsulfanyl group, decylsulfanyl group, dodecylsulfanyl group, octadecylsulfanyl group, methoxyethylsulfanyl group, aminoethylsulfanyl group, benzylaminoethylsulfanyl group, methylcarbonylaminoethylsulfanyl group, phenylcarbonylaminoethylsulfanyl group, sulfanylmethyl group, 2-sulfanylethyl group, 1-sulfanylethyl group, aminomethyl group, aminoethyl group, N-methylaminoethyl group, N-dimethylaminoethyl group, N-ethylaminoethyl group, N-(aminoethyl)aminoethyl group, N-(hydroxyethyl)aminoethyl group, N-propylaminoethyl group, N-isopropylaminoethyl group, N-butylaminoethyl group, aminopropyl group, N-methylaminopropyl group, N-ethylaminopropyl group, N-propylaminopropyl group, N-(aminopropyl)aminopropyl group, N-isopropylaminopropyl group, N-butylaminopropyl group, dibutylaminopropyl group, dimethylaminopropyl group, diethylaminopropyl group, aminobutyl group, aminopentyl group, aminohexyl group, aminooctyl group, aminodecyl group, aminododecyl group, aminooctadecyl group, aminoethoxymethyl group, aminoethoxyethyl group, aminoethylaminoethyl group, aminoethylaminomethylphenyl group, aminoethylaminocarbonylmethyl group, aminoethylaminocarbonylphenyl group, hydroxymethyl group, 2-hydroxyethyl group, N-(hydroxyethyl)aminoethyl group, 2-hydroxypropyl group, 3-hydroxypropyl group, 4-hydroxybutyl group, cyclopropylmethyl group, cyclobutylmethyl group, cyclopentylmethyl group, cyclohexylmethyl group, cyclopentylethyl group, cyclohexylethyl group, cyclopentylpropyl group, cyclohexylpropyl group, 2-(1-piperazinyl)ethyl group, and the like. Preferred examples of the alkyl group having a substituent include aminoethyl group, N-methylaminoethyl group, N-ethylaminoethyl group, N-(aminoethyl)aminoethyl group, N-propylaminoethyl group, N-isopropylaminoethyl group, N-butylaminoethyl group, aminopropyl group, N-methylaminopropyl group, N-ethylaminopropyl group, N-propylaminopropyl group, N-(aminopropyl)aminopropyl group, N-isopropylaminopropyl group, N-butylaminopropyl group, aminobutyl group, and 2-(1-piperazinyl)ethyl group, and more preferred examples include aminoethyl group, N-methylaminoethyl group, N-ethylaminoethyl group, and N-(aminoethyl)aminoethyl group.

The heterocyclic group which may have a substituent includes, for example, 2-pyridyl group, 3-pyridyl group, 4-pyridyl group, 2-pyrrolyl group, 3-pyrrolyl group, 2-furyl group, 3-furyl group, 2-thienyl group, 3-thienyl group, 2-imidazolyl group, 2-oxazolyl group, 2-thiazolyl group, piperidino group, 4-piperidyl group, morpholino group, 2-morpholinyl group, N-indolyl group, 2-indolyl group, 2-benzofuryl group, 2-benzothienyl group, 2-quinolino group, N-carbazolyl group, piperidinyl group, and the like.

The cycloalkyl group which may have a substituent includes, for example, cyclopropyl group, methylcyclopropyl group, cyclobutyl group, cyclopentyl group, methylcyclopentyl group, ethylcyclopentyl group, cyclohexyl group, methylcyclohexyl group, ethylcyclohexyl group, propylcyclohexyl group, 4,4'-methylenebis(cyclohexyl) group, and the like.

From the viewpoint of balancing carbon dioxide adsorption performance and carbon dioxide release performance at low temperatures, $R^1$ preferably includes a nitrogen atom, and more preferably, it is a group represented by the following general formula (2), general formula (3), general formula (4), or general formula (5). In the formulas, * represents a bond with N in formula (1).

[Chemical formula 5]

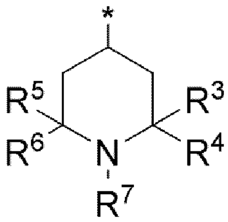

(2)

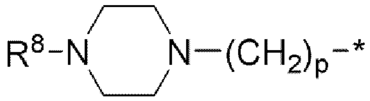

(3)

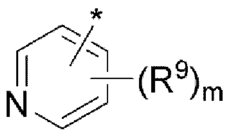

(4)

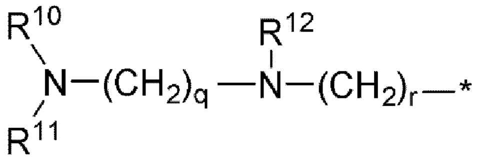

(5)

In formula (2), $R^3$, $R^4$, $R^5$, and $R^6$ are each independently a hydrogen atom or a methyl group, and $R^7$ is a hydrogen atom or a methyl group. From the viewpoint of carbon dioxide adsorption performance and low-temperature release performance, $R^3$ to $R^6$ are preferably methyl groups. Also, from the viewpoint of carbon dioxide adsorption performance and low-temperature release performance, $R^7$ is preferably a hydrogen atom.

In formula (3), $R^8$ is a hydrogen atom or a methyl group, and p is an integer from 0 to 4. From the viewpoint of carbon dioxide adsorption performance and low-temperature release performance, $R^8$ is preferably a hydrogen atom. Also, from the viewpoint of carbon dioxide adsorption performance, low-temperature release performance, and ease of synthesis, p is preferably 1 to 4, and more preferably 2 to 3.

In formula (4), $R^9$ is an alkyl group having 1 to 8 carbon atoms, and m is an integer from 0 to 4.

The alkyl group in $R^9$ includes, for example, methyl group, ethyl group, n-propyl group, isopropyl group, n-butyl group, tert-butyl group, hexyl group, octyl group, and the like. From the viewpoint of carbon dioxide adsorption performance and low-temperature release performance, $R^9$ is preferably an alkyl group having 1 to 4 carbon atoms, and among them, methyl group is particularly preferred. Also, m represents the number of substitutions of $R^9$, and from the viewpoint of carbon dioxide adsorption performance and low-temperature release performance, it is preferably 0 to 2, and more preferably 0 to 1.

In formula (5), $R^{10}$ and $R^{12}$ are each independently a hydrogen atom, an alkyl group having 1 to 8 carbon atoms, or a hydroxyalkyl group, $R^{11}$ is a hydrogen atom, an alkyl group having 1 to 8 carbon atoms, a hydroxyalkyl group, or $-(CH_2)_s-R^{13}$, $R^{13}$ is a hydroxyl group or $-N(R^{14})R^{15}$, $R^{14}$ and $R^{15}$ are each independently a hydrogen atom, a methyl group, or a hydroxyalkyl group, q is 2 or 3, r is 2 or 3, s is 2 or 3, and from the viewpoint of carbon dioxide adsorption performance and low-temperature release performance, q is preferably 3 and r is preferably 3. The alkyl groups having 1 to 8 carbon atoms in $R^{10}$ to $R^{12}$ include those similar to the aforementioned $R^9$, and from the viewpoint of carbon dioxide adsorption performance and low-temperature release performance, $R^{10}$ to $R^{12}$ are preferably alkyl groups having 1 to 4 carbon atoms, and methyl group or ethyl group is preferred.

In the hydroxyalkyl groups of $R^{10}$ to $R^{12}$, $R^{14}$, and $R^{15}$, the number of carbon atoms in the alkyl group is preferably 1 to 8, and more preferably 1 to 4, from the viewpoint of carbon dioxide adsorption performance and low-temperature release performance. Specific examples of the hydroxyalkyl group include hydroxymethyl group, hydroxyethyl group, hydroxypropyl group, hydroxybutyl group, and the like.

From the viewpoint of carbon dioxide adsorption performance and low-temperature release performance, among these, $R^1$ is preferably a group represented by the general formula (5).

Specific examples of $R^1$ are shown by the specific examples of the amine compound (A) described later.

$R^2$ is a hydrogen atom, an alkyl group having 1 to 8 carbon atoms in which a carbon atom adjacent to N is a primary carbon atom, or $-CH_2CH(OH)CH_2X^2A^2$.

Examples of the alkyl group having 1 to 8 carbon atoms in $R^2$ include methyl group, ethyl group, n-propyl group, isopropyl group, n-butyl group, sec-butyl group, isobutyl group, neopentyl group, isopentyl group, sec-pentyl group, 1-hexyl group, 2-hexyl group, heptyl group, methylcyclohexyl group, octyl group, 2-ethylhexyl group, and the like.

In the case where $R^2$ is $-CH_2CH(OH)CH_2X^2A^2$, n in formula (1) is preferably 1. In this case, formula (1) is represented by $A^2X^2CH_2CH(OH)CH_2N(R^1)CH_2CH(OH)CH_2X^2A^1$: formula (1a). When the amine compound (A) is represented by formula (1a), from the viewpoint of carbon dioxide adsorption performance, low-temperature release performance, and ease of synthesis, $X^1A^1$ and $X^2A^2$ are preferably the same substituent.

From the viewpoint of carbon dioxide adsorption performance, low-temperature release performance, and ease of synthesis, among these, $R^2$ is preferably a hydrogen atom or an alkyl group having 1 to 4 carbon atoms, and more preferably a hydrogen atom.

$X^1$ and $X^2$ are, independently of each other, a direct bonding, -O-, -OC(=O)-, -CO(=O)-, or $-NA^3-$. From the viewpoint of carbon dioxide adsorption performance, low-temperature release performance, and suppression of degradation of the absorbing liquid during repeated use, $X^1$ and $X^2$ are, independently of each other, preferably a direct bonding or -O-, and more preferably -O-.

In addition, in the case where there are multiple $X^1$ (i.e., when n is 2 or more), the multiple $X^1$ may be the same or different, but from the viewpoint of carbon dioxide adsorption performance, low-temperature release performance, and ease of synthesis, it is preferable that the multiple $X^1$ are the same.

$A^1$ is a hydrogen atom or an n-valent organic residue. However, in the case where $X^1$ is a direct bonding, $A^1$ is an n-valent organic residue. The n-valent organic residue refers to a residue obtained by removing n hydrogen atoms from an organic group. In the specific examples described later, the names of monovalent substituents are used, but $A^1$ is further a residue obtained by removing any n-1 hydrogen atoms.

Examples of the n-valent organic residue in $A^1$ include: straight-chain or branched aliphatic hydrocarbon residues which may have substituents and may have heteroatoms in the carbon chain; (meth)acryloyl residues which may have substituents; alicyclic hydrocarbon residues which may have substituents and may have heteroatoms in the carbon chain; aromatic hydrocarbon residues which may have substituents; and aromatic heterocyclic residues which may have substituents. Examples of the heteroatoms include O, N, S, and Si. As the heteroatom in the aliphatic hydrocarbon residue, O or N is preferable, and O is more preferable. As the heteroatom in the alicyclic hydrocarbon residue, O or N is preferable, and N is more preferable. The organic residue may have two or more heteroatoms. Examples of the straight-chain or branched aliphatic hydrocarbon residues having heteroatoms include straight-chain or branched polyoxyalkyl residues.

In addition, n represents an integer of 1 to 6, preferably 1 to 4, and more preferably 1 to 2.

Examples of the n-valent aliphatic hydrocarbon residues which may have substituent include alkyl groups, alkenyl groups, and alkynyl groups.

Specific examples of alkyl groups include alkyl groups having 1 to 18 carbon atoms such as methyl group, ethyl group, propyl group, isopropyl group, butyl group, isobutyl group, sec-butyl group, tert-butyl group, pentyl group, isopentyl group, hexyl group, heptyl group, octyl group, 2-ethylhexyl group, nonyl group, decyl group, dodecyl group, tetradecyl group, pentadecyl group, and octadecyl group.

Examples of alkenyl groups include alkenyl groups having 2 to 18 carbon atoms such as vinyl group, 1-propenyl group, 2-propenyl group, isopropenyl group, 1-butenyl group, 2-butenyl group, 3-butenyl group, 1-octenyl group, 1-decenyl group, and 1-octadecenyl group.

Examples of alkynyl groups include alkynyl groups having 2 to 18 carbon atoms such as ethynyl group, 1-propynyl group, 2-propynyl group, 1-butynyl group, 2-butynyl group, 3-butynyl group, 1-octynyl group, 1-decynyl group, and 1-octadecynyl group.

Examples of substituents in the n-valent straight-chain or branched hydrocarbon residues which may have substituents include straight-chain or branched alkyl groups, alkoxy groups, polyoxyalkyl groups, phenyl groups, 4-nitrophenyl groups, 2-methoxyphenyl groups, hydroxyl groups, halogen atoms, and epoxy groups, the aforementioned substituents may further have substituents, and such substituents include those mentioned above.

Specific alkyl groups as substituents are synonymous with the alkyl groups of the n-valent straight-chain or branched hydrocarbon residues which may have substituents mentioned above.

Specific examples of alkoxy groups as substituents include methoxy groups and ethoxy groups.

Specific examples of polyoxyalkyl groups as substituents include ethylene oxide groups with a number of repetition of 4 to 16, and straight-chain or branched propylene oxide groups with a number of repetition of 4 to 16.

Specific examples of halogen atoms as substituents include chlorine atoms, bromine atoms, and iodine atoms.

Examples of n-valent straight-chain or branched polyoxyalkyl groups which may have substituents include ethylene oxide groups with a number of repetition of 4 to 16, and straight-chain or branched propylene oxide groups with a number of repetition of 4 to 16. The substituents are similar to those mentioned above for the n-valent straight-chain or branched hydrocarbon residues which may have substituents, with alkyl groups, phenyl groups, and hydroxyl groups being preferred.

Examples of n-valent (meth)acryloyl residues which may have substituents include methacryl groups and acryloyl groups as (meth)acryloyl groups. The substituents are similar to those mentioned above for the n-valent straight-chain or branched hydrocarbon residues which may have substituents.

Examples of alicyclic hydrocarbon groups in n-valent alicyclic hydrocarbon residues which may have substituents include cycloalkyl groups, specifically cycloalkyl groups with 3 to 18 carbon atoms such as cyclopropyl groups, cyclobutyl groups, cyclopentyl groups, cyclohexyl groups, cycloheptyl groups, cyclooctyl groups, cyclooctadecyl groups, and 2-indeno groups. Further, the alicyclic hydrocarbon groups also include groups in which multiple cycloalkyl groups are linked by groups such as alkylene groups. The substituents in the n-valent alicyclic hydrocarbon residues which may have substituents are similar to those mentioned above for the n-valent straight-chain or branched hydrocarbon residues which may have substituents, with branched alkylene groups being preferred, and tert-butylene groups being particularly preferred.

Examples of aromatic hydrocarbons in n-valent aromatic hydrocarbon residues which may have substituents include aromatic hydrocarbons with a condensed number of 1 to 4, specifically, benzene, biphenyl, naphthalene, anthracene, phenanthrene, tetracene, pyrene, 9,9-diphenyl fluorene, bis(3-methylphenyl)fluorene, and binaphthyl.

The substituents in the n-valent aromatic hydrocarbon residues which may have substituents are similar to those mentioned above for the n-valent straight-chain or branched hydrocarbon residues which may have substituents. Preferred substituents include alkyl groups, alkylene groups, and halogen atoms, with methyl groups, methylene groups, tert-butylene groups, and bromine atoms being particularly preferred.

Examples of aromatic heterocycles in n-valent aromatic heterocyclic residues which may have substituents include aromatic heterocycles with a condensed number of 1 to 4, such as pyrrole, imidazole, pyridine, triazine, indole, quinoline, carbazole, and phthalimide. The substituents are similar to those mentioned above for the n-valent straight-chain or branched hydrocarbon residues which may have substituents.

The structure of $A^1(X^1)_n$ may also be considered as an n-valent straight-chain or branched alkoxy residue which may have substituents, an n-valent alkyl ester residue which may have substituents, an n-valent aromatic ester residue which may have substituents, or an n-valent amino residue which may have substituents.

Examples of alkoxy groups in n-valent straight-chain or branched alkoxy residues which may have substituents include methoxy groups and ethoxy groups. The substituents are similar to those mentioned above for the n-valent straight-chain or branched hydrocarbon residues which may have substituents.

Examples of alkyl ester groups in n-valent alkyl ester residues which may have substituents include methyl ester groups, ethyl ester groups, propyl ester groups, butyl ester groups, pentyl ester groups, heptyl ester groups, hexyl ester groups, octyl ester groups, hexadecyl ester groups, cyclohexyl ester groups, 1,2-cyclohexane diester groups, and 1,2-cyclohexene diester groups. The substituents are similar to those mentioned above for the n-valent straight-chain or branched hydrocarbon residues which may have substituents.

Examples of aromatic ester groups in n-valent aromatic ester residues which may have substituents include phenyl ester groups and 4-tert-butylphenyl ester groups. The substituents are similar to those mentioned above for the n-valent straight-chain or branched hydrocarbon residues which may have substituents.

An example of an amino group in n-valent amino residues which may have substituents includes an aniline group. The substituents are similar to those mentioned above for the n-valent straight-chain or branched hydrocarbon residues which may have substituents. Preferred substituents include alkyl groups, with methyl groups being more preferred.

$A^1$ is preferably an n-valent straight-chain or branched hydrocarbon residue which may have substituents, or an n-valent aromatic hydrocarbon residue which may have substituents, more preferably an n-valent straight-chain or branched hydrocarbon residue which may have substituents, and particularly preferably an n-valent straight-chain hydrocarbon residue.

The number of carbon atoms in $A^1$ is preferably 1 to 15, more preferably 1 to 10, and even more preferably 2 to 8.

$A^2$ and $A^3$ are each independently a hydrogen atom or a monovalent organic residue. However, in the case where $X^2$ is a direct bonding, $A^2$ is a monovalent organic residue.

The monovalent organic residues in $A^2$ and $A^3$ are similar to those described for the n-valent organic residues in $A^1$ above, with n being replaced by 1.

The following Table 1-1 to Table 1-8 show representative examples of amine compounds (A), compounds (A1) to (A128). However, this embodiment is not limited to these representative examples.

[Table 1-1]

Table 1-1

| Compound | Chemical Structure | Compound | Chemical Structure |
|---|---|---|---|
| (A1) | 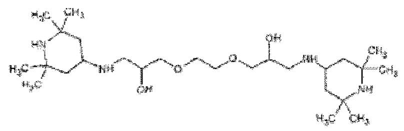 | (A2) | 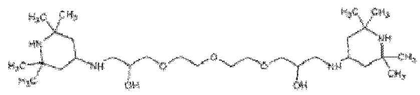 |
| (A3) | 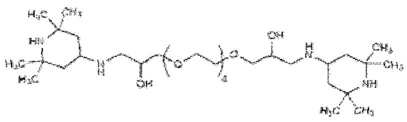 | (A4) | 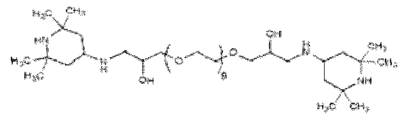 |
| (A5) | 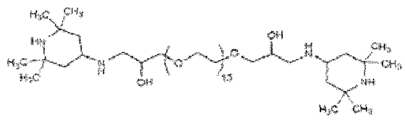 | (A6) | 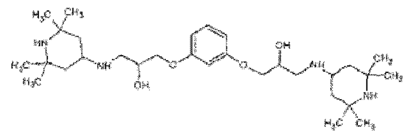 |
| (A7) | 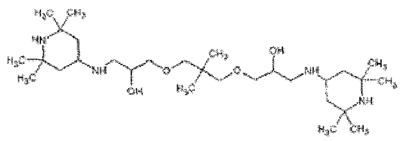 | (A8) | 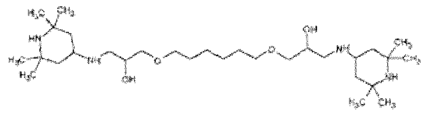 |
| (A9) | 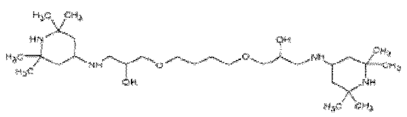 | (A10) | 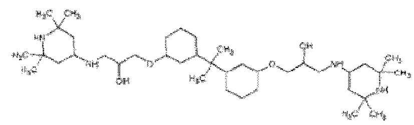 |
| (A11) | 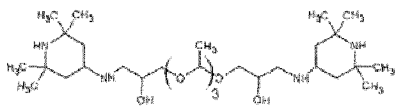 | (A12) | 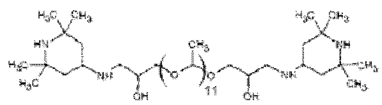 |
| (A13) | 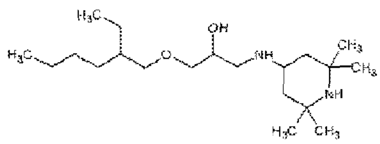 | (A14) | 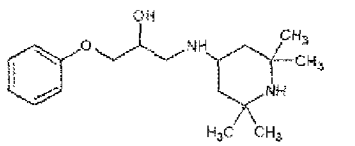 |
| (A15) | 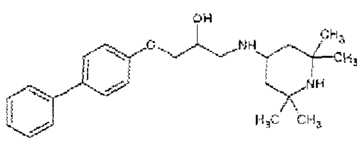 | (A16) | 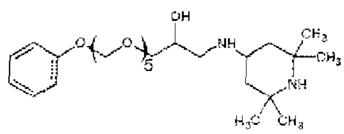 |

[Table 1-2]

Table 1-2

| Compound | Chemical Structure | Compound | Chemical Structure |
|---|---|---|---|
| (A17) | 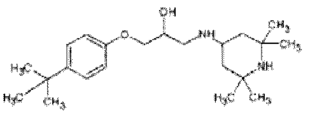 | (A18) | 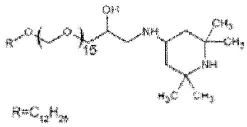 $R=C_{12}H_{25}$ |
| (A19) | 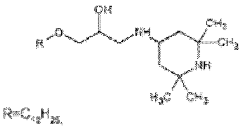 $R=C_{12}H_{25}$, | (A20) | 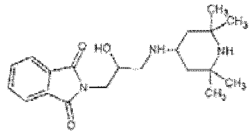 |
| (A21) | 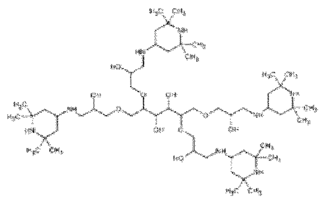 | (A22) | 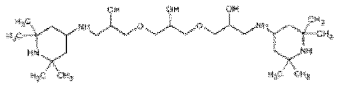 |
| (A23) | 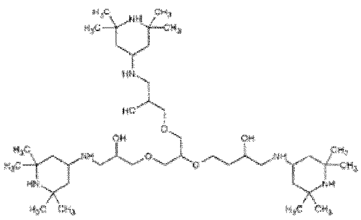 | (A24) | 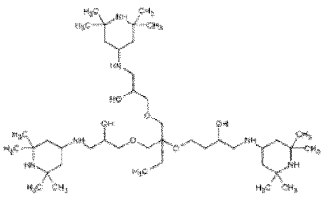 |
| (A25) | 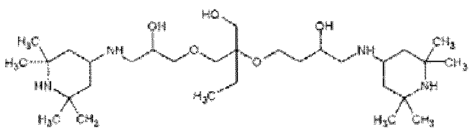 | (A26) | 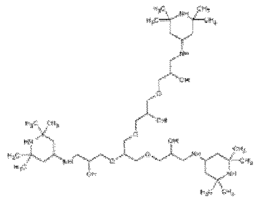 |
| (A27) | 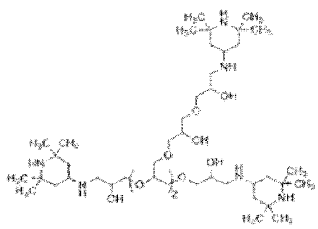 | (A28) | 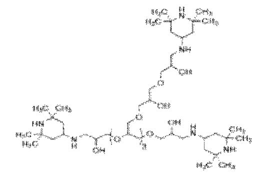 |
| (A29) | 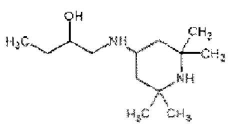 | (A30) | 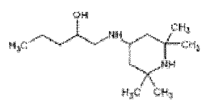 |
| (A31) | 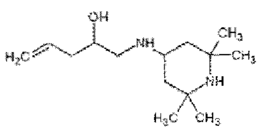 | (A32) | 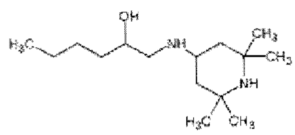 |

[Table 1-3]

Table 1-3

| Compound | Chemical Structure | Compound | Chemical Structure |
|---|---|---|---|
| (A33) | 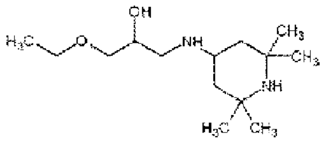 | (A34) | 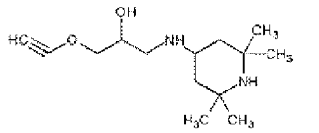 |
| (A35) | 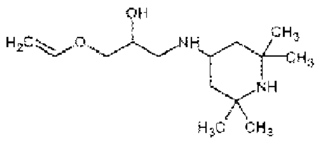 | (A36) | 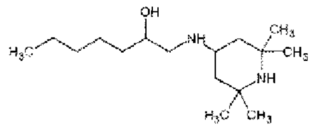 |
| (A37) | 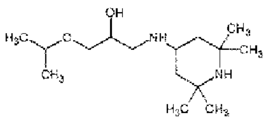 | (A38) | 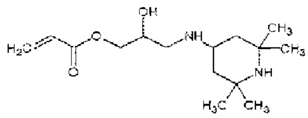 |
| (A39) | 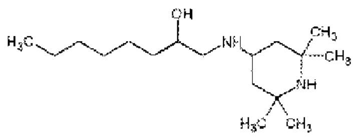 | (A40) | 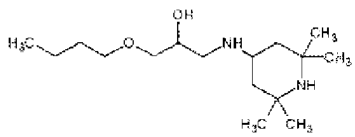 |
| (A41) | 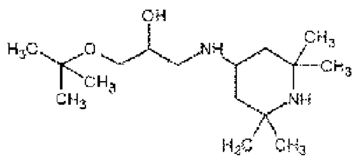 | (A42) | 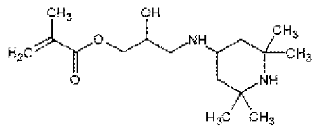 |
| (A43) | 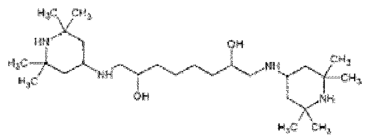 | (A44) | 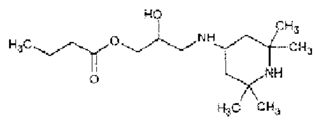 |
| (A45) | 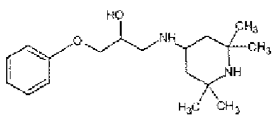 | (A46) | 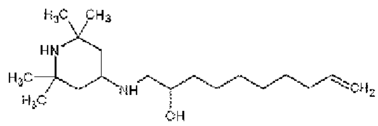 |
| (A47) | 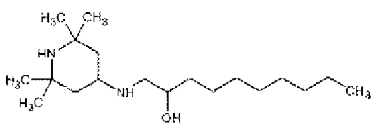 | (A48) | 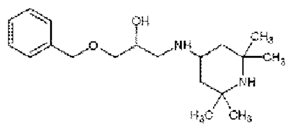 |

[Table 1-4]

Table 1-4

| Compound | Chemical Structure | Compound | Chemical Structure |
|---|---|---|---|
| (A49) | 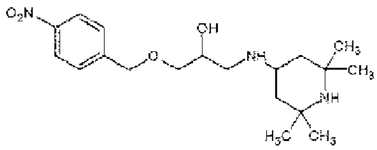 | (A50) | 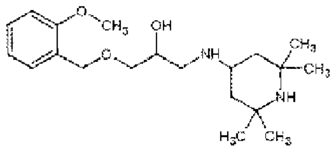 |
| (A51) | 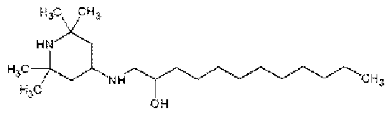 | (A52) | 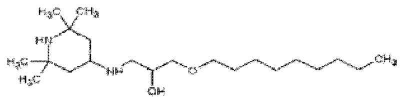 |
| (A53) | 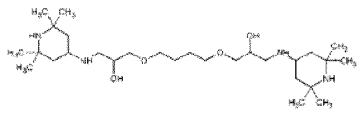 | (A54) | 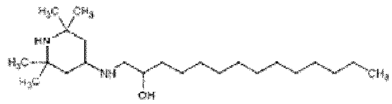 |
| (A55) | 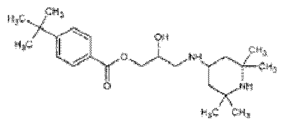 | (A56) | 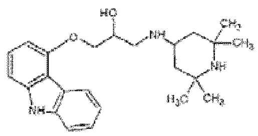 |
| (A57) | 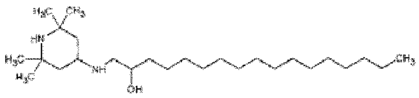 | (A58) | 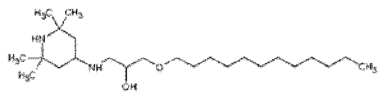 |
| (A59) | 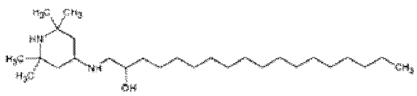 | (A60) | 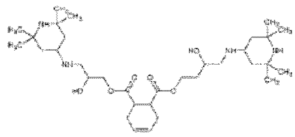 |
| (A61) | 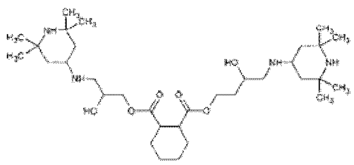 | (A62) | 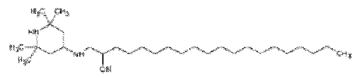 |
| (A63) | 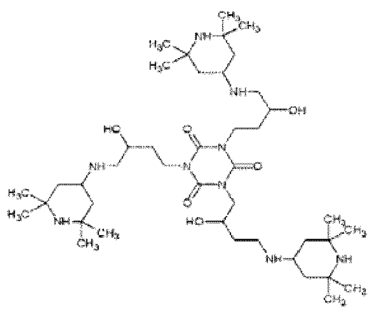 | (A64) | 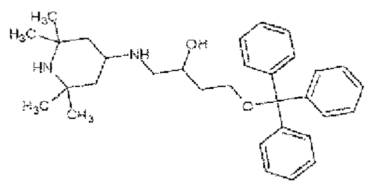 |

[Table 1-5]

Table 1-5

| Compound | Chemical Structure | Compound | Chemical Structure |
|---|---|---|---|
| (A65) | 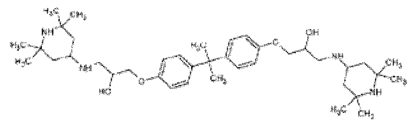 | (A66) | 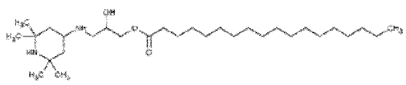 |
| (A67) | 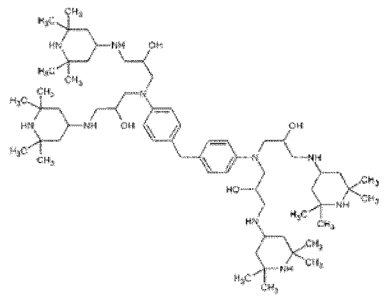 | (A68) | 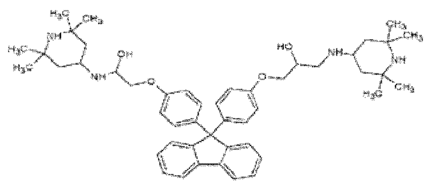 |
| (A69) | 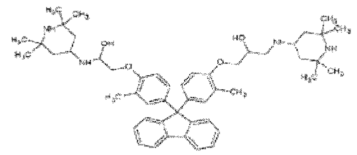 | (A70) | 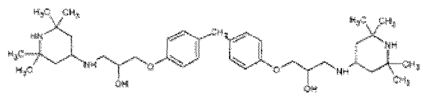 |
| (A71) | 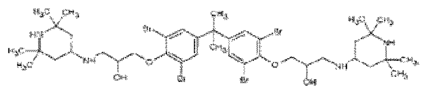 | (A72) | 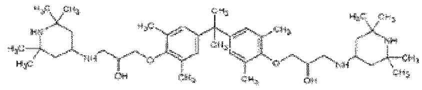 |
| (A73) | 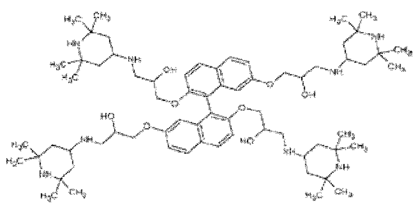 | (A74) | 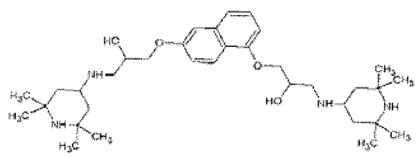 |
| (A75) | 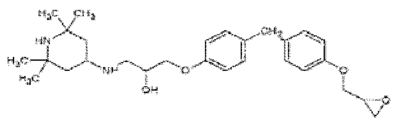 | (A76) | 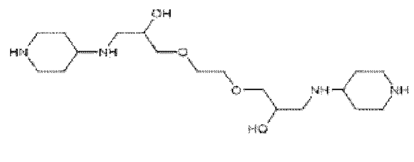 |
| (A77) | 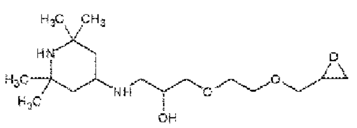 | (A78) | 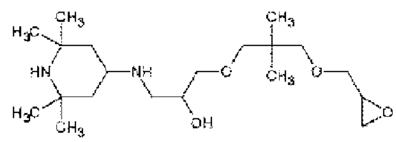 |

[Table 1-6]

Table 1-6

| Compound | Chemical Structure | Compound | Chemical Structure |
|---|---|---|---|
| (A79) | 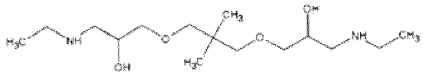 | (A80) | 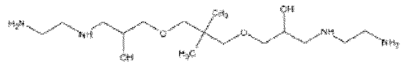 |
| (A81) | 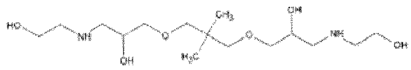 | (A82) | 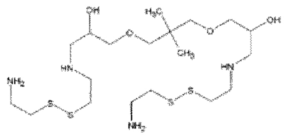 |
| (A83) | 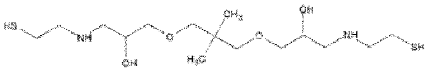 | (A84) | 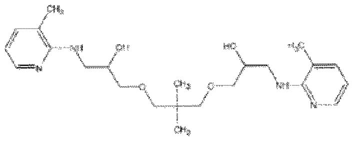 |
| (A85) | 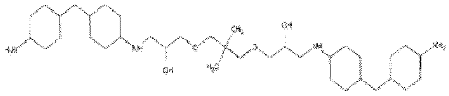 | (A86) | 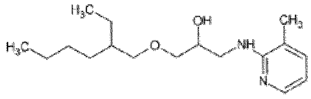 |
| (A87) | 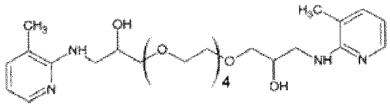 | (A88) | 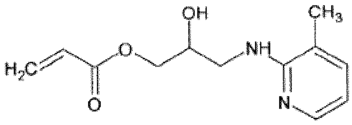 |
| (A89) | 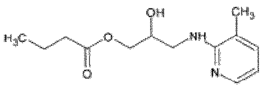 | (A90) | 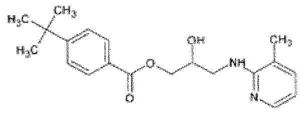 |
| (A91) | 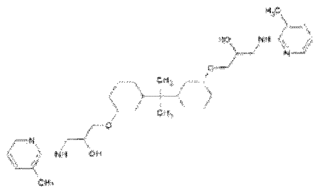 | (A92) | 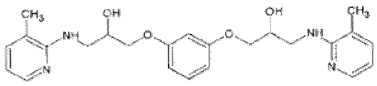 |
| (A93) | 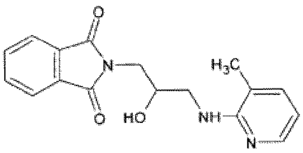 | (A94) | 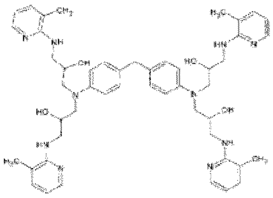 |

[Table 1-7]

Table 1-7

| Compound | Chemical Structure | Compound | Chemical Structure |
|---|---|---|---|
| (A95) | 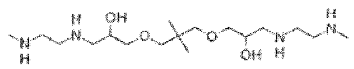 | (A96) | 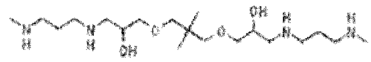 |
| (A97) | 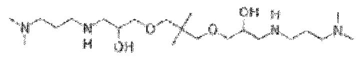 | (A98) | 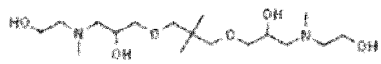 |
| (A99) | 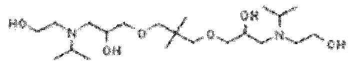 | (A100) | 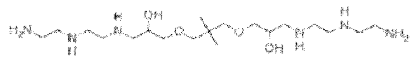 |
| (A101) | 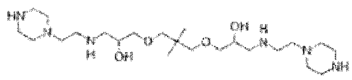 | (A102) | 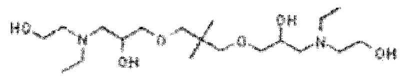 |
| (A103) | 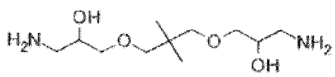 | (A104) | 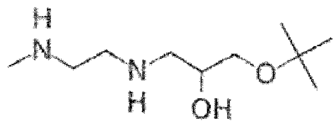 |
| (A105) | 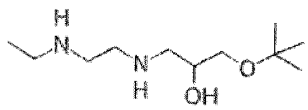 | (A106) | 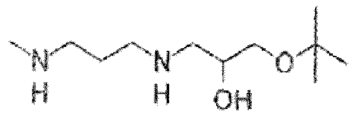 |
| (A107) | 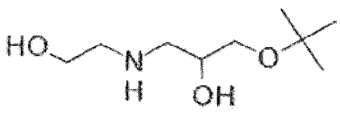 | (A108) | 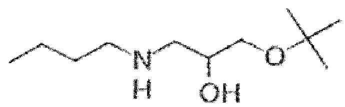 |
| (A109) | 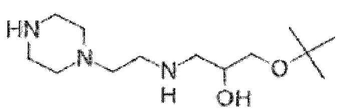 | (A110) | 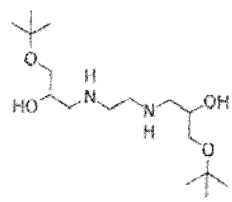 |

[Table 1-8]

Table 1-8

| Compound | Chemical Structure | Compound | Chemical Structure |
|---|---|---|---|
| (A111) | 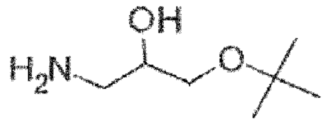 | (A112) | 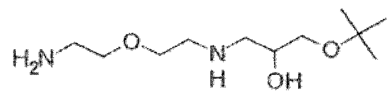 |
| (A113) | 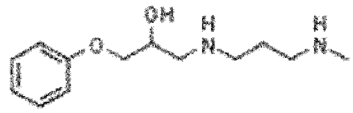 | (A114) | 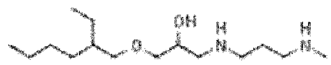 |
| (A115) | 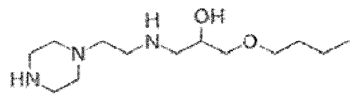 | (A116) | 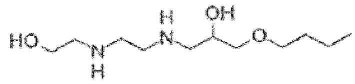 |
| (A117) | 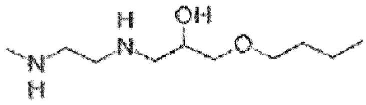 | (A118) | 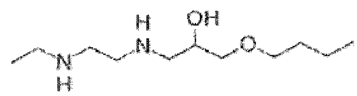 |
| (A119) | 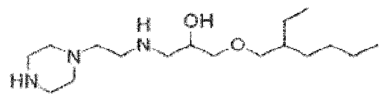 | (A120) | 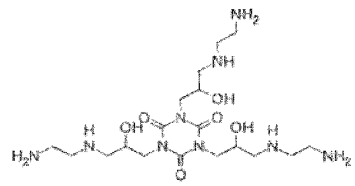 |
| (A121) | 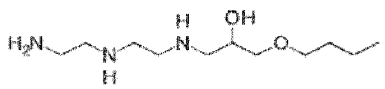 | (A122) | 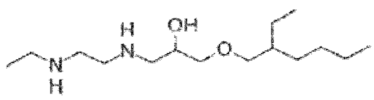 |
| (A123) | 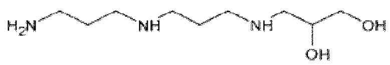 | (A124) | 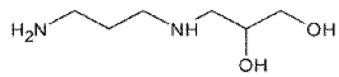 |
| (A125) | 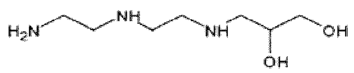 | (A126) | 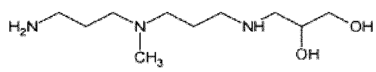 |
| (A127) | 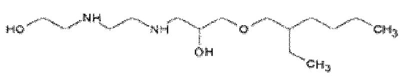 | (A128) | 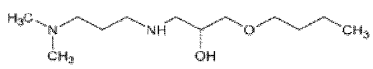 |

(Manufacturing method of amine compound (A))

An example of a manufacturing method for amine compound (A) is described, but the manufacturing method of amine compound (A) is not limited to the following method. In addition, if commercially available products exist, such commercially available products may be used.

Amine compound (A) may be obtained, for example, by reacting a compound (1b) represented by $HNR^1(R^2)$ with a monofunctional or polyfunctional epoxy compound (1c) in a solvent.

As solvents, alkanols (for example, methanol, ethanol, propanol, butanol), dimethylformamide (DMF), dimethyl sulfoxide (DMSO), and the like may be mentioned. It is preferable to perform the reaction under anhydrous conditions in

order to obtain the amine compound (A) represented by formula (1), which is the target compound.

For example, in the case of manufacturing an amine compound (A) where $R^1$ is a group represented by the above general formula (2), 4-amino-2,2,6,6-tetramethylpiperidine and the like may be used as the above compound (1b).

In addition, compounds (b1) to (b26) in Table 2 below may be mentioned as compound (1b).

[Table 2]

Table 2

| Amine raw material | Chemical Structure | Amine raw material | Chemical Structure |
|---|---|---|---|
| (b1) | 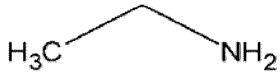 | (b2) | 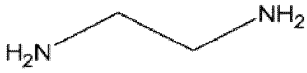 |
| (b3) | 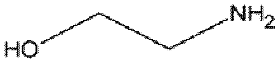 | (b4) | 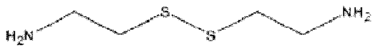 |
| (b5) | 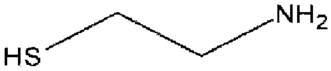 | (b6) | 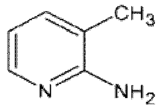 |
| (b7) | 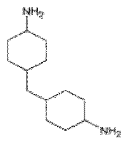 | (b8) | 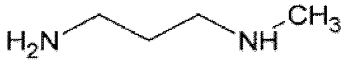 |
| (b9) | 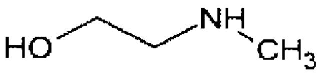 | (b10) | 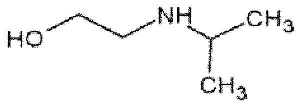 |
| (b11) | 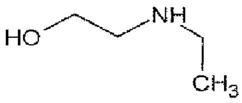 | (b12) | 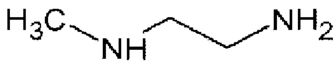 |
| (b13) | 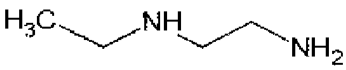 | (b14) | 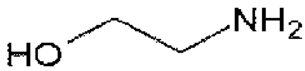 |
| (b15) | 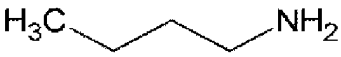 | (b16) | 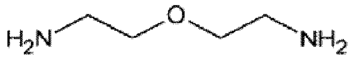 |
| (b17) | 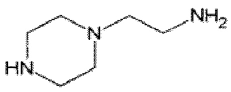 | (b18) | 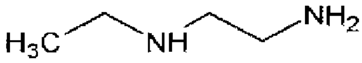 |
| (b19) | 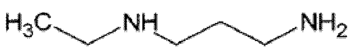 | (b20) | 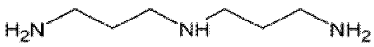 |
| (b21) | 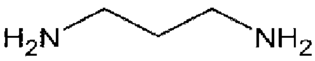 | (b22) | 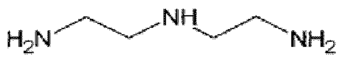 |
| (b23) | 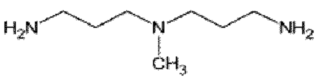 | (b24) | 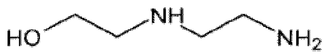 |
| (b25) | 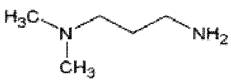 | (b26) | 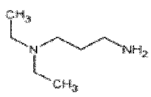 |

The mixing amount of compound (1b) and epoxy compound (1c) is preferably in the range of 0.95 to 1.1 equivalents in terms of the primary amino group equivalent ratio of compound (1b) to the epoxy equivalent of epoxy compound (1c).

In epoxy compound (1c), a monofunctional epoxy compound refers to a compound having one epoxy group in one molecule, and a polyfunctional epoxy compound is a compound having two or more epoxy groups in one molecule.

Monofunctional epoxy compounds include monofunctional aliphatic epoxy compounds and monofunctional aromatic epoxy compounds.

Monofunctional aliphatic epoxy compounds include glycidyl ethers of aliphatic alcohols, glycidyl esters of alkyl carboxylic acids, and the like. Specific examples include allyl glycidyl ether, butyl glycidyl ether, sec-butylphenyl glycidyl ether, 2-ethylhexyl glycidyl ether, alkyl glycidyl ether with a mixture of carbon 12 and 13, glycidyl ethers of alcohols, monoglycidyl ethers of aliphatic higher alcohols, glycidyl esters of higher fatty acids, and the like.

The above-mentioned monofunctional aliphatic epoxy compounds may be synthesized or commercially available products may be used. In the case of commercially available products, for example, Denacol EX-121, Denacol EX-171, Denacol EX-192 (manufactured by Nagase ChemteX Corporation);
Epolite M-1230 (manufactured by Kyoeisha Chemical Co., Ltd.), Adeka Glycirol ED-502, Adeka Glycirol ED-502S, Adeka Glycirol ED-509E, Adeka Glycirol ED-509S, Adeka Glycirol ED-529 (manufactured by ADEKA Corporation), and the like may be mentioned.

As the above-mentioned monofunctional aromatic epoxy compounds, monoglycidyl ethers of phenol compounds such as phenol, cresol, butylphenol, or their alkylene oxide adducts; monoglycidyl etherified products of aromatic compounds having two or more phenolic hydroxyl groups such as resorcinol, hydroquinone, and catechol;

monoglycidyl etherified products of aromatic compounds having two or more alcoholic hydroxyl groups such as phenyldimethanol, phenyldiethanolol, and phenyldibutanol;
monoglycidyl esters of polybasic acid aromatic compounds having two or more carboxylic acids such as phthalic acid, terephthalic acid, and trimellitic acid; and
glycidyl ester of benzoic acid, styrene oxide, or monoepoxidized products of divinylbenzene may be mentioned.

As the above-mentioned monofunctional aromatic epoxy compounds, commercially available products may be used, and commercially available compounds may be used. In the case of commercially available products, for example, Denacol EX-141, Denacol EX-146, Denacol EX-147 (manufactured by Nagase ChemteX Corporation) and the like may be mentioned.

The polyfunctional epoxy compound may be one generally used in epoxy resin compositions, and its type is not particularly limited as long as it has two or more epoxy groups in one molecule.

The polyfunctional epoxy compounds include polyfunctional aliphatic epoxy compounds and polyfunctional aromatic epoxy compounds.

For the polyfunctional aliphatic epoxy compounds, synthesized products may be used, or commercially available products may be used.

The polyfunctional aliphatic epoxy compounds include bifunctional aliphatic epoxy compounds having two epoxy groups in the molecule such as alkylene glycol diglycidyl ethers, alkenylene glycol diglycidyl ethers;
polyfunctional aliphatic epoxy compounds having three or more epoxy groups in the molecule such as polyglycidyl ethers of alcohols with three or more functional groups like trimethylolpropane, pentaerythritol, dipentaerythritol [trimethylolpropane triglycidyl ether, pentaerythritol (tri- or tetra-) glycidyl ether, dipentaerythritol (tri-, tetra-, penta-, or hexa-) glycidyl ether, etc.] ethylene glycol diglycidyl ether, propylene glycol diglycidyl ether, neopentyl glycol diglycidyl ether, butanediol diglycidyl ether, hexanediol diglycidyl ether, cyclohexanedimethanol diglycidyl ether, polyethylene glycol diglycidyl ether, polypropylene glycol diglycidyl ether, trimethylolpropane diglycidyl ether, trimethylolpropane triglycidyl ether, or trimethylolpropane polyglycidyl ether such as a mixture of trimethylolpropane diglycidyl ether and trimethylolpropane triglycidyl ether (for example, Denacol EX-321L: manufactured by Nagase ChemteX Corporation), pentaerythritol triglycidyl ether, pentaerythritol tetraglycidyl ether, sorbitol heptaglycidyl ether, sorbitol hexaglycidyl ether, resorcinol diglycidyl ether, and the like may be mentioned.

For the polyfunctional aliphatic epoxy compounds, commercially available products may be used, for example, "EP-4088S" (manufactured by ADEKA Corporation), "EHPE3150" (manufactured by Daicel Corporation), "EX-211L", "EX-212L" (both manufactured by Nagase ChemteX Corporation) and the like may be mentioned.

The polyfunctional aromatic epoxy compounds include polyglycidyl ethers of polyvalent phenols having at least one aromatic ring such as bisphenol A, bisphenol F, or their alkylene oxide adducts;

epoxy novolac resins;
polyglycidyl ethers of aromatic compounds having two or more phenolic hydroxyl groups such as resorcinol, hydroquinone, catechol, and the like;

polyglycidyl ethers of aromatic compounds having two or more alcoholic hydroxyl groups such as phenyldimethanol, phenyldiethanol, phenyldibutanol, and the like;
polyglycidyl esters of polybasic acid aromatic compounds having two or more carboxylic acids such as phthalic acid, terephthalic acid, trimellitic acid, and the like;
diepoxides of divinylbenzene and the like may be mentioned.

For the polyfunctional aromatic epoxy compounds, synthesized compounds or commercially available products may be used.
Commercially available products may be used as the polyfunctional aromatic epoxy compounds, for example, "Denacol EX-201", "Denacol EX-711", and "Denacol EX-721" (all manufactured by Nagase ChemteX Corporation);

"Ogsol EG-280", and "Ogsol CG-400" (both manufactured by Osaka Gas Chemicals Co., Ltd.); "EXA-80CRP", and "HP4032D" (both manufactured by DIC Corporation);
"jER828", and "jER828EL" (both manufactured by Mitsubishi Chemical Corporation);
"Adeka Resin EP-4100", "Adeka Resin EP-4100G", "Adeka Resin EP-4100E", "Adeka Resin EP-4100L", "Adeka Resin EP-4100TX", "Adeka Resin EP-4000", "Adeka Resin EP-4005", "Adeka Resin EP-4901", "Adeka Resin EP-4901E" (all manufactured by ADEKA Corporation) and the like may be mentioned.

In this embodiment, the monofunctional epoxy compound and the polyfunctional epoxy compound used to obtain the amine compound represented by formula (3) may be used alone or in combination of multiple different types.
The structures of specific monofunctional or polyfunctional epoxy compounds (c1) to (c75) are shown in Table 3-1 to Table 3-5.

[Table 3-1]

Table 3-1

| Compound | Chemical Structure | Compound | Chemical Structure |
|---|---|---|---|
| (c1) | 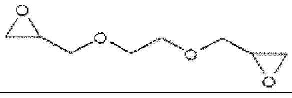 | (c2) | 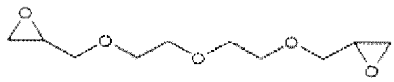 |
| (c3) | 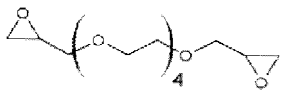 | (c4) | 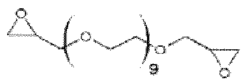 |
| (c5) | 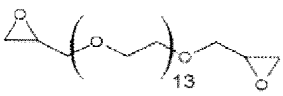 | (c6) | 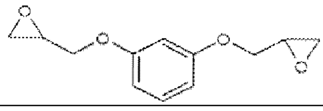 |
| (c7) | 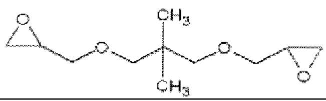 | (c8) | 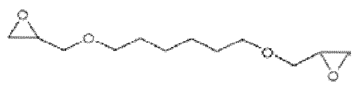 |
| (c9) | 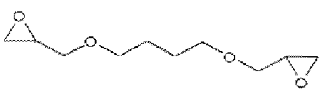 | (c10) | 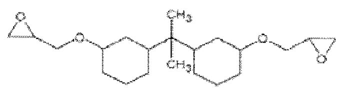 |
| (c11) | 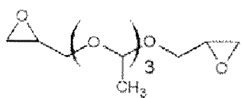 | (c12) | 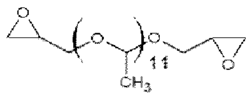 |
| (c13) | 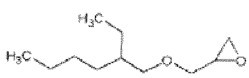 | (c14) | 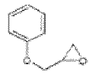 |
| (c15) | 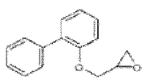 | (c16) | 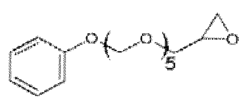 |

[Table 3-2]

Table 3-2

| Compound | Chemical Structure | Compound | Chemical Structure |
|---|---|---|---|
| (c17) | 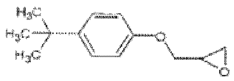 | (c18) | 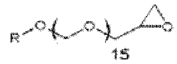<br>$R=C_{12}H_{25}$ |
| (c19) | 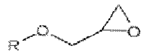<br>$R=C_{12}H_{25}$ | (c20) | 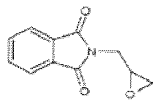 |
| (c21) | 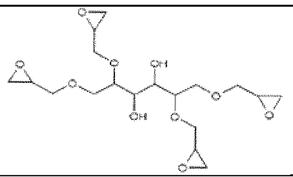 | (c22) | 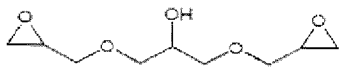 |
| (c23) | 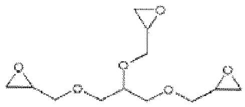 | (c24) | 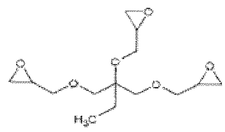 |
| (c25) | 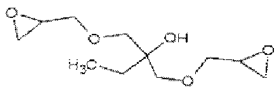 | (c26) | 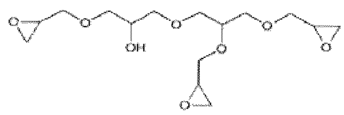 |
| (c27) | 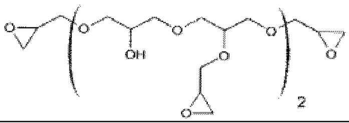 | (c28) | 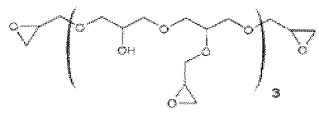 |
| (c29) | 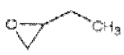 | (c30) | 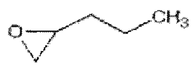 |
| (c31) | 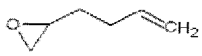 | (c32) | 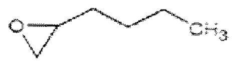 |

[Table 3-3]

Table 3-3

| Compound | Chemical Structure | Compound | Chemical Structure |
|---|---|---|---|
| (c33) | 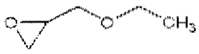 | (c34) | 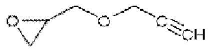 |
| (c35) | 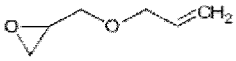 | (c36) | 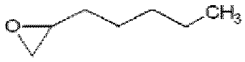 |
| (c37) | 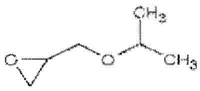 | (c38) | 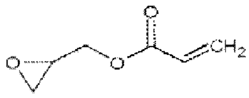 |
| (c39) | 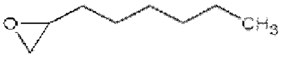 | (c40) | 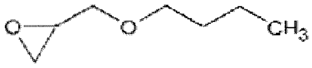 |
| (c41) | 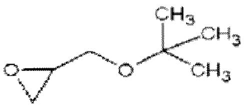 | (c42) | 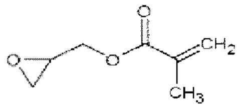 |
| (c43) | 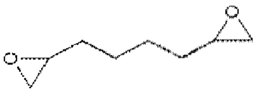 | (c44) | 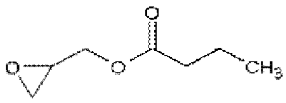 |
| (c45) | 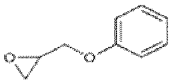 | (c46) | 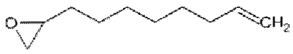 |
| (c47) | 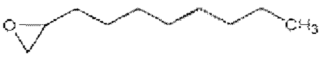 | (c48) | 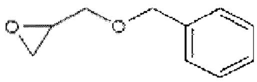 |

[Table 3-4]

Table 3-4

| Compound | Chemical Structure | Compound | Chemical Structure |
|---|---|---|---|
| (c49) | 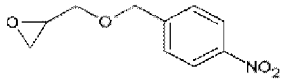 | (c50) | 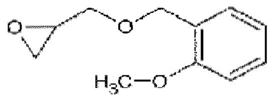 |
| (c51) | 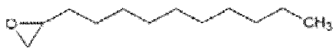 | (c52) | 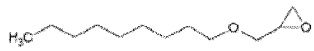 |
| (c53) | 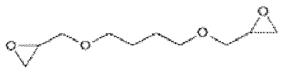 | (c54) | 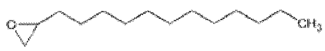 |
| (c55) | 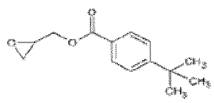 | (c56) | 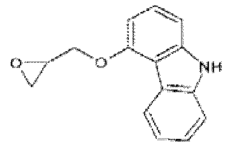 |
| (c57) | 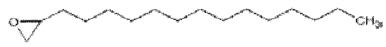 | (c58) | 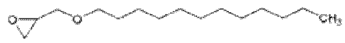 |
| (c59) | 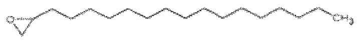 | (c60) | 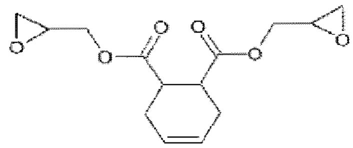 |
| (c61) | 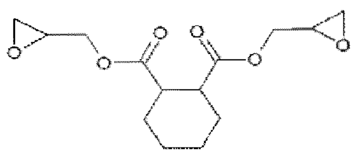 | (c62) | 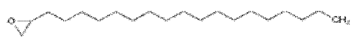 |
| (c63) | 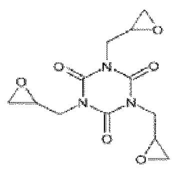 | (c64) | 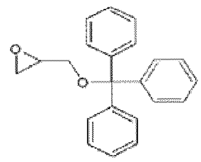 |

[Table 3-5]

Table 3-5

| Compound | Chemical Structure | Compound | Chemical Structure |
|---|---|---|---|
| (c65) | 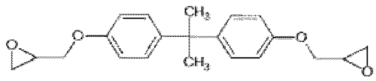 | (c66) | 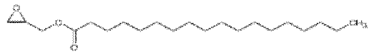 |
| (c67) | 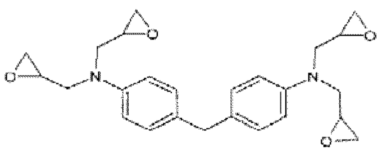 | (c68) | 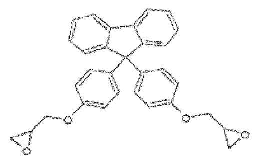 |
| (c69) | 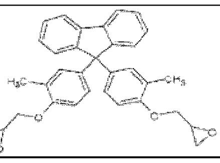 | (c70) | 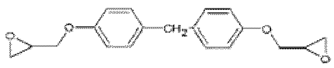 |
| (c71) | 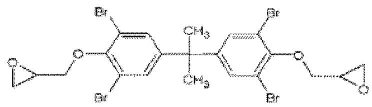 | (c72) | 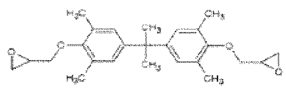 |
| (c73) | 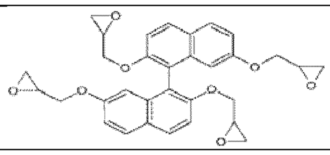 | (c74) | 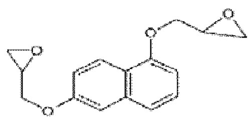 |
| (c75) | 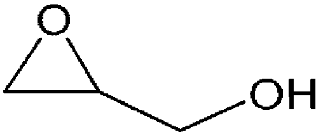 | | |

<Liquid medium (B)>

The liquid medium (B) is not particularly limited as long as it may be mixed with the amine compound (A). The above-mentioned amine compound (A) may absorb carbon dioxide and release carbon dioxide by heating, but high energy is required for the separation of carbon dioxide. By using a liquid medium, the separation efficiency of the amine compound (A) and carbon dioxide may be improved, the release temperature of carbon dioxide may be lowered, and the release efficiency of carbon dioxide may be increased.

Specific examples of the liquid medium (B) include water, organic solvents, ionic liquids, or mixtures thereof. The amine compound (A), as shown in the above formula (1), includes hydroxyl groups, alkyl groups, and organic residues, and therefore may be suitably dissolved not only in water but also in organic solvents.

Ionic liquids refer to salts that are liquid at 100°C and atmospheric pressure, consisting of cations and anions. It is preferable for the ionic liquid to be in a liquid state particularly at room temperature (25°C). In other words, while the melting point of the ionic liquid is not particularly limited as long as it is 100°C or below, it is preferably below 50°C, more preferably below 25°C, and especially preferably below 10°C. Furthermore, there is no specific lower limit for the melting point of the ionic liquid. It is noted that ionic liquids often remain in a liquid state due to supercooling even below their melting point, and if such a liquid state is maintained, they may be suitably used even with a higher melting point. Further, the melting point of ionic liquids may decrease when mixed with amines, and such ionic liquids may also be utilized as carbon dioxide absorbing liquids.

The anions constituting the present ionic liquid include anions of phosphoric acid, phosphonic acid, phosphoric acid esters, or phosphonic acid esters.

In the ionic liquid, while the cation is not particularly limited, it is preferably imidazolium, ammonium, or phosphonium.

Specific examples of water and organic solvents that may be used as the liquid medium (B), along with their total

Hansen solubility parameters (δT), are listed in Table 4-1 and Table 4-2.

(Total Hansen Solubility Parameter (δT))

The Hansen solubility parameter divides the total Hildebrand value into three components: dispersive force (δD), polar component (δP), and hydrogen bonding (δH) component. The Hildebrand value is calculated using the relationship between vaporization, van der Waals forces, and solubility. The total Hansen solubility parameter (δT) is decomposed into dispersive (δD), polar (δP), and hydrogen bonding (δH) forces, and is calculated using equation (1).

$$\delta T^2 = \delta D^2 + \delta P^2 + \delta H^2 (1)$$

(1)

In the formula,

- δD is the dispersive component,
- δP is the polar component, and
- δH is the hydrogen bonding component.

In this specification, the calculation of "Hansen solubility parameter" refers to the value calculated using the computer software "Hansen Solubility Parameters in Practice (HSPiP)". It is noted that the version of "HSPiP" used for the calculation is "5.4.02".

Further, in the case of using a mixed solvent, first, the total Hansen solubility parameter for each solvent is calculated individually, then the weighted average is determined using the mass fraction of each solvent as the weight, and this weighted average is used as the total Hansen solubility parameter (δT) of the liquid medium (B).

Table 4-1 and Table 4-2 show the dispersive component, polar component, and hydrogen bonding component of different solvents, as well as the calculation results of the total Hansen solubility parameter (δT) for different solvents.

[Table 4-1]

Table 4-1

| Solvent Type | $d_D$ | $d_P$ | $d_H$ | $d_T$ |
|---|---|---|---|---|
| Hexane | 14.9 | 0 | 0 | 14.90 |
| Heptane | 15.3 | 0 | 0 | 15.30 |
| Methylcyclohexane | 16 | 0 | 1 | 16.03 |
| Dimethylcyclohexane | 16.1 | 0 | 0.5 | 16.11 |
| Isobutyl isobutyrate | 15.1 | 2.8 | 5.8 | 16.42 |
| Tertiary butyl acetate | 15 | 3.7 | 6 | 16.57 |
| Cyclohexane | 16.8 | 0 | 0.2 | 16.80 |
| Diisobutyl ketone | 16 | 3.7 | 4.1 | 16.93 |
| Methyl isobutyl ketone | 15.3 | 6.1 | 4.1 | 16.97 |
| Diethylene glycol dibutyl ether | 15.9 | 4.0 | 4.7 | 17.00 |
| Isopentyl acetate | 15.3 | 3.1 | 7 | 17.11 |
| Triethylene glycol dibutyl ether | 15.9 | 4.2 | 5.1 | 17.20 |
| Sec-butyl acetate | 15 | 3.7 | 7.6 | 17.22 |
| Methyl oleate | 16.2 | 3.8 | 4.5 | 17.24 |
| Amyl acetate | 15.8 | 3.3 | 6.1 | 17.26 |
| Butyl acetate | 15.8 | 3.7 | 6.3 | 17.41 |
| Propyl propionate | 15.5 | 5.6 | 5.7 | 17.44 |
| Methyl isoamyl ketone | 16 | 5.7 | 4.1 | 17.47 |
| Isopropyl acetate | 14.9 | 4.5 | 8.2 | 17.59 |
| Normal propyl acetate | 15.3 | 4.3 | 7.6 | 17.62 |
| Normal butyl propionate | 15.7 | 5.5 | 5.9 | 17.65 |
| Cymene | 17.4 | 2.3 | 2.4 | 17.71 |
| Cyclo Pentyl methyl ether | 16.7 | 4.3 | 4.3 | 17.77 |
| Tetraethylene glycol dibutyl ether | 15.8 | 5.4 | 6.1 | 17.80 |
| d-Limonene | 17.2 | 1.8 | 4.3 | 17.82 |
| Ethyl benzoate | 17.8 | 0.6 | 1.4 | 17.87 |
| Xylene | 17.6 | 1 | 3.1 | 17.90 |
| Tributyl phosphine | 16.3 | 6.3 | 4.3 | 18.00 |
| Ethyl acetate | 15.8 | 5.3 | 7.2 | 18.15 |
| Toluene | 18 | 1.4 | 2 | 18.16 |
| 2-Pentanone | 16 | 7.6 | 4.7 | 18.33 |
| Butyl glycol acetate | 15.3 | 7.5 | 6.8 | 18.35 |
| Propylene glycol monobutyl ether | 15.3 | 4.5 | 9.2 | 18.41 |
| 2-(2-butoxyethoxy)ethyl acetate | 16 | 4.1 | 8.2 | 18.44 |
| Propylene glycol monoethyl ether acetate | 15.6 | 6.3 | 7.7 | 18.50 |
| 2-Ethoxyethyl acetate | 16.1 | 5.9 | 7.3 | 18.60 |
| Methyl acetate | 15.5 | 7.2 | 7.6 | 18.70 |
| Texanol | 15.1 | 6.1 | 9.8 | 19.01 |
| Methyl ethyl ketone | 16 | 9 | 5.1 | 19.05 |
| Propylene glycol monomethyl ether acetate | 15.6 | 5.6 | 9.8 | 19.26 |
| Dibasic ester | 16.2 | 6.5 | 8.4 | 19.37 |
| Glycerol triacetate | 16.5 | 4.5 | 9.1 | 19.37 |
| Isophorone | 17 | 8 | 5 | 19.44 |
| Tetrahydrofuran | 16.8 | 5.7 | 8 | 19.46 |
| Anisole | 17.8 | 4.4 | 6.9 | 19.59 |
| Dipropylene glycol mono-n-butyl ether | 15.7 | 6.5 | 10 | 19.72 |
| Butyl benzoate | 18.3 | 5.6 | 5.5 | 19.91 |
| Acetone | 15.5 | 10.4 | 7 | 19.94 |
| Dipropylene glycol methyl ether | 15.5 | 5.7 | 11.2 | 19.95 |
| Methyl isobutyl carbitol | 15.4 | 3.3 | 12.3 | 19.98 |
| 1,4-Dioxane | 17.1 | 6.8 | 7.8 | 19.99 |

[Table 4-2]

Table 4-2

| Solvent Type | $d_D$ | $d_P$ | $d_H$ | $d_T$ |
|---|---|---|---|---|
| Propylene glycol monopropyl ether | 16.10 | 6.10 | 10.20 | 20.01 |
| Cyclohexanone | 17.8 | 8.4 | 5.1 | 20.33 |
| Dimethyl isosorbide | 17.6 | 7.1 | 7.5 | 20.41 |
| Diethylene glycol monobutyl ether | 16 | 7 | 10.6 | 20.43 |
| Propylene glycol monomethyl ether | 15.6 | 6.3 | 11.6 | 20.44 |
| 3-Hydroxybutyric acid | 16.6 | 5.8 | 10.8 | 20.64 |
| Ethylene glycol monobutyl ether | 16 | 5.1 | 12.3 | 20.82 |
| Diacetone alcohol | 15.8 | 8.2 | 10.8 | 20.82 |
| 3-Methoxy-1-butanol | 15.3 | 5.4 | 13.6 | 21.17 |
| Benzyl benzoate | 20 | 5.1 | 5.2 | 21.28 |
| Isopentyl alcohol | 15.8 | 5.2 | 13.3 | 21.30 |
| 1-Nitropropane | 16.6 | 12.3 | 5.5 | 21.38 |
| 1,3-Dioxolane | 18.1 | 6.6 | 9.3 | 21.39 |
| Propylene glycol phenyl ether | 17.4 | 5.3 | 11.5 | 21.52 |
| Ethyl lactate | 16 | 7.6 | 12.5 | 21.68 |
| t-Butyl alcohol | 15.2 | 5.1 | 14.7 | 21.75 |
| n-Amyl alcohol | 15.9 | 5.9 | 13.9 | 21.93 |
| Methyl carbitol | 16.2 | 7.8 | 12.6 | 21.96 |
| 2-Butanol | 15.8 | 5.7 | 14.5 | 22.19 |
| Cyclohexanol | 17.4 | 4.1 | 13.5 | 22.40 |
| Isobutanol | 15.1 | 5.7 | 15.9 | 22.66 |
| N,N-Dimethylacetamide | 16.8 | 11.5 | 10.2 | 22.77 |
| N -Methyl-2-pyrrolidone | 18 | 12.3 | 7.2 | 22.96 |
| 1-butanol | 16 | 5.7 | 15.8 | 23.20 |
| Ethylene glycol monomethyl ether | 16 | 8.2 | 15 | 23.41 |
| Methyl cellosolve | 16 | 8.2 | 15 | 23.41 |
| Hexylene glycol | 16.7 | 6.7 | 15 | 23.43 |
| Glycerol diacetate | 16.4 | 8.9 | 14.2 | 23.45 |
| Tetrahydrofurfuryl alcohol | 17.8 | 8.2 | 12.9 | 23.46 |
| 2-phenoxyethanol | 17.8 | 5.7 | 14.3 | 23.53 |
| 2-propanol | 15.8 | 6.1 | 16.4 | 23.58 |
| Benzyl alcohol | 18.4 | 6.3 | 13.7 | 23.79 |
| Dihydrolevoglucosenone | 18.9 | 12.7 | 7.1 | 23.85 |
| Acetonitrile | 15.3 | 18 | 6.1 | 24.40 |
| γ-valerolactone | 16.8 | 16.5 | 6.7 | 24.48 |
| 1-propanol | 16 | 6.8 | 17.4 | 24.60 |
| N,N-dimethylformamide | 17.4 | 13.7 | 11.3 | 24.86 |
| γ-butyrolactone | 18 | 16.6 | 7.4 | 25.58 |
| ε-caprolactone | 19.7 | 15 | 7.4 | 25.84 |
| Sulfolane | 17.8 | 17.4 | 8.7 | 26.37 |
| Dipropylene glycol | 16.5 | 10.6 | 17.7 | 26.42 |
| Ethanol | 15.8 | 8.8 | 19.4 | 26.52 |
| Dimethyl sulfoxide | 18.4 | 16.4 | 10.2 | 26.68 |
| Propylene carbonate | 20 | 18 | 4.1 | 27.22 |
| Ethylene carbonate | 18 | 21.7 | 5.1 | 28.65 |
| Propylene glycol | 16.8 | 10.4 | 21.3 | 29.05 |
| Methanol | 14.7 | 12.3 | 22.3 | 29.41 |
| Ethylene glycol | 17 | 11 | 26 | 32.95 |
| Water | 15.5 | 16 | 42.3 | 47.81 |

As the liquid medium (B), an organic solvent or ionic liquid with a total Hansen solubility parameter (δT) of 17

$MPa^{1/2}$ to 35 $MPa^{1/2}$ is preferable, an organic solvent or ionic liquid with 20 $MPa^{1/2}$ to 35 $MPa^{1/2}$ is more preferable, and an organic solvent or ionic liquid with 23 $MPa^{1/2}$ to 35 $MPa^{1/2}$ is even more preferable.

In addition, the absorbing liquid of the disclosure possesses high carbon dioxide absorption performance and release performance even with a small proportion of water. Specifically, the proportion of water in the liquid medium (B) may be 50 % by mass or less, preferably 20 % by mass or less, and more preferably 10 % by mass or less. It is noted that when using the absorbing liquid, the proportion of water in the liquid medium (B) may fluctuate depending on the composition of the gas. However, the proportion of water mentioned here refers to the initial value (at the start of use), and does not prevent the proportion of water in the liquid medium (B) from exceeding 50 % by mass due to fluctuations.

In the absorbing liquid of the disclosure, the mass ratio of the amine compound (A) and the liquid medium (B) in the absorbing liquid is preferably in the range of 5:95 to 95:5, more preferably in the range of 5:95 to 50:50, and particularly preferably in the range of 10:90 to 25:75. The content of the amine compound (A) in the absorbing liquid is preferably 5 % by mass or more, more preferably 10 % by weight or more, and particularly preferably 25 % by mass or more. From the viewpoint of absorption efficiency of the absorbing liquid, 75 % by mass or less is preferable.

The liquid medium (B) used in the absorbing liquid may dissolve the reaction product of the amine compound (A) and carbon dioxide, or it may not dissolve the reaction product.

<Optional Components>

The absorbing liquid of the disclosure may include additional components within a range that does not impair the effects of the present invention. These additional components may include: at least one amine compound (C) selected from a group consisting of amino alcohols, cyclic polyamines, and chain polyamines; stabilizers (such as antioxidants or side reaction inhibitors) to ensure chemical or physical stability of the absorbing liquid; and preventive agents (such as corrosion inhibitors) to prevent deterioration of materials in equipment and facilities using the absorbing liquid. The total content of these additional components in the absorbing liquid is preferably 5 % by mass or less. It is noted that compounds that could be classified as both amine compound (A) and amine compound (C) are treated as belonging to amine compound (A). In other words, the amino alcohols in amine compound (C) refer to amino alcohols that do not correspond to the general formula (1).

(Amine Compound (C))

By using the amine compound (C), improvements or enhancements may be achieved in the absorbing liquid's properties such as absorption amount, release amount, absorption rate, and release rate.

Suitable amino alcohols include, for example, monoethanolamine, 2-amino-2-methyl-1-propanol, 2-amino-2-methyl-1,3-dipropanol, 2-aminobutanol, 4-aminobutanol, diethanolamine, bis(2-hydroxy-1-methylethyl)amine, methyldiethanolamine, dimethylethanolamine, diethylethanolamine, triethanolamine, dimethylamino-1-methylethanol, 2-methylaminoethanol, 2-ethylaminoethanol, 2-propylaminoethanol, n-butylaminoethanol, 2-(isopropylamino)ethanol, 3-ethylaminopropanol, triethanolamine, diethanolamine, etc. These compounds may be used alone or in combination of two or more.

Among these, from the viewpoint of further improving the reaction ability or release ability with carbon dioxide and the amine compound (A), at least one selected from a group consisting of 2-(isopropylamino)ethanol, 2-aminobutanol, and 2-amino-2-methyl-1-propanol is preferable as the amino alcohols.

Suitable cyclic polyamines are compounds in which two or more nitrogen atoms are substituted in a cycloalkyl group, specifically including piperazine, 2-methylpiperazine, 2,5-dimethylpiperazine, 2,6-dimethylpiperazine, N-(2-aminoethyl)piperazine, 1,4-bis(3-aminopropyl)piperazine, and 1-hydroxyethylpiperazine, diazabicycloundecene, and diazabicyclononene. These compounds may be used alone or in combination of two or more.

Among these, from the viewpoint of further improving the reaction ability or release ability with carbon dioxide and the amine compound (A), at least one selected from a group consisting of piperazine, N-(2-aminoethyl)piperazine, and diazabicycloundecene is preferable as the cyclic polyamines.

Suitable chain polyamines specifically include compounds having two or more substituted nitrogen atoms, with a straight-chain or branched alkyl group having 2 to 6 carbon atoms therebetween, such as ethylenediamine, N-isopropylethylenediamine, N-methylethylenediamine, N-ethylethylenediamine, N,N'-dimethylethylenediamine, N,N'-diethylethylenediamine, diethylenetriamine, 2,2-diamino-N-methyldiethylamine, N,N'-diisopropylethylenediamine, N,N'-di-tert-butylethylenediamine, N,N',N''-trimethylethylenediamine, triethylenetetramine, triethylenepentamine, N,N,N',N'-tetramethylethylenediamine, N,N-diethyl-N',N'-dimethylethylenediamine, N,N-diethyl-N',N'-dimethylethylenediamine, N,N,N',N'-tetraethylethylenediamine, tetraethylenepentamine, 1,3-diaminopropane, 3-(methylamino)propylamine, N-methyl-1,3-propanediaminopropane, N,N-dimethyl-1,3-propanediamine, N,N-diethyl-1,3-propanediamine, N,N-dibutyl-1,3-propanediamine, 3,3-diaminodipropylamine, tris(3-aminopropyl)amine, 3,3-diamino-N-methyldipropylamine, N,N'-bis(3-aminopropyl)ethylenediamine, N,N,N',N'-tetramethyl-1,3-propanediamine, N,N,N',N'-tetraethyl-1,3-propanediamine, 1,4-diaminobutane, aminoethylaminoethanol, and guanidine derivatives. These compounds may be used alone or in combination of two or more.

Among these, from the viewpoint of further improving the reaction ability or release ability with carbon dioxide and the amine compound (A), at least one selected from a group consisting of 1,4-diaminobutane, 1,3-diaminopropane, 3,3-diaminodipropylamine, 3,3-diamino-N-methyldipropylamine, N,N'-bis(3-aminopropyl)ethylenediamine, and N,N-dibutyl-1,3-propanediamine is preferable as the chain polyamines.

(Antioxidant)

Examples of the above-mentioned antioxidant include dibutylhydroxytoluene, butylhydroxyanisole, sodium erythorbate, sodium sulfite, and sulfur dioxide.

(Corrosion inhibitor)

Examples of the above-mentioned corrosion inhibitor include 1-hydroxyethane-1,1-diphosphonic acid, 2-phosphonobutane-1,2,4-tricarboxylic acid, 1-phosphonopropane-2-dicarboxylic acid, phosphonosuccinic acid, 2-hydroxyphosphonoacetic acid, maleic acid-based polymers (e.g., a copolymer of maleic acid and amylene, or a terpolymer of maleic acid, acrylic acid, and styrene).

(Defoaming agent)

Examples of the above-mentioned defoaming agent include silicone-based, polyether-based, acetylene diol-based, metal soap-based, phosphate ester-based, and fatty acid ester-based agents.

(pH adjusting agent)

Examples of the above-mentioned pH adjusting agent include inorganic acids (such as hydrochloric acid, sulfuric acid, phosphoric acid, and boric acid), organic acids (such as citric acid, formic acid, acetic acid, oxalic acid, and p-toluenesulfonic acid), inorganic bases (such as sodium bicarbonate, sodium carbonate, potassium carbonate, sodium hydroxide, potassium hydroxide, and ammonia), and organic bases (such as methylamine, dimethylamine, trimethylamine, diazabicycloundecene, piperazine, ethanolamine, and triethanolamine).

(Viscosity adjusting agent)

Examples of the above-mentioned viscosity adjusting agent include polyimine, polyvinyl alcohol, and polyethylene oxide.

<Gas containing carbon dioxide>

Examples of gas containing carbon dioxide include exhaust gas from thermal power plants using coal, heavy oil, or natural gas as fuel, boilers in manufacturing plants, kilns in cement factories, blast furnaces in steel manufacturing where iron oxide is reduced with coke, steel-making converters where carbon in pig iron is combusted, coal gasification combined cycle power generation facilities, natural gas during extraction, and reformed gas. The carbon dioxide concentration in such gas is typically about 5 to 50% by volume, particularly preferably about 10 to 40% by volume. Within this range of carbon dioxide concentration, the effects of the present carbon dioxide absorbing liquid are favorably exhibited. It is noted that the gas containing carbon dioxide may also include gases other than carbon dioxide, such as nitrogen, water vapor, carbon monoxide, hydrogen sulfide, carbonyl sulfide, sulfur dioxide, nitrogen dioxide, methane, and hydrogen.

The absorbing liquid of the disclosure also excels in absorbing hydrogen sulfide in addition to carbon dioxide.

[Method for separating and recovering carbon dioxide using the absorbing liquid]

The method for separating and recovering carbon dioxide of the disclosure is a method for separating and recovering carbon dioxide from a gas containing carbon dioxide, including: Process A in which the absorbing liquid of the disclosure is brought into contact with a gas containing carbon dioxide to obtain an absorbing liquid that has absorbed carbon dioxide from the gas containing carbon dioxide, and Process B in which the absorbing liquid that has absorbed carbon dioxide obtained in Process A is heated to desorb and release carbon dioxide from the absorbing liquid and the released carbon dioxide is recovered.

(Process A)

In Process A, the absorbing liquid is brought into contact with the gas containing carbon dioxide to absorb and separate the carbon dioxide from the gas containing carbon dioxide into the absorbing liquid.

In Process A, the method of bringing the absorbing liquid into contact with the gas containing carbon dioxide is not particularly limited. For example, methods include bubbling the gas containing carbon dioxide into the absorbing liquid, spraying the absorbing liquid in a mist form into the gas containing carbon dioxide (spray or spraying method), and counter-currently contacting high-pressure gas containing carbon dioxide with the absorbing liquid in an absorption tower filled with ceramic or metal mesh packing materials.

The temperature in Process A may be 25 to 40°C. Within this range, the absorbing liquid excels in carbon dioxide recovery amount and carbon dioxide absorption rate. The temperature in Process A is preferably 25 to 35°C.

The pressure in Process A may normally be 1.0 bar or higher, preferably 1.0 to 3.5 bar. Moreover, by conducting the process at a higher pressure, even higher carbon dioxide absorption performance may be obtained.

(Process B)

In Process B, the absorbing liquid that has absorbed carbon dioxide obtained in Process A is heated to desorb and release carbon dioxide from the absorbing liquid and the released carbon dioxide is recovered.

The temperature in the process of desorbing and releasing carbon dioxide in Process B may be 50 to 160°C. Within this range, the absorbing liquid excels in carbon dioxide release rate. The heating temperature in Process B is preferably 50 to 80°C, and more preferably 50 to 60°C.

The pressure in the process of desorbing and releasing carbon dioxide in Process B may normally be 3.5 bar or lower, preferably 1.0 to 3.5 bar. Moreover, by conducting the process at a lower pressure, even higher carbon dioxide release performance may be obtained.

The method of heating the absorbing liquid that has absorbed carbon dioxide to desorb and release carbon dioxide, and recover the same is not particularly limited. For example, methods such as heating the absorbing liquid and foaming it in a kettle to desorb carbon dioxide, similar to distillation, or heating while expanding the liquid interface in a release tower filled with packing materials such as tray columns, spray towers, ceramic, or metal mesh may be mentioned. By these methods, pure or very high concentration carbon dioxide may be recovered.

The absorbing liquid after releasing carbon dioxide in Process B may be returned to Process A again for circulation and reuse. In this circulation process, the heat added in Process B is utilized for raising the temperature of the absorbing liquid through heat exchange with the absorbing liquid that has absorbed carbon dioxide. This heat exchange contributes to reducing the overall energy consumption of the carbon dioxide separation and recovery process.

The carbon dioxide separated and recovered by the method for separating and recovering carbon dioxide using the absorbing liquid of the disclosure typically has a volume concentration of 95 to 100%, and may be pure or very highly concentrated. The separated and recovered carbon dioxide may be used for isolation storage (CCS) in underground or seabed locations, or for enhanced oil recovery (EOR), technologies which are currently under development. Other uses for the separated and recovered carbon dioxide are not particularly limited. For example, it may be used as a synthetic raw material for chemical products, or as a refrigerant for food freezing.

Examples

The present invention is described in more detail below with reference to examples. However, the present invention is not limited to these examples. It is noted that the molecular weight was measured using a time-of-flight mass spectrometer (TOF-MS).

Instrument name of TOF-MS: AutoFlex II manufactured by Bruker Daltonics

1. Example Group 1

[Synthesis Example 1]

Synthesis method of compound (A1)

[Chemical formula 6]

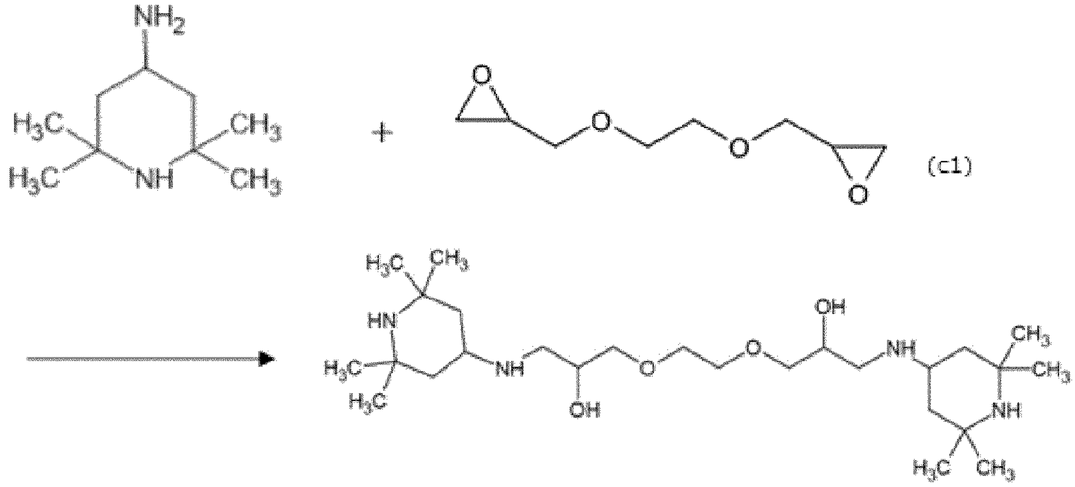

Under a nitrogen atmosphere, 20.0 g (128.0 mmol) of 4-amino-2,2,6,6-tetramethylpiperidine mixed with 50 ml of methanol and 11.15 g (64.0 mmol) of ethylene glycol diglycidyl ether (c1) were added and stirred. After addition, the mixture was stirred at room temperature for 24 hours, and the progress of the reaction was confirmed by the absence of the reactant (c1) detected by TOF-MS. The solvent methanol was removed by reducing pressure at 40°C or lower, and the target compound (A1) was obtained.

<TOF-MS measurement results of compound (A1)>

Calculated molecular weight: C26H54N4O4, Mol. Wt. 486.7;
Observed molecular weight: m/z 486.9

[Synthesis Examples 2 to 74]

In the same manner as in Synthesis Example 1, compounds (A2) to (A74) were synthesized using 4-amino-2,2,6,6-tetramethylpiperidine and mono-functional or multi-functional epoxy compounds ((c2) to (c74)) described in Table 3-1 to Table 3-5 The obtained compounds were identified by TOF-MS in the same manner as in Synthesis Example 1. The mass spectrum results of the synthesized compounds are shown in Table 5. It is noted that the compound numbers are the same as those described in Table 1-1 to Table 1-5 of this specification.

[Synthesis Example 75]

Synthesis method of compound (A75)

[Chemical formula 7]

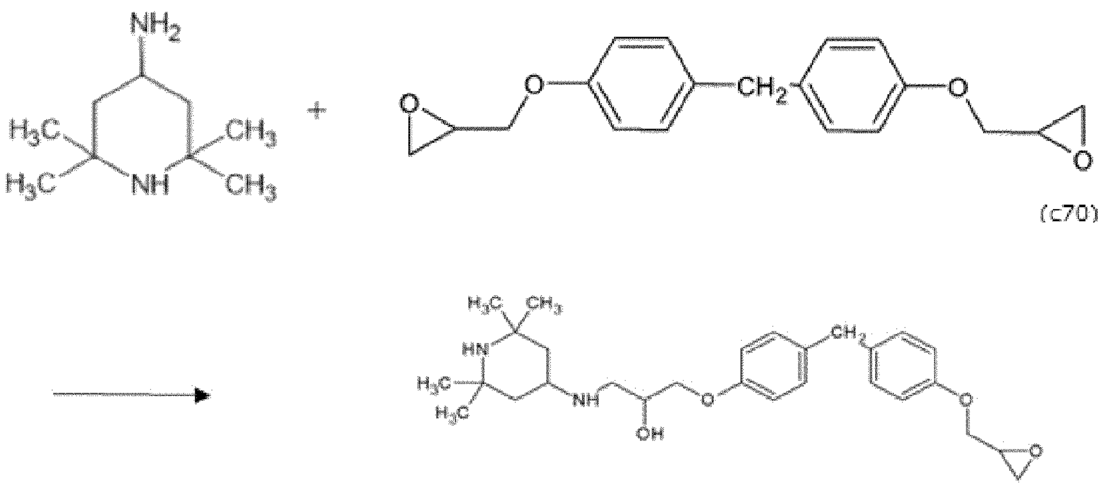

(c70)

Under a nitrogen atmosphere, 5.0 g (49.9 mmol) of 4-amino-2,2,6,6-tetramethylpiperidine mixed with 50 ml of methanol and 8.69 g (49.9 mmol) of 2,2-bis(4-glycidyloxyphenyl)methane (c70) were added and stirred. After addition, the mixture was stirred at room temperature for 24 hours, and the progress of the reaction was confirmed by the absence of the reactant (c70) detected by TOF-MS. The solvent methanol was removed by reducing pressure at 40°C or lower, and the target compound (A75) was obtained.

[Synthesis Example 76]

Synthesis method of compound (A76)

[Chemical formula 8]

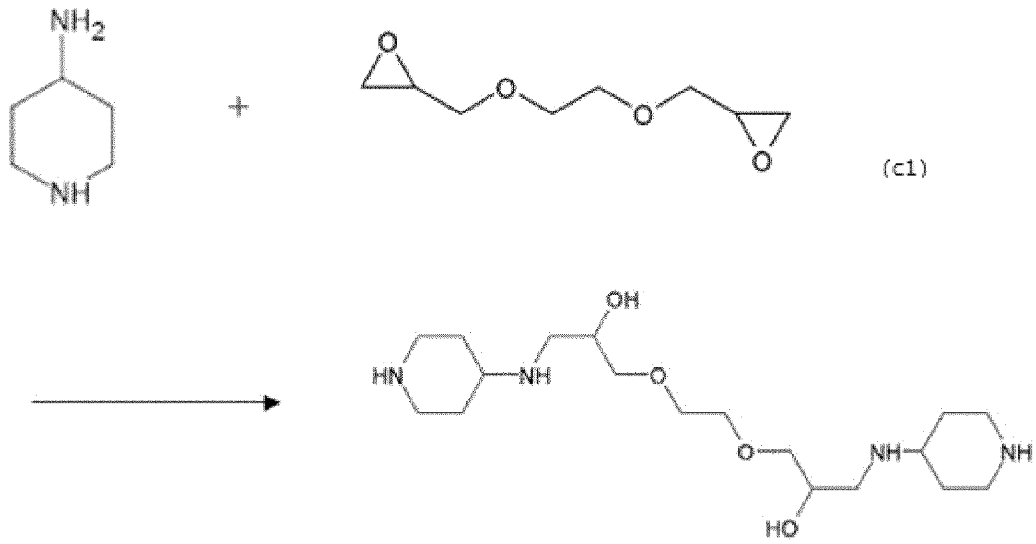

(c1)

Under a nitrogen atmosphere, 5.0 g (49.9 mmol) of 4-amino-piperidine mixed with 50 ml of methanol and 4.34 g (25.0 mmol) of ethylene glycol diglycidyl ether (c1) were added and stirred. After addition, the mixture was stirred at room temperature for 24 hours, and the progress of the reaction was confirmed by the absence of the reactant (c1) detected by TOF-MS. The solvent methanol was removed by reducing pressure at 40°C or lower, and the target compound (A76) was obtained.

[Synthesis Example 77]

Synthesis method of compound (A77)

[Chemical formula 9]

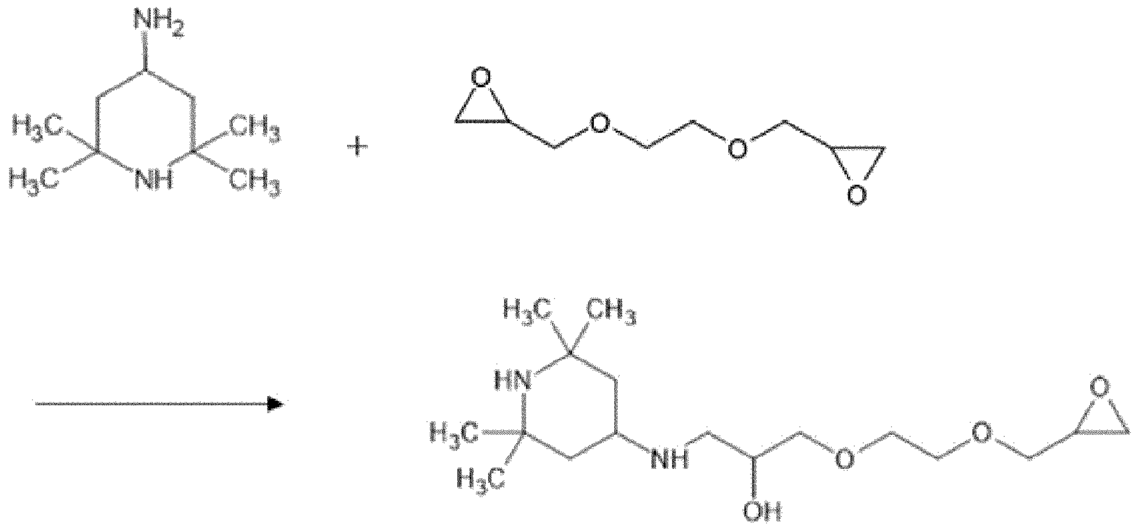

Under a nitrogen atmosphere, in Synthesis Example 1, the amount of 4-amino-2,2,6,6-tetramethylpiperidine was changed from 20 g to 10 g (64 mmol), and the synthesis reaction was carried out in the same manner. After addition, the mixture was stirred at room temperature for 24 hours, and the progress of the reaction was confirmed by the absence of the reactant 4-amino-2,2,6,6-tetramethylpiperidine detected by TOF-MS. The solvent methanol was removed by reducing pressure at 40°C or lower, and the target compound (A77) was obtained.

[Synthesis Example 78]

Synthesis method of compound (A78)

[Chemical formula 10]

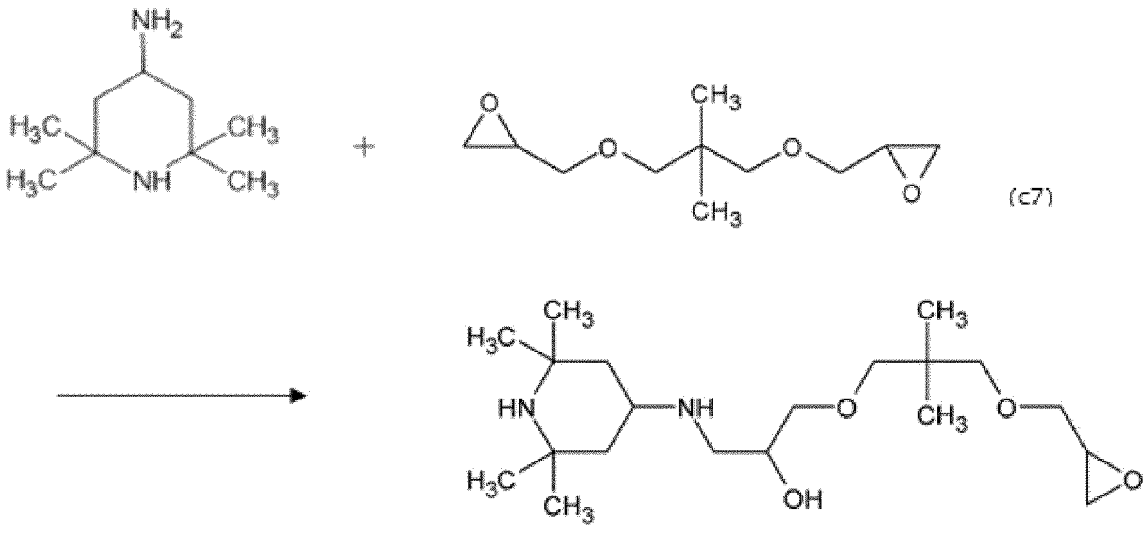

(c7)

Under a nitrogen atmosphere, in Synthesis Example 7, the amount of 4-amino-2,2,6,6-tetramethylpiperidine was

changed from 20 g to 10.5 g (64.0mmol), and the synthesis reaction was carried out in the same manner. After addition, the mixture was stirred at room temperature for 24 hours, and the progress of the reaction was confirmed by the absence of the reactant 4-amino-2,2,6,6-tetramethylpiperidine detected by TOF-MS. The solvent methanol was removed by reducing pressure at 40°C or lower, and the target compound (A78) was obtained.

[Table 5]

Table 5

| Synthesis example | Compound | TOF-MS measured value | Theoretical value | Synthesis example | Compound | TOF-MS measured value | Theoretical value |
|---|---|---|---|---|---|---|---|
| 1 | (A1) | 486.9 | 486.7 | 40 | (A40) | 286.6 | 286.5 |
| 2 | (A2) | 530.9 | 530.8 | 41 | (A41) | 286.6 | 286.5 |
| 3 | (A3) | 667.1 | 666.9 | 42 | (A42) | 298.5 | 298.4 |
| 4 | (A4) | 1026.9 | 1026.8 | 43 | (A43) | 454.8 | 454.7 |
| 5 | (A5) | 1207.5 | 1207.3 | 44 | (A44) | 300.5 | 300.4 |
| 6 | (A6) | 534.8 | 534.8 | 45 | (A45) | 306.5 | 306.4 |
| 7 | (A7) | 528.9 | 528.8 | 46 | (A46) | 310.6 | 310.5 |
| 8 | (A8) | 542.9 | 542.8 | 47 | (A47) | 312.6 | 312.5 |
| 9 | (A9) | 514.9 | 514.8 | 48 | (A48) | 320.6 | 320.5 |
| 10 | (A10) | 665.2 | 665.1 | 49 | (A49) | 365.6 | 365.5 |
| 11 | (A11) | 574.9 | 574.8 | 50 | (A50) | 350.6 | 350.5 |
| 12 | (A12) | 927.3 | 927.2 | 51 | (A51) | 340.7 | 340.6 |
| 13 | (A13) | 342.7 | 342.6 | 52 | (A52) | 356.7 | 356.6 |
| 14 | (A14) | 306.5 | 306.4 | 53 | (A53) | 514.9 | 514.8 |
| 15 | (A15) | 382.6 | 382.5 | 54 | (A54) | 368.7 | 368.6 |
| 16 | (A16) | 456.8 | 456.7 | 55 | (A55) | 390.7 | 390.6 |
| 17 | (A17) | 362.7 | 362.6 | 56 | (A56) | 395.6 | 395.5 |
| 18 | (A18) | 878.8 | 878.7 | 57 | (A57) | 396.8 | 396.7 |
| 19 | (A19) | 398.4 | 398.3 | 58 | (A58) | 398.8 | 398.7 |
| 20 | (A20) | 359.6 | 359.5 | 59 | (A59) | 424.9 | 424.8 |
| 21 | (A21) | 1031.6 | 1031.5 | 60 | (A60) | 609.0 | 608.9 |
| 22 | (A22) | 516.9 | 516.8 | 61 | (A61) | 611.0 | 610.9 |
| 23 | (A23) | 743.2 | 743.1 | 62 | (A62) | 452.9 | 452.8 |
| 24 | (A24) | 771.3 | 771.2 | 63 | (A63) | 808.3 | 808.2 |
| 25 | (A25) | 544.9 | 544.8 | 64 | (A64) | 486.8 | 486.7 |
| 26 | (A26) | 803.3 | 803.2 | 65 | (A65) | 653.1 | 653.0 |
| 27 | (A27) | 1179.8 | 1179.7 | 66 | (A66) | 496.9 | 496.8 |
| 28 | (A28) | 1556.2 | 1556.1 | 67 | (A67) | 1047.7 | 1047.6 |
| 29 | (A29) | 228.5 | 228.4 | 68 | (A68) | 761.2 | 761.1 |
| 30 | (A30) | 242.5 | 242.4 | 69 | (A69) | 789.2 | 789.1 |
| 31 | (A31) | 240.5 | 240.4 | 70 | (A70) | 625.0 | 624.9 |
| 32 | (A32) | 256.5 | 256.4 | 71 | (A71) | 968.6 | 968.5 |
| 33 | (A33) | 258.5 | 258.4 | 72 | (A72) | 709.2 | 709.1 |
| 34 | (A34) | 254.5 | 254.4 | 73 | (A73) | 1167.8 | 1167.7 |
| 35 | (A35) | 256.5 | 256.4 | 74 | (A74) | 584.9 | 584.8 |
| 36 | (A36) | 270.6 | 270.5 | 75 | (A75) | 1249.9 | 1249.8 |
| 37 | (A37) | 272.5 | 272.4 | 76 | (A76) | 468.7 | 468.6 |
| 38 | (A38) | 284.5 | 284.4 | 77 | (A77) | 330.6 | 330.5 |
| 39 | (A39) | 284.6 | 284.5 | 78 | (A78) | 372.7 | 372.6 |

[Measurement method for release efficiency of carbon dioxide gas]

The carbon dioxide absorbing liquid (100 g) (in a state placed in a 200 ml gas absorption bottle) prepared in the later-described examples was temperature-adjusted to 25°C in a water bath. A mixed gas (500 ml/min) consisting of carbon dioxide gas at 100 ml/min and nitrogen gas at 400 ml/min was bubbled through this carbon dioxide absorbing liquid

for 1 hour. The absorption amount of carbon dioxide gas (carbon dioxide absorption amount for 1 hour (L)) during this process was measured using a gas flow meter and a carbon dioxide concentration meter. Using this carbon dioxide absorption amount for 1 hour (L), the carbon dioxide absorption amount (L) per 1 kg of carbon dioxide absorbing liquid was calculated.

Next, this carbon dioxide absorbing liquid was temperature-adjusted to 60°C in a water bath. Nitrogen gas at 500 ml/min was bubbled through this carbon dioxide absorbing liquid for 2 hours. The release amount of carbon dioxide gas (carbon dioxide release amount for 2 hours (L)) during this process was measured using a gas flow meter and a carbon dioxide concentration meter. Using this carbon dioxide release amount for 2 hours (L), the carbon dioxide release amount (L) per 1 kg of carbon dioxide absorbing liquid was calculated. From the aforementioned carbon dioxide release amount for 2 hours (L) and the aforementioned carbon dioxide absorption amount for 1 hour (L), the carbon dioxide gas release efficiency (= carbon dioxide release amount for 2 hours (L) ÷ carbon dioxide absorption amount for 1 hour (L)) was calculated.

Based on the measurement of the calculated carbon dioxide gas release efficiency, the following criteria were established for evaluation, with S, A, and B considered as the usable range. The evaluation results are shown in Table 7-1 to Table 7-8.

S: Release efficiency of 0.8 or higher
A: Release efficiency of 0.7 or higher and less than 0.8
B: Release efficiency of 0.6 or higher and less than 0.7
C: Release efficiency less than 0.6

[Materials and gas types used for evaluation]

The following abbreviations were used to simplify the notation.

ATMP: 4-amino-2,2,6,6-tetramethylpiperidine
MDEA: N-methyldiethanolamine
MBZA: methylbenzylamine
MEA: monoethanolamine
AB: 2-aminobutanol
DMAPA: dimethylaminopropylamine

The gas types used for evaluation are shown in Table 6.

[Table 6]

| Table 6 | Gas type | Purity [%] |
|---|---|---|
| Carbon dioxide gas cylinder | $CO_2$ | 99.9 |
| Nitrogen gas cylinder | $N_2$ | 99.99 |

[Example 1]

30 g of compound (A1) obtained in Synthesis Example 1 was mixed and stirred with 70 g of dimethyl sulfoxide to prepare a carbon dioxide absorbing liquid (100 g). This liquid was placed in a 200 mL gas absorption bottle, and the release efficiency of carbon dioxide gas was measured.

The absorption amount of carbon dioxide for 1 hour (L) was 2.12 L when converted to standard state. In other words, the absorption amount of carbon dioxide per 1 kg of carbon dioxide absorbing liquid for 1 hour (L) was 21.2 L at standard state. (The carbon dioxide absorption amount per 1 kg of carbon dioxide absorbing liquid per 1 hour (mL/min) was 353 mL/min (= 21.2 [L/hour] × 1000 [mL/L] ÷ 60 [min/hour]).

The carbon dioxide release amount for 2 hours (L) was 1.83 L when converted to standard state. In other words, the carbon dioxide release amount per 1 kg of carbon dioxide absorbing liquid for 2 hours (L) was 18.3 L when converted to standard state.

(The carbon dioxide release amount per 1 kg of carbon dioxide absorbing liquid for 2 hours (mL/min) was 152 mL/min (= 18.3 [L/2 hours] × 1000 [mL/L] ÷ 120 [min/2 hours]).

From these results, the release efficiency of carbon dioxide gas was 0.85. The above results are shown in Table 7-1.

[Example 2 (absorbing liquid 2)]

The carbon dioxide absorbing liquid (100 g) was prepared in the same manner by changing 30 g of compound (A1) to 15 g and changing dimethyl sulfoxide from 70 g to 85 g. This liquid was placed in a 200 mL gas absorption bottle, and the release efficiency of carbon dioxide gas was measured. The evaluation results are shown in Table 7-1.

[Examples 3 to 116, E1 to E20, G1 to G99 (absorbing liquids 3 to 116, E1 to E20, G1 to G99)]

The compounds and liquid media described in Example 1 were changed to the compounds and liquid media described in Table 7-1 to Table 7-8, respectively, and similar experiments were conducted. The evaluation results are shown in Table 7-1 to Table 7-8.

[Table 7-1]

Table 7-1

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid Medium | dT of liquid medium [MPa1/2] | Liquid medium /wt% | | | | |
| Example 1 | Absorbing liquid1 | (A1) | 30 | Dimethyl sulfoxide | 26.68 | 70 | 21.2 | 18.0 | 0.85 | S |
| Example 2 | Absorbing liquid2 | | 15 | Dimethyl sulfoxide | 26.68 | 85 | 11.5 | 9.2 | 0.80 | S |
| Example 3 | Absorbing liquid3 | | 30 | N-Methyl-2-pyrrolidone | 22.96 | 70 | 18.2 | 12.8 | 0.70 | A |
| Example 4 | Absorbing liquid4 | | 30 | Diethylene glycol dibutyl ether | 17.00 | 70 | 18.5 | 13.2 | 0.71 | A |
| Example 5 | Absorbing liquid5 | | 30 | Triethylene glycol dibutyl ether | 17.20 | 70 | 19.0 | 13.4 | 0.71 | A |
| Example 6 | Absorbing liquid6 | | 30 | Tetraethylene glycol dibutyl ether | 17.80 | 70 | 19.2 | 13.6 | 0.71 | A |
| Example 7 | Absorbing liquid7 | | 30 | Propylene glycol monopropyl ether | 20.00 | 70 | 18.2 | 13.8 | 0.76 | A |
| Example 8 | Absorbing liquid8 | | 30 | Ethylene glycol monobutyl ether | 20.82 | 70 | 17.8 | 12.9 | 0.72 | A |
| Example 9 | Absorbing liquid9 | | 30 | Ethylene glycol monomethyl ether | 23.41 | 70 | 20.0 | 16.2 | 0.81 | S |
| Example 10 | Absorbing liquid10 | | 30 | Sulfolane | 26.37 | 70 | 20.5 | 16.4 | 0.80 | S |
| Example 11 | Absorbing liquid11 | | 30 | Water | 47.81 | 70 | 22.0 | 13.5 | 0.61 | B |
| Example E1 | Absorbing liquidE1 | | 30 | Methanol | 29.41 | 70 | 20.1 | 16.5 | 0.82 | S |
| Example E2 | Absorbing liquidE2 | | 30 | Ethanol | 26.52 | 70 | 21.0 | 17.4 | 0.83 | S |
| Example E3 | Absorbing liquidE3 | | 30 | 1-Propanol | 24.60 | 70 | 19.8 | 16.6 | 0.84 | S |
| Example E4 | Absorbing liquidE4 | | 30 | 3-Methoxy-1-butanol | 21.17 | 70 | 19.0 | 14.3 | 0.75 | A |
| Example E5 | Absorbing liquidE5 | | 30 | 1-Methoxy-2-propanol | 20.44 | 70 | 18.4 | 14.0 | 0.76 | A |
| Example 12 | Absorbing liquid12 | (A2) | 30 | Dimethyl sulfoxide | 26.68 | 70 | 21.8 | 18.0 | 0.83 | S |
| Example 13 | Absorbing liquid13 | | 30 | N-Methyl-2-pyrrolidone | 22.96 | 70 | 20.9 | 16.4 | 0.78 | A |
| Example 14 | Absorbing liquid14 | | 30 | Diethylene glycol dibutyl ether | 17.00 | 70 | 17.8 | 12.5 | 0.70 | A |
| Example 15 | Absorbing liquid15 | | 30 | Triethylene glycol dibutyl ether | 17.20 | 70 | 18.2 | 12.9 | 0.71 | A |
| Example 16 | Absorbing liquid16 | | 30 | Tetraethylene glycol dibutyl ether | 17.80 | 70 | 18.4 | 13.1 | 0.71 | A |
| Example 17 | Absorbing liquid17 | | 30 | Propylene glycol monopropyl ether | 20.00 | 70 | 19.2 | 13.5 | 0.70 | A |
| Example 18 | Absorbing liquid18 | | 30 | Ethylene glycol monobutyl ether | 20.82 | 70 | 19.3 | 13.8 | 0.72 | A |
| Example 19 | Absorbing liquid19 | | 30 | Ethylene glycol monomethyl ether | 23.41 | 70 | 19.9 | 16.1 | 0.81 | S |
| Example 20 | Absorbing liquid20 | | 30 | Sulfolane | 26.37 | 70 | 20.5 | 16.4 | 0.80 | S |
| Example 21 | Absorbing liquid21 | | 30 | Water | 47.81 | 70 | 21.2 | 12.9 | 0.61 | B |
| Example E6 | Absorbing liquidE6 | | 30 | Methanol | 29.41 | 70 | 21.2 | 17.2 | 0.81 | S |
| Example E7 | Absorbing liquidE7 | | 30 | Ethanol | 26.52 | 70 | 19.9 | 15.9 | 0.80 | S |
| Example E8 | Absorbing liquidE8 | | 30 | 1-Propanol | 24.60 | 70 | 20.4 | 16.5 | 0.81 | S |
| Example E9 | Absorbing liquidE9 | | 30 | 3-Methoxy-1-butanol | 21.17 | 70 | 20.7 | 14.7 | 0.71 | A |
| Example E10 | Absorbing liquidE10 | | 30 | 1-Methoxy-2-propanol | 20.44 | 70 | 20.3 | 15.0 | 0.74 | A |

[Table 7-2]

Table 7-2

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid medium | dT of liquid medium [MPa1/2] | Liquid medium /wt% | | | | |
| Example 22 | Absorbing liquid 22 | (A6) | 30 | Dimethyl sulfoxide | 26.68 | 70 | 19.8 | 17.7 | 0.89 | S |
| Example 23 | Absorbing liquid 23 | | 30 | Methylcyclohexane | 16.03 | 70 | 18.0 | 10.8 | 0.60 | B |
| Example 24 | Absorbing liquid 24 | | 30 | Diethylene glycol dibutyl ether | 17.00 | 70 | 15.8 | 11.2 | 0.71 | A |
| Example 25 | Absorbing liquid 25 | | 30 | Triethylene glycol dibutyl ether | 17.20 | 70 | 15.9 | 11.3 | 0.71 | A |
| Example 26 | Absorbing liquid 26 | | 30 | Tetraethylene glycol dibutyl ether | 17.80 | 70 | 16.1 | 11.5 | 0.71 | A |
| Example 27 | Absorbing liquid 27 | | 30 | Propylene glycol monopropyl ether | 20.00 | 70 | 16.7 | 11.8 | 0.71 | A |
| Example 28 | Absorbing liquid 28 | | 30 | Ethylene glycol monobutyl ether | 20.82 | 70 | 18.0 | 13.2 | 0.73 | A |
| Example 29 | Absorbing liquid 29 | | 30 | Ethylene glycol monomethyl ether | 23.41 | 70 | 19.6 | 15.6 | 0.80 | S |
| Example 30 | Absorbing liquid 30 | | 30 | Sulfolane | 26.37 | 70 | 18.9 | 15.6 | 0.83 | S |
| Example 31 | Absorbing liquid 31 | | 30 | Water | 47.81 | 70 | 17.8 | 10.8 | 0.61 | B |
| Example E11 | Absorbing liquid E11 | | 30 | Methanol | 29.41 | 70 | 20.2 | 16.6 | 0.82 | S |
| Example E12 | Absorbing liquid E12 | | 30 | Ethanol | 26.52 | 70 | 20.8 | 17.1 | 0.82 | S |
| Example E13 | Absorbing liquid E13 | | 30 | 1-Propanol | 24.60 | 70 | 19.4 | 16.1 | 0.83 | S |
| Example E14 | Absorbing liquid E14 | | 30 | 3-Methoxy-1-butanol | 21.17 | 70 | 19.5 | 14.2 | 0.73 | A |
| Example E15 | Absorbing liquid E15 | | 30 | 1-Methoxy-2-propanol | 20.44 | 70 | 18.3 | 13.4 | 0.73 | A |
| Example 32 | Absorbing liquid 32 | (A7) | 30 | Dimethyl sulfoxide | 26.68 | 70 | 21.1 | 17.0 | 0.81 | S |
| Example 33 | Absorbing liquid 33 | | 30 | N-Methyl-2-pyrrolidone | 22.96 | 70 | 18.6 | 13.5 | 0.73 | A |
| Example 34 | Absorbing liquid 34 | | 30 | Diethylene glycol dibutyl ether | 17.00 | 70 | 17.3 | 12.9 | 0.75 | A |
| Example 35 | Absorbing liquid 35 | | 30 | Triethylene glycol dibutyl ether | 17.20 | 70 | 16.8 | 12.1 | 0.72 | A |
| Example 36 | Absorbing liquid 36 | | 30 | Tetraethylene glycol dibutyl ether | 17.80 | 70 | 15.6 | 11.0 | 0.71 | A |
| Example 37 | Absorbing liquid 37 | | 30 | Propylene glycol monopropyl ether | 20.00 | 70 | 17.6 | 12.3 | 0.70 | A |
| Example 38 | Absorbing liquid 38 | | 30 | Ethylene glycol monobutyl ether | 20.82 | 70 | 17.8 | 12.7 | 0.71 | A |
| Example 39 | Absorbing liquid 39 | | 30 | Ethylene glycol monomethyl ether | 23.41 | 70 | 18.9 | 15.9 | 0.84 | S |
| Example 40 | Absorbing liquid 40 | | 30 | Sulfolane | 26.37 | 70 | 19.2 | 16.8 | 0.88 | S |
| Example 41 | Absorbing liquid 41 | | 30 | Water | 47.81 | 70 | 21.9 | 13.2 | 0.60 | B |
| Example 42 | Absorbing liquid 42 | | 30 | 1-Ethyl-3-methylimidazolium methyl phosphite | - | 70 | 20.7 | 17.8 | 0.86 | S |
| Example E16 | Absorbing liquid E16 | | 30 | Methanol | 29.41 | 70 | 21.3 | 17.3 | 0.81 | S |
| Example E17 | Absorbing liquid E17 | | 30 | Ethanol | 26.52 | 70 | 20.6 | 17.1 | 0.83 | S |
| Example E18 | Absorbing liquid E18 | | 30 | 1-Propanol | 24.60 | 70 | 20.7 | 16.8 | 0.81 | S |
| Example E19 | Absorbing liquid E19 | | 30 | 3-Methoxy-1-butanol | 21.17 | 70 | 18.9 | 13.4 | 0.71 | A |
| Example E20 | Absorbing liquid E20 | | 30 | 1-Methoxy-2-propanol | 20.44 | 70 | 19.7 | 14.2 | 0.72 | A |

[Table 7-3]

Table 7-3

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid medium | dT of liquid medium [MPa1/2] | Liquid medium /wt% | | | | |
| Example 43 | Absorbing liquid 43 | (A3) | 30 | Dimethyl sulfoxide | 26.68 | 70 | 19.2 | 15.7 | 0.82 | S |
| Example 44 | Absorbing liquid 44 | (A4) | 30 | | | 70 | 19.5 | 14.0 | 0.72 | A |
| Example 45 | Absorbing liquid 45 | (A5) | 30 | | | 70 | 18.9 | 13.8 | 0.73 | A |
| Example 46 | Absorbing liquid 46 | (A8) | 30 | | | 70 | 18.3 | 13.1 | 0.72 | A |
| Example 47 | Absorbing liquid 47 | (A9) | 30 | | | 70 | 18.6 | 13.4 | 0.72 | A |
| Example 48 | Absorbing liquid 48 | (A10) | 30 | | | 70 | 18.4 | 13.5 | 0.73 | A |
| Example 49 | Absorbing liquid 49 | (A11) | 30 | | | 70 | 18.3 | 13.6 | 0.74 | A |
| Example 50 | Absorbing liquid 50 | (A12) | 30 | | | 70 | 18.0 | 13.1 | 0.73 | A |
| Example 51 | Absorbing liquid 51 | (A13) | 30 | | | 70 | 18.4 | 14.7 | 0.80 | S |
| Example 52 | Absorbing liquid 52 | (A14) | 30 | | | 70 | 18.3 | 14.9 | 0.81 | S |
| Example 53 | Absorbing liquid 53 | (A15) | 30 | | | 70 | 19.2 | 15.9 | 0.83 | S |
| Example 54 | Absorbing liquid 54 | (A16) | 30 | | | 70 | 19.1 | 16.0 | 0.84 | S |
| Example 55 | Absorbing liquid 55 | (A17) | 30 | | | 70 | 19.4 | 16.2 | 0.84 | S |
| Example 56 | Absorbing liquid 56 | (A18) | 30 | | | 70 | 19.6 | 16.7 | 0.85 | S |
| Example 57 | Absorbing liquid 57 | (A19) | 30 | | | 70 | 19.4 | 15.9 | 0.82 | S |
| Example 58 | Absorbing liquid 58 | (A20) | 30 | | | 70 | 19.2 | 15.9 | 0.83 | S |
| Example 59 | Absorbing liquid 59 | (A21) | 30 | | | 70 | 19.6 | 15.6 | 0.80 | S |
| Example 60 | Absorbing liquid 60 | (A22) | 30 | | | 70 | 19.7 | 15.9 | 0.81 | S |
| Example 61 | Absorbing liquid 61 | (A23) | 30 | | | 70 | 19.3 | 15.4 | 0.80 | S |
| Example 62 | Absorbing liquid 62 | (A24) | 30 | | | 70 | 19.2 | 16.1 | 0.84 | S |
| Example 63 | Absorbing liquid 63 | (A25) | 30 | | | 70 | 19.1 | 15.9 | 0.83 | S |
| Example 64 | Absorbing liquid 64 | (A26) | 30 | | | 70 | 19.5 | 16.8 | 0.86 | S |
| Example 65 | Absorbing liquid 65 | (A27) | 30 | | | 70 | 19.7 | 17.6 | 0.89 | S |
| Example 66 | Absorbing liquid 66 | (A28) | 30 | | | 70 | 19.9 | 17.6 | 0.88 | S |
| Example 67 | Absorbing liquid 67 | (A29) | 30 | | | 70 | 20.6 | 18.2 | 0.88 | S |
| Example 68 | Absorbing liquid 68 | (A30) | 30 | | | 70 | 20.7 | 18.0 | 0.87 | S |
| Example 69 | Absorbing liquid 69 | (A31) | 30 | | | 70 | 20.4 | 17.8 | 0.87 | S |
| Example 70 | Absorbing liquid 70 | (A32) | 30 | | | 70 | 20.1 | 15.4 | 0.77 | A |
| Example 71 | Absorbing liquid 71 | (A33) | 30 | | | 70 | 20.7 | 15.2 | 0.73 | A |
| Example 72 | Absorbing liquid 72 | (A34) | 30 | | | 70 | 19.3 | 14.2 | 0.74 | A |
| Example 73 | Absorbing liquid 73 | (A35) | 30 | | | 70 | 19.1 | 13.8 | 0.72 | A |
| Example 74 | Absorbing liquid 74 | (A36) | 30 | | | 70 | 19.4 | 13.5 | 0.70 | A |

[Table 7-4]

Table 7-4

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid medium | dT of liquid medium [MPa1/2] | Liquid medium /wt% | | | | |
| Example 75 | Absorbing liquid 75 | (A37) | 30 | Dimethyl sulfoxide | 26.68 | 70 | 19.3 | 14.0 | 0.73 | A |
| Example 76 | Absorbing liquid 76 | (A38) | 30 | | | 70 | 18.7 | 14.6 | 0.78 | A |
| Example 77 | Absorbing liquid 77 | (A39) | 30 | | | 70 | 18.6 | 15.1 | 0.81 | S |
| Example 78 | Absorbing liquid 78 | (A40) | 30 | | | 70 | 17.9 | 14.8 | 0.83 | S |
| Example 79 | Absorbing liquid 79 | (A41) | 30 | | | 70 | 17.4 | 14.0 | 0.80 | S |
| Example 80 | Absorbing liquid 80 | (A42) | 30 | | | 70 | 19.2 | 15.6 | 0.81 | S |
| Example 81 | Absorbing liquid 81 | (A43) | 30 | | | 70 | 18.2 | 14.8 | 0.81 | S |
| Example 82 | Absorbing liquid 82 | (A44) | 30 | | | 70 | 19.3 | 15.9 | 0.82 | S |
| Example 83 | Absorbing liquid 83 | (A45) | 30 | | | 70 | 19.4 | 15.5 | 0.80 | S |
| Example 84 | Absorbing liquid 84 | (A46) | 30 | | | 70 | 19.0 | 16.3 | 0.86 | S |
| Example 85 | Absorbing liquid 85 | (A47) | 30 | | | 70 | 19.8 | 15.9 | 0.80 | S |
| Example 86 | Absorbing liquid 86 | (A48) | 30 | | | 70 | 19.2 | 15.6 | 0.81 | S |
| Example 87 | Absorbing liquid 87 | (A49) | 30 | | | 70 | 18.6 | 14.9 | 0.80 | S |
| Example 88 | Absorbing liquid 88 | (A50) | 30 | | | 70 | 18.4 | 14.7 | 0.80 | S |
| Example 89 | Absorbing liquid 89 | (A51) | 30 | | | 70 | 18.9 | 15.6 | 0.83 | S |
| Example 90 | Absorbing liquid 90 | (A52) | 30 | | | 70 | 18.4 | 15.4 | 0.84 | S |
| Example 91 | Absorbing liquid 91 | (A53) | 30 | | | 70 | 18.7 | 15.7 | 0.84 | S |
| Example 92 | Absorbing liquid 92 | (A54) | 30 | | | 70 | 18.6 | 15.8 | 0.85 | S |
| Example 93 | Absorbing liquid 93 | (A55) | 30 | | | 70 | 18.8 | 15.9 | 0.85 | S |
| Example 94 | Absorbing liquid 94 | (A56) | 30 | | | 70 | 18.9 | 15.7 | 0.83 | S |
| Example 95 | Absorbing liquid 95 | (A57) | 30 | | | 70 | 19.4 | 15.5 | 0.80 | S |
| Example 96 | Absorbing liquid 96 | (A58) | 30 | | | 70 | 19.7 | 14.0 | 0.71 | A |
| Example 97 | Absorbing liquid 97 | (A59) | 30 | | | 70 | 19.5 | 13.9 | 0.71 | A |
| Example 98 | Absorbing liquid 98 | (A60) | 30 | | | 70 | 19.2 | 13.9 | 0.72 | A |
| Example 99 | Absorbing liquid 99 | (A61) | 30 | | | 70 | 17.9 | 13.7 | 0.77 | A |
| Example 100 | Absorbing liquid 100 | (A62) | 30 | | | 70 | 18.6 | 14.0 | 0.75 | A |
| Example 101 | Absorbing liquid 101 | (A63) | 30 | | | 70 | 18.8 | 14.3 | 0.76 | A |
| Example 102 | Absorbing liquid 102 | (A64) | 30 | | | 70 | 18.9 | 14.4 | 0.76 | A |
| Example 103 | Absorbing liquid 103 | (A65) | 30 | | | 70 | 18.6 | 15.6 | 0.84 | S |
| Example 104 | Absorbing liquid 104 | (A66) | 30 | | | 70 | 19.2 | 15.8 | 0.82 | S |
| Example 105 | Absorbing liquid 105 | (A67) | 30 | | | 70 | 19.6 | 15.6 | 0.80 | S |
| Example 106 | Absorbing liquid 106 | (A68) | 30 | | | 70 | 19.4 | 16.3 | 0.84 | S |

[Table 7-5]

Table 7-5

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid medium | | Liquid medium /wt% | | | | |
| Example 107 | Absorbing liquid 107 | (A69) | 30 | Dimethyl sulfoxide | 26.68 | 70 | 19.3 | 15.9 | 0.82 | S |
| Example 108 | Absorbing liquid 108 | (A70) | 30 | | | 70 | 19.8 | 16.7 | 0.84 | S |
| Example 109 | Absorbing liquid 109 | (A71) | 30 | | | 70 | 19.2 | 16.0 | 0.83 | S |
| Example 110 | Absorbing liquid 110 | (A72) | 30 | | | 70 | 19.0 | 15.9 | 0.84 | S |
| Example 111 | Absorbing liquid 111 | (A73) | 30 | | | 70 | 19.3 | 15.7 | 0.81 | S |
| Example 112 | Absorbing liquid 112 | (A74) | 30 | | | 70 | 19.5 | 15.6 | 0.80 | S |
| Example 113 | Absorbing liquid 113 | (A75) | 30 | | | 70 | 19.6 | 15.6 | 0.80 | S |
| Example 114 | Absorbing liquid 114 | (A76) | 30 | | | 70 | 17.2 | 12.0 | 0.70 | A |
| Example 115 | Absorbing liquid 115 | (A77) | 30 | | | 70 | 18.0 | 13.4 | 0.74 | A |
| Example 116 | Absorbing liquid 116 | (A78) | 30 | | | 70 | 18.5 | 13.5 | 0.73 | A |
| Example G1 | Absorbing liquid G1 | (A41) | 30 | Hexane | 14.90 | 70 | 17.7 | 11.2 | 0.63 | B |
| Example G2 | Absorbing liquid G2 | | | Heptane | 15.30 | 70 | 18.2 | 11.3 | 0.62 | B |
| Example G3 | Absorbing liquid G3 | | | Methylcyclohexane | 16.03 | 70 | 18.4 | 11.6 | 0.63 | B |
| Example G4 | Absorbing liquid G4 | | | Dimethylcyclohexane | 16.11 | 70 | 18.8 | 12.0 | 0.64 | B |
| Example G5 | Absorbing liquid G5 | | | Isobutyl isobutyrate | 16.42 | 70 | 17.9 | 12.2 | 0.68 | B |
| Example G6 | Absorbing liquid G6 | | | Tertiary butyl acetate | 16.57 | 70 | 17.9 | 12.0 | 0.67 | B |
| Example G7 | Absorbing liquid G7 | | | Cyclohexane | 16.80 | 70 | 18.1 | 11.9 | 0.66 | B |
| Example G8 | Absorbing liquid G8 | | | Diisobutyl ketone | 16.93 | 70 | 17.5 | 11.7 | 0.67 | B |
| Example G9 | Absorbing liquid G9 | | | Methyl isobutyl ketone | 16.97 | 70 | 18.9 | 13.0 | 0.69 | B |
| Example G10 | Absorbing liquid G10 | | | Diethylene glycol dibutyl ether | 17.00 | 70 | 18.8 | 13.2 | 0.70 | A |
| Example G11 | Absorbing liquid G11 | | | Isopentyl acetate | 17.11 | 70 | 18.5 | 13.0 | 0.70 | A |
| Example G12 | Absorbing liquid G12 | | | Triethylene glycol dibutyl ether | 17.20 | 70 | 18.4 | 12.9 | 0.70 | A |
| Example G13 | Absorbing liquid G13 | | | Sec-butyl acetate | 17.22 | 70 | 18.0 | 12.6 | 0.70 | A |
| Example G14 | Absorbing liquid G14 | | | Methyl oleate | 17.24 | 70 | 17.8 | 12.5 | 0.70 | A |
| Example G15 | Absorbing liquid G15 | | | Amyl acetate | 17.26 | 70 | 19.2 | 13.4 | 0.70 | A |
| Example G16 | Absorbing liquid G16 | | | Butyl acetate | 17.41 | 70 | 18.0 | 12.6 | 0.70 | A |
| Example G17 | Absorbing liquid G17 | | | Propyl propionate | 17.44 | 70 | 18.7 | 13.1 | 0.70 | A |
| Example G18 | Absorbing liquid G18 | | | Methyl isoamyl ketone | 17.47 | 70 | 17.7 | 12.6 | 0.71 | A |
| Example G19 | Absorbing liquid G19 | | | Isopropyl acetate | 17.59 | 70 | 17.5 | 12.4 | 0.71 | A |
| Example G20 | Absorbing liquid G20 | | | Normal propyl acetate | 17.62 | 70 | 18.2 | 12.9 | 0.71 | A |
| Example G21 | Absorbing liquid G21 | | | Normal butyl propionate | 17.65 | 70 | 18.2 | 12.9 | 0.71 | A |
| Example G22 | Absorbing liquid G22 | | | Cymene | 17.71 | 70 | 17.8 | 12.6 | 0.71 | A |

[Table 7-6]

Table 7-6

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid medium | | Liquid medium /wt% | | | | |
| Example G23 | Absorbing liquid G23 | (A41) | 30 | Cyclopentyl methyl ether | 17.77 | 70 | 18.4 | 12.9 | 0.70 | A |
| Example G24 | Absorbing liquid G24 | | | Tetraethylene glycol dibutyl ether | 17.80 | 70 | 17.9 | 12.7 | 0.71 | A |
| Example G25 | Absorbing liquid G25 | | | d-Limonene | 17.82 | 70 | 18.2 | 12.9 | 0.71 | A |
| Example G26 | Absorbing liquid G26 | | | Ethyl benzoate | 17.87 | 70 | 18.3 | 13.2 | 0.72 | A |
| Example G27 | Absorbing liquid G27 | | | Xylene | 17.90 | 70 | 18.3 | 13.0 | 0.71 | A |
| Example G28 | Absorbing liquid G28 | | | Tributyl phosphine | 18.00 | 70 | 17.8 | 12.6 | 0.71 | A |
| Example G29 | Absorbing liquid G29 | | | Ethyl acetate | 18.15 | 70 | 18.7 | 13.1 | 0.70 | A |
| Example G30 | Absorbing liquid G30 | | | Toluene | 18.16 | 70 | 18.7 | 13.3 | 0.71 | A |
| Example G31 | Absorbing liquid G31 | | | 2-Pentanone | 18.33 | 70 | 17.5 | 12.6 | 0.72 | A |
| Example G32 | Absorbing liquid G32 | | | Butyl glycol acetate | 18.35 | 70 | 18.2 | 12.9 | 0.71 | A |
| Example G33 | Absorbing liquid G33 | | | Propylene glycol monobutyl ether | 18.41 | 70 | 18.6 | 13.4 | 0.72 | A |
| Example G34 | Absorbing liquid G34 | | | 2-(A2-butoxyethoxy)ethyl acetate | 18.44 | 70 | 18.0 | 12.8 | 0.71 | A |
| Example G35 | Absorbing liquid G35 | | | Propylene glycol monoethyl ether acetate | 18.50 | 70 | 18.8 | 13.3 | 0.71 | A |
| Example G36 | Absorbing liquid G36 | | | 2-Ethoxyethyl acetate | 18.60 | 70 | 18.4 | 13.4 | 0.73 | A |
| Example G37 | Absorbing liquid G37 | | | Methyl acetate | 18.70 | 70 | 18.5 | 13.5 | 0.73 | A |
| Example G38 | Absorbing liquid G38 | | | Texanol | 19.01 | 70 | 17.5 | 12.8 | 0.73 | A |
| Example G39 | Absorbing liquid G39 | | | Methyl ethyl ketone | 19.05 | 70 | 17.5 | 12.4 | 0.71 | A |
| Example G40 | Absorbing liquid G40 | | | Propylene glycol monomethyl ether acetate | 19.26 | 70 | 18.2 | 13.3 | 0.73 | A |
| Example G41 | Absorbing liquid G41 | | | Dibasic ester | 19.37 | 70 | 18.1 | 13.2 | 0.73 | A |
| Example G42 | Absorbing liquid G42 | | | Glycerol triacetate | 19.37 | 70 | 18.1 | 13.2 | 0.73 | A |
| Example G43 | Absorbing liquid G43 | | | Isophorone | 19.44 | 70 | 18.8 | 13.9 | 0.74 | A |
| Example G44 | Absorbing liquid G44 | | | Tetrahydrofuran | 19.46 | 70 | 18.1 | 13.6 | 0.75 | A |
| Example G45 | Absorbing liquid G45 | | | Anisole | 19.59 | 70 | 17.9 | 13.2 | 0.74 | A |
| Example G46 | Absorbing liquid G46 | | | Dipropylene glycol mono-n-butyl ether | 19.72 | 70 | 18.4 | 13.4 | 0.73 | A |
| Example G47 | Absorbing liquid G47 | | | Butyl benzoate | 19.91 | 70 | 17.6 | 13.0 | 0.74 | A |
| Example G48 | Absorbing liquid G48 | | | Acetone | 19.94 | 70 | 18.2 | 13.5 | 0.74 | A |
| Example G49 | Absorbing liquid G49 | | | Dipropylene glycol methyl ether | 19.95 | 70 | 17.9 | 12.9 | 0.72 | A |
| Example G50 | Absorbing liquid G50 | | | Methyl isobutyl carbitol | 19.98 | 70 | 18.2 | 13.5 | 0.74 | A |

[Table 7-7]

Table 7-7

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid medium | | Liquid medium /wt% | | | | |
| Example G51 | Absorbing liquid G51 | (A41) | 30 | 1,4-Dioxane | 19.99 | 70 | 18.5 | 13.7 | 0.74 | A |
| Example G52 | Absorbing liquid G52 | | | Propylene glycol monopropyl ether | 20.00 | 70 | 17.7 | 12.7 | 0.72 | A |
| Example G53 | Absorbing liquid G53 | | | Cyclohexanone | 20.33 | 70 | 18.3 | 13.5 | 0.74 | A |
| Example G54 | Absorbing liquid G54 | | | Dimethyl isosorbide | 20.41 | 70 | 18.0 | 13.0 | 0.72 | A |
| Example G55 | Absorbing liquid G55 | | | Diethylene glycol monobutyl ether | 20.43 | 70 | 17.9 | 12.9 | 0.72 | A |
| Example G56 | Absorbing liquid G56 | | | Propylene glycol monomethyl ether | 20.44 | 70 | 17.7 | 13.3 | 0.75 | A |
| Example G57 | Absorbing liquid G57 | | | 3-Hydroxybutyric acid | 20.64 | 70 | 18.3 | 13.4 | 0.73 | A |
| Example G58 | Absorbing liquid G58 | | | Ethylene glycol monobutyl ether | 20.82 | 70 | 18.4 | 13.6 | 0.74 | A |
| Example G59 | Absorbing liquid G59 | | | Diacetone alcohol | 20.82 | 70 | 17.7 | 12.6 | 0.71 | A |
| Example G60 | Absorbing liquid G60 | | | 3-Methoxy-1-butanol | 21.17 | 70 | 18.5 | 14.1 | 0.76 | A |
| Example G61 | Absorbing liquid G61 | | | Benzyl benzoate | 21.28 | 70 | 18.7 | 14.8 | 0.79 | A |
| Example G62 | Absorbing liquid G62 | | | Isopentyl alcohol | 21.30 | 70 | 17.6 | 12.7 | 0.72 | A |
| Example G63 | Absorbing liquid G63 | | | 1-Nitropropane | 21.38 | 70 | 17.8 | 12.8 | 0.72 | A |
| Example G64 | Absorbing liquid G64 | | | 1,3-Dioxolane | 21.39 | 70 | 19.0 | 14.6 | 0.77 | A |
| Example G65 | Absorbing liquid G65 | | | Propylene glycol phenyl ether | 21.52 | 70 | 18.1 | 13.6 | 0.75 | A |
| Example G66 | Absorbing liquid G66 | | | Ethyl lactate | 21.68 | 70 | 18.8 | 14.1 | 0.75 | A |
| Example G67 | Absorbing liquid G67 | | | t-Butyl alcohol | 21.75 | 70 | 17.9 | 13.6 | 0.76 | A |
| Example G68 | Absorbing liquid G68 | | | n-Amyl alcohol | 21.93 | 70 | 18.9 | 13.8 | 0.73 | A |
| Example G69 | Absorbing liquid G69 | | | Methyl carbitol | 21.96 | 70 | 18.0 | 13.9 | 0.77 | A |
| Example G70 | Absorbing liquid G70 | | | 2-Butanol | 22.19 | 70 | 18.8 | 14.7 | 0.78 | A |
| Example G71 | Absorbing liquid G71 | | | Cyclohexanol | 22.40 | 70 | 18.5 | 15.0 | 0.79 | A |
| Example G72 | Absorbing liquid G72 | | | Isobutanol | 22.66 | 70 | 18.0 | 13.7 | 0.76 | A |
| Example G73 | Absorbing liquid G73 | | | N,N-Dimethylacetamide | 22.77 | 70 | 17.8 | 13.5 | 0.76 | A |
| Example G74 | Absorbing liquid G74 | | | N-Methyl-2-pyrrolidone | 22.96 | 70 | 18.8 | 15.4 | 0.78 | A |
| Example G75 | Absorbing liquid G75 | | | 1-Butanol | 23.20 | 70 | 19.1 | 15.5 | 0.81 | S |

[Table 7-8]

Table 7-8

| Example | Absorbing liquid | Composition of carbon dioxide absorbing liquid | | | | Liquid medium /wt% | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | | | Liquid medium | | | | | | |
| Example G76 | Absorbing liquid G76 | (A41) | 30 | Ethylene glycol monomethyl ether | 23.41 | 70 | 18.1 | 14.7 | 0.81 | S |
| Example G77 | Absorbing liquid G77 | | | Methyl cellosolve | 23.41 | 70 | 17.8 | 14.2 | 0.80 | S |
| Example G78 | Absorbing liquid G78 | | | Hexylene glycol | 23.43 | 70 | 18.1 | 14.8 | 0.82 | S |
| Example G79 | Absorbing liquid G79 | | | Glycerol diacetate | 23.45 | 70 | 18.0 | 14.8 | 0.82 | S |
| Example G80 | Absorbing liquid G80 | | | Tetrahydrofurfuryl alcohol | 23.46 | 70 | 18.7 | 15.3 | 0.82 | S |
| Example G81 | Absorbing liquid G81 | | | 2-phenoxyethanol | 23.53 | 70 | 18.7 | 15.1 | 0.81 | S |
| Example G82 | Absorbing liquid G82 | | | 2-propanol | 23.58 | 70 | 18.0 | 14.8 | 0.82 | S |
| Example G83 | Absorbing liquid G83 | | | Benzyl alcohol | 23.79 | 70 | 17.9 | 14.5 | 0.81 | S |
| Example G84 | Absorbing liquid G84 | | | Dihydrolevoglucosenone | 23.85 | 70 | 18.2 | 15.1 | 0.83 | S |
| Example G85 | Absorbing liquid G85 | | | Acetonitrile | 24.40 | 70 | 17.8 | 14.6 | 0.82 | S |
| Example G86 | Absorbing liquid G86 | | | γ-valerolactone | 24.48 | 70 | 18.9 | 15.7 | 0.83 | S |
| Example G87 | Absorbing liquid G87 | | | 1-propanol | 24.60 | 70 | 18.7 | 15.5 | 0.83 | S |
| Example G88 | Absorbing liquid G88 | | | N,N-dimethylformamide | 24.86 | 70 | 17.3 | 14.2 | 0.82 | S |
| Example G89 | Absorbing liquid G89 | | | γ-butyrolactone | 25.58 | 70 | 18.3 | 15.0 | 0.82 | S |
| Example G90 | Absorbing liquid G90 | | | ε-caprolactone | 25.84 | 70 | 18.0 | 15.1 | 0.84 | S |
| Example G91 | Absorbing liquid G91 | | | Sulfolane | 26.37 | 70 | 18.7 | 15.3 | 0.82 | S |
| Example G92 | Absorbing liquid G92 | | | Dipropylene glycol | 26.42 | 70 | 17.5 | 14.9 | 0.85 | S |
| Example G93 | Absorbing liquid G93 | | | Ethanol | 26.52 | 70 | 18.2 | 15.3 | 0.84 | S |
| Example G94 | Absorbing liquid G94 | | | Propylene carbonate | 27.22 | 70 | 18.1 | 14.8 | 0.82 | S |
| Example G95 | Absorbing liquid G95 | | | Ethylene carbonate | 28.65 | 70 | 17.6 | 14.8 | 0.84 | S |
| Example G96 | Absorbing liquid G96 | | | Propylene glycol | 29.05 | 70 | 18.5 | 15.2 | 0.82 | S |
| Example G97 | Absorbing liquid G97 | | | Methanol | 29.41 | 70 | 18.4 | 15.6 | 0.85 | S |
| Example G98 | Absorbing liquid G98 | | | Ethylene glycol | 32.95 | 70 | 18.2 | 15.3 | 0.84 | S |
| Example G99 | Absorbing liquid G99 | | | Water | 47.81 | 70 | 17.8 | 11.6 | 0.65 | B |

[Example 117 (absorbing liquid 117)]

25 g of Compound (A1) and 5 g of MDEA were mixed with 70 g of dimethyl sulfoxide and stirred to prepare a carbon dioxide absorbing liquid (100 g). This liquid was placed in a 200 mL gas absorption bottle, and the experiment was performed in the same manner. The evaluation results are shown in Table 8-1.

[Example 118 (absorbing liquid 118)]

25 g of Compound (A7) and 5 g of MBZA were mixed with 60 g of sulfolane and 10 g of water, and stirred to prepare a carbon dioxide absorbing liquid (100 g). This liquid was placed in a 200 mL gas absorption bottle, and the experiment was performed in the same manner. The evaluation results are shown in Table 8-1.

[Example 119 (absorbing liquid 119)]

25 g of Compound (A30) and 5 g of MBZA were mixed with 60 g of dimethyl sulfoxide and 10 g of water, and stirred to prepare a carbon dioxide absorbing liquid (100 g). This liquid was placed in a 200 mL gas absorption bottle, and the experiment was performed in the same manner. The evaluation results are shown in Table 8-1.

[Example 120 (absorbing liquid 120)]

25 g of Compound (A1) and 5 g of Compound (A77) were mixed with 70 g of dimethyl sulfoxide and stirred to prepare a carbon dioxide absorbing liquid (100 g). This liquid was placed in a 200 mL gas absorption bottle, and the experiment was performed in the same manner. The evaluation results are shown in Table 8-1.

[Example 121 (absorbing liquid 121)]

25 g of Compound (A7) and 5 g of Compound (A78) were mixed with 70 g of dimethyl sulfoxide and stirred to prepare a carbon dioxide absorbing liquid (100 g). This liquid was placed in a 200 mL gas absorption bottle, and the experiment was performed in the same manner. The evaluation results are shown in Table 8-1.

[Example 122 (absorbing liquid 122)]

25 g of Compound (A7) and 2 g of ATMP were mixed with 60 g of dimethyl sulfoxide and 10 g of N-methyl-2-pyrrolidone, and stirred to prepare a carbon dioxide absorbing liquid (100 g). This liquid was placed in a 200 mL gas absorption bottle, and the experiment was performed in the same manner. The evaluation results are shown in Table 8-1.

[Example 123 (absorbing liquid 123)]

30 g of Compound (A1) was mixed with 60 g of dimethyl sulfoxide and 10 g of N-methyl-2-pyrrolidone, and stirred to prepare a carbon dioxide absorbing liquid (100 g). This liquid was placed in a 200 mL gas absorption bottle, and the experiment was performed in the same manner. The evaluation results are shown in Table 8-1.

[Comparative Example 1 (absorbing liquid 124)]

All 30 g of Compound (A1) in Example 1 was replaced with MEA, and 70 g of water was added and mixed by stirring to prepare a carbon dioxide absorbing liquid (100 g). This liquid was placed in a 200 mL gas absorption bottle, and the experiment was performed in the same manner. The evaluation results are shown in Table 8-1.

[Comparative Example 2 (absorbing liquid 125)]

All 30 g of Compound (A1) in Example 1 was replaced with MEA to prepare a carbon dioxide absorbing liquid (100 g). This liquid was placed in a 200 mL gas absorption bottle, and the experiment was performed in the same manner. The evaluation results are shown in Table 8-1.

[Reference Example 1 (absorbing liquid 126)]

All 30 g of Compound (A1) in Example 1 was replaced with ATMP to prepare a carbon dioxide absorbing liquid (100 g). This liquid was placed in a 200 mL gas absorption bottle, and the experiment was performed in the same manner.

The evaluation results are shown in Table 8-1.

[Examples A1 to A83 (absorbing liquids A1 to A83)]

The compounds and liquid media were changed to those listed in Table 8-2 to Table 8-4, respectively. The carbon dioxide gas absorption amount was measured according to the method described in [Measurement method for release efficiency of carbon dioxide gas] mentioned above. The carbon dioxide absorption amount (L) per 1 kg of carbon dioxide absorbing liquid and the carbon dioxide gas release efficiency were calculated. It is noted that the gas types used for the evaluation are as shown in Table 6 above.

Based on the measurement of the calculated carbon dioxide gas release efficiency, the following criteria were established for evaluation, with S, A, and B considered as the usable range. The evaluation results are shown in Table 8-1 to Table 8-4.

S: Release efficiency of 0.8 or higher
A: Release efficiency of 0.7 or higher and less than 0.8
B: Release efficiency of 0.6 or higher and less than 0.7
C: Release efficiency less than 0.6

[Table 8-1]

Table 8-1

| Example | Absorbing liquid | Composition of carbon dioxide absorbing liquid | | | | | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | dT [MPa1/2] of the entire liquid medium | | | | |
| Example 117 | Absorbing liquid 117 | (A1) | 25 | MDEA | 5 | Dimethyl sulfoxide | 70 | - | - | 26.68 | 19.8 | 16.2 | 0.82 | S |
| Example 118 | Absorbing liquid 118 | (A7) | 25 | MBZA | 5 | Sulfolane | 60 | Water | 10 | 29.43 | 20.1 | 16.7 | 0.83 | S |
| Example 119 | Absorbing liquid 119 | (A30) | 25 | MBZA | 5 | Dimethyl sulfoxide | 60 | Water | 10 | 29.70 | 15 | 11.2 | 0.75 | A |
| Example 120 | Absorbing liquid 120 | (A1) | 25 | (A77) | 5 | Dimethyl sulfoxide | 70 | - | - | 26.68 | 18.2 | 15.2 | 0.84 | S |
| Example 121 | Absorbing liquid 121 | (A7) | 25 | (A78) | 5 | Dimethyl sulfoxide | 70 | - | - | 26.68 | 18.6 | 15.4 | 0.83 | S |
| Example 122 | Absorbing liquid 122 | (A7) | 28 | ATMP | 2 | Dimethyl sulfoxide | 60 | N-Methyl-2-pyrrolidone | 10 | 26.15 | 19.3 | 15.9 | 0.82 | S |
| Example 123 | Absorbing liquid 123 | (A1) | 30 | - | - | Dimethyl sulfoxide | 60 | N-Methyl-2-pyrrolidone | 10 | 26.15 | 19.3 | 16.7 | 0.87 | S |
| Comparative Example 1 | Absorbing liquid 124 | MEA | 30 | - | - | Water | 70 | - | - | 47.81 | 9.5 | 2.2 | 0.23 | C |
| Comparative Example 2 | Absorbing liquid 125 | MEA | 30 | - | - | Dimethyl sulfoxide | 70 | - | - | 26.68 | 8.5 | 2.3 | 0.27 | C |
| Reference Example 1 | Absorbing liquid 126 | ATMP | 30 | - | - | Dimethyl sulfoxide | 70 | - | - | 26.68 | 18.3 | 11.6 | 0.63 | B |

[Table 8-2]

Table 8-2

| Example | Absorbing liquid | Composition of carbon dioxide absorbing liquid | | | | | | | | dT [MPa1/2] of the entire liquid medium | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | | | | | |
| Example A1 | Absorbing liquid A1 | (A1) | 30 | - | - | Dimethyl sulfoxide | 50 | N-Methyl-2-pyrrolidone | 20 | 25.61 | 20.7 | 17.4 | 0.84 | S |
| Example A2 | Absorbing liquid A2 | (A1) | 30 | - | - | Dimethyl sulfoxide | 35 | N-Methyl-2-pyrrolidone | 35 | 24.82 | 19.4 | 16.3 | 0.84 | S |
| Example A3 | Absorbing liquid A3 | (A1) | 30 | - | - | Dimethyl sulfoxide | 20 | N-Methyl-2-pyrrolidone | 50 | 24.02 | 20.6 | 16.9 | 0.82 | S |
| Example A4 | Absorbing liquid A4 | (A1) | 30 | - | - | Dimethyl sulfoxide | 10 | N-Methyl-2-pyrrolidone | 60 | 23.49 | 20.8 | 16.6 | 0.80 | S |
| Example A5 | Absorbing liquid A5 | (A2) | 30 | - | - | Dimethyl sulfoxide | 60 | N-Methyl-2-pyrrolidone | 10 | 26.14 | 20.7 | 17.2 | 0.83 | S |
| Example A6 | Absorbing liquid A6 | (A2) | 30 | - | - | Dimethyl sulfoxide | 50 | N-Methyl-2-pyrrolidone | 20 | 25.61 | 20.7 | 17.2 | 0.83 | S |
| Example A7 | Absorbing liquid A7 | (A2) | 30 | - | - | Dimethyl sulfoxide | 35 | N-Methyl-2-pyrrolidone | 35 | 24.82 | 20.6 | 17.1 | 0.83 | S |
| Example A8 | Absorbing liquid A8 | (A2) | 30 | - | - | Dimethyl sulfoxide | 20 | N-Methyl-2-pyrrolidone | 50 | 24.02 | 20.4 | 17.5 | 0.86 | S |
| Example A9 | Absorbing liquid A9 | (A2) | 30 | - | - | Dimethyl sulfoxide | 10 | N-Methyl-2-pyrrolidone | 60 | 23.49 | 20.6 | 17.1 | 0.83 | S |
| Example A10 | Absorbing liquid A10 | (A6) | 30 | - | - | Dimethyl sulfoxide | 60 | N-Methyl-2-pyrrolidone | 10 | 26.14 | 20.1 | 17.1 | 0.85 | S |
| Example A11 | Absorbing liquid A11 | (A6) | 30 | - | - | Dimethyl sulfoxide | 50 | N-Methyl-2-pyrrolidone | 20 | 25.61 | 19.5 | 16.2 | 0.83 | S |
| Example A12 | Absorbing liquid A12 | (A6) | 30 | - | - | Dimethyl sulfoxide | 35 | N-Methyl-2-pyrrolidone | 35 | 24.82 | 21.1 | 17.7 | 0.84 | S |
| Example A13 | Absorbing liquid A13 | (A6) | 30 | - | - | Dimethyl sulfoxide | 20 | N-Methyl-2-pyrrolidone | 50 | 24.02 | 20.3 | 17.3 | 0.85 | S |
| Example A14 | Absorbing liquid A14 | (A6) | 30 | - | - | Dimethyl sulfoxide | 10 | N-Methyl-2-pyrrolidone | 60 | 23.49 | 20 | 17.2 | 0.86 | S |
| Example A15 | Absorbing liquid A15 | (A7) | 30 | - | - | Dimethyl sulfoxide | 60 | N-Methyl-2-pyrrolidone | 10 | 26.14 | 20.7 | 17.4 | 0.84 | S |
| Example A16 | Absorbing liquid A16 | (A7) | 30 | - | - | Dimethyl sulfoxide | 50 | N-Methyl-2-pyrrolidone | 20 | 25.61 | 20.2 | 16.6 | 0.82 | S |
| Example A17 | Absorbing liquid A17 | (A7) | 30 | - | - | Dimethyl sulfoxide | 35 | N-Methyl-2-pyrrolidone | 35 | 24.82 | 20.5 | 17.2 | 0.84 | S |
| Example A18 | Absorbing liquid A18 | (A7) | 30 | - | - | Dimethyl sulfoxide | 20 | N-Methyl-2-pyrrolidone | 50 | 24.02 | 21.1 | 17.7 | 0.84 | S |
| Example A19 | Absorbing liquid A19 | (A7) | 30 | - | - | Dimethyl sulfoxide | 10 | N-Methyl-2-pyrrolidone | 60 | 23.49 | 19.8 | 16.2 | 0.82 | S |
| Example A20 | Absorbing liquid A20 | (A1) | 30 | - | - | Dimethyl sulfoxide | 50 | Methanol | 20 | 27.46 | 20.2 | 16.6 | 0.82 | S |
| Example A21 | Absorbing liquid A21 | (A1) | 30 | - | - | Dimethyl sulfoxide | 50 | Ethanol | 20 | 26.63 | 19.5 | 16 | 0.82 | S |
| Example A22 | Absorbing liquid A22 | (A1) | 30 | - | - | Dimethyl sulfoxide | 50 | 1-Propanol | 20 | 26.08 | 20.9 | 17.3 | 0.83 | S |
| Example A23 | Absorbing liquid A23 | (A1) | 30 | - | - | Dimethyl sulfoxide | 50 | 3-Methoxy-1-butanol | 20 | 25.10 | 21.3 | 17.5 | 0.82 | S |
| Example A24 | Absorbing liquid A24 | (A1) | 30 | - | - | Dimethyl sulfoxide | 50 | 1-Methoxy-2-propanol | 20 | 24.89 | 20.5 | 16.8 | 0.82 | S |
| Example A25 | Absorbing liquid A25 | (A1) | 30 | - | - | Dimethyl sulfoxide | 50 | Water | 20 | 32.71 | 21 | 17.4 | 0.83 | S |
| Example A26 | Absorbing liquid A26 | (A1) | 30 | - | - | Dimethyl sulfoxide | 50 | Sulfolane | 20 | 26.59 | 20.9 | 16.9 | 0.81 | S |
| Example A27 | Absorbing liquid A27 | (A1) | 30 | - | - | Dimethyl sulfoxide | 50 | Ethylene glycol monomethyl ether | 20 | 25.74 | 20.9 | 17.8 | 0.85 | S |
| Example A28 | Absorbing liquid A28 | (A1) | 30 | - | - | Methanol | 50 | Ethanol | 20 | 28.58 | 20.8 | 17.1 | 0.82 | S |
| Example A29 | Absorbing liquid A29 | (A1) | 30 | - | - | Methanol | 50 | 1-Propanol | 20 | 28.03 | 20.8 | 16.8 | 0.81 | S |
| Example A30 | Absorbing liquid A30 | (A1) | 30 | - | - | Methanol | 50 | Water | 20 | 34.66 | 20.7 | 17 | 0.82 | S |

[Table 8-3]

Table 8-3

| Example | Absorbing liquid | Composition of carbon dioxide absorbing liquid | | | | | | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | dT [MPa1/2] of the entire liquid medium | | | | | |
| Example A31 | Absorbing liquid A31 | (A1) | 30 | - | - | 1-propanol | 50 | 1-Methoxy-2-propanol | 20 | 23.41 | 19.6 | 16.3 | 0.83 | S |
| Example A32 | Absorbing liquid A32 | (A1) | 30 | - | - | 1-propanol | 50 | 3-Methoxy-1-butanol | 20 | 23.62 | 20.2 | 17.2 | 0.85 | S |
| Example A33 | Absorbing liquid A33 | (A1) | 30 | - | - | 1-propanol | 50 | Water | 20 | 31.23 | 20.7 | 16.8 | 0.81 | S |
| Example A34 | Absorbing liquid A34 | (A1) | 30 | - | - | Ethylene glycol monomethyl ether | 50 | Tetraethylene glycol dibutyl ether | 20 | 21.81 | 19 | 14.3 | 0.75 | A |
| Example A35 | Absorbing liquid A35 | (A1) | 30 | - | - | Tetraethylene glycol dibutyl ether | 50 | Sulfolane | 20 | 20.25 | 18.6 | 13.8 | 0.74 | A |
| Example A36 | Absorbing liquid A36 | (A2) | 30 | - | - | Dimethyl sulfoxide | 50 | Methanol | 20 | 27.46 | 20.1 | 16.9 | 0.84 | S |
| Example A37 | Absorbing liquid A37 | (A2) | 30 | - | - | Dimethyl sulfoxide | 50 | Ethanol | 20 | 26.63 | 21 | 17.4 | 0.83 | S |
| Example A38 | Absorbing liquid A38 | (A2) | 30 | - | - | Dimethyl sulfoxide | 50 | 1-propanol | 20 | 26.08 | 20.7 | 17.4 | 0.84 | S |
| Example A39 | Absorbing liquid A39 | (A2) | 30 | - | - | Dimethyl sulfoxide | 50 | 3-Methoxy-1-butanol | 20 | 25.10 | 20.2 | 16.6 | 0.82 | S |
| Example A40 | Absorbing liquid A40 | (A2) | 30 | - | - | Dimethyl sulfoxide | 50 | 1-Methoxy-2-propanol | 20 | 24.89 | 21.7 | 18.2 | 0.84 | S |
| Example A41 | Absorbing liquid A41 | (A2) | 30 | - | - | Dimethyl sulfoxide | 50 | Water | 20 | 32.71 | 20.3 | 16.8 | 0.83 | S |
| Example A42 | Absorbing liquid A42 | (A2) | 30 | - | - | Dimethyl sulfoxide | 50 | Sulfolane | 20 | 26.59 | 20.4 | 17.5 | 0.86 | S |
| Example A43 | Absorbing liquid A43 | (A2) | 30 | - | - | Dimethyl sulfoxide | 50 | Ethylene glycol monomethyl ether | 20 | 25.74 | 20.8 | 17.5 | 0.84 | S |
| Example A44 | Absorbing liquid A44 | (A2) | 30 | - | - | Methanol | 50 | Ethanol | 20 | 28.58 | 21.1 | 18.1 | 0.86 | S |
| Example A45 | Absorbing liquid A45 | (A2) | 30 | - | - | Methanol | 50 | 1-propanol | 20 | 28.03 | 20.3 | 17.1 | 0.84 | S |
| Example A46 | Absorbing liquid A46 | (A2) | 30 | - | - | Methanol | 50 | Water | 20 | 34.66 | 20.5 | 16.8 | 0.82 | S |
| Example A47 | Absorbing liquid A47 | (A2) | 30 | - | - | 1-propanol | 50 | 1-Methoxy-2-propanol | 20 | 23.41 | 20.6 | 17.3 | 0.84 | S |
| Example A48 | Absorbing liquid A48 | (A2) | 30 | - | - | 1-propanol | 50 | 3-Methoxy-1-butanol | 20 | 23.62 | 19.8 | 16.4 | 0.83 | S |
| Example A49 | Absorbing liquid A49 | (A2) | 30 | - | - | 1-propanol | 50 | Water | 20 | 31.23 | 20.4 | 16.7 | 0.82 | S |
| Example A50 | Absorbing liquid A50 | (A2) | 30 | - | - | Ethylene glycol monomethyl ether | 50 | Tetraethylene glycol dibutyl ether | 20 | 21.81 | 19.3 | 14.1 | 0.73 | A |
| Example A51 | Absorbing liquid A51 | (A2) | 30 | - | - | Tetraethylene glycol dibutyl ether | 50 | Sulfolane | 20 | 20.25 | 18.4 | 13.8 | 0.75 | A |
| Example A52 | Absorbing liquid A52 | (A6) | 30 | - | - | Dimethyl sulfoxide | 50 | Methanol | 20 | 27.46 | 20.3 | 16.8 | 0.83 | S |
| Example A53 | Absorbing liquid A53 | (A6) | 30 | - | - | Dimethyl sulfoxide | 50 | Ethanol | 20 | 26.63 | 20.9 | 17.1 | 0.82 | S |
| Example A54 | Absorbing liquid A54 | (A6) | 30 | - | - | Dimethyl sulfoxide | 50 | 1-propanol | 20 | 26.08 | 21.5 | 17.8 | 0.83 | S |
| Example A55 | Absorbing liquid A55 | (A6) | 30 | - | - | Dimethyl sulfoxide | 50 | 3-Methoxy-1-butanol | 20 | 25.10 | 20.7 | 17.2 | 0.83 | S |
| Example A56 | Absorbing liquid A56 | (A6) | 30 | - | - | Dimethyl sulfoxide | 50 | 1-Methoxy-2-propanol | 20 | 24.89 | 20.7 | 17 | 0.82 | S |
| Example A57 | Absorbing liquid A57 | (A6) | 30 | - | - | Dimethyl sulfoxide | 50 | Water | 20 | 32.71 | 21.5 | 17.6 | 0.82 | S |
| Example A58 | Absorbing liquid A58 | (A6) | 30 | - | - | Dimethyl sulfoxide | 50 | Sulfolane | 20 | 26.59 | 20 | 16.2 | 0.81 | S |
| Example A59 | Absorbing liquid A59 | (A6) | 30 | - | - | Dimethyl sulfoxide | 50 | Ethylene glycol monomethyl ether | 20 | 25.74 | 20.9 | 17.8 | 0.85 | S |
| Example A60 | Absorbing liquid A60 | (A6) | 30 | - | - | Methanol | 50 | Ethanol | 20 | 28.58 | 20.9 | 17.1 | 0.82 | S |

[Table 8-4]

Table 8-4

| Example | Absorbing liquid | Composition of carbon dioxide absorbing liquid | | | | | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | dT [MPa1/2] of the entire liquid medium | | | | |
| Example A61 | Absorbing liquid A61 | (A6) | 30 | - | - | Methanol | 50 | 1-propanol | 20 | 28.03 | 21.4 | 18 | 0.84 | S |
| Example A62 | Absorbing liquid A62 | (A6) | 30 | - | - | Methanol | 50 | Water | 20 | 34.66 | 20.1 | 17.3 | 0.86 | S |
| Example A63 | Absorbing liquid A63 | (A6) | 30 | - | - | 1-propanol | 50 | 1-Methoxy-2-propanol | 20 | 23.41 | 20.4 | 16.9 | 0.83 | S |
| Example A64 | Absorbing liquid A64 | (A6) | 30 | - | - | 1-propanol | 50 | 3-Methoxy-1-butanol | 20 | 23.62 | 20.1 | 16.9 | 0.84 | S |
| Example A65 | Absorbing liquid A65 | (A6) | 30 | - | - | 1-propanol | 50 | Water | 20 | 31.23 | 20.6 | 17.5 | 0.85 | S |
| Example A66 | Absorbing liquid A66 | (A6) | 30 | - | - | Ethylene glycol monomethyl ether | 50 | Tetraethylene glycol dibutyl ether | 20 | 21.81 | 18.9 | 13.8 | 0.73 | A |
| Example A67 | Absorbing liquid A67 | (A6) | 30 | - | - | Tetraethylene glycol dibutyl ether | 50 | Sulfolane | 20 | 20.25 | 18.9 | 14.4 | 0.76 | A |
| Example A68 | Absorbing liquid A68 | (A7) | 30 | - | - | Dimethyl sulfoxide | 50 | Methanol | 20 | 27.46 | 20.3 | 17.1 | 0.84 | S |
| Example A69 | Absorbing liquid A69 | (A7) | 30 | - | - | Dimethyl sulfoxide | 50 | Ethanol | 20 | 26.63 | 21.3 | 17.7 | 0.83 | S |
| Example A70 | Absorbing liquid A70 | (A7) | 30 | - | - | Dimethyl sulfoxide | 50 | 1-propanol | 20 | 26.08 | 19.8 | 16.4 | 0.83 | S |
| Example A71 | Absorbing liquid A71 | (A7) | 30 | - | - | Dimethyl sulfoxide | 50 | 3-Methoxy-1-butanol | 20 | 25.10 | 20.8 | 17.5 | 0.84 | S |
| Example A72 | Absorbing liquid A72 | (A7) | 30 | - | - | Dimethyl sulfoxide | 50 | 1-Methoxy-2-propanol | 20 | 24.89 | 19.7 | 16.2 | 0.82 | S |
| Example A73 | Absorbing liquid A73 | (A7) | 30 | - | - | Dimethyl sulfoxide | 50 | Water | 20 | 32.71 | 19.8 | 16.6 | 0.84 | S |
| Example A74 | Absorbing liquid A74 | (A7) | 30 | - | - | Dimethyl sulfoxide | 50 | Sulfolane | 20 | 26.59 | 20.4 | 16.9 | 0.83 | S |
| Example A75 | Absorbing liquid A75 | (A7) | 30 | - | - | Dimethyl sulfoxide | 50 | Ethylene glycol monomethyl ether | 20 | 25.74 | 20.2 | 17 | 0.84 | S |
| Example A76 | Absorbing liquid A76 | (A7) | 30 | - | - | Methanol | 50 | Ethanol | 20 | 28.58 | 21.3 | 17.5 | 0.82 | S |
| Example A77 | Absorbing liquid A77 | (A7) | 30 | - | - | Methanol | 50 | 1-propanol | 20 | 28.03 | 20.8 | 17.9 | 0.86 | S |
| Example A78 | Absorbing liquid A78 | (A7) | 30 | - | - | Methanol | 50 | Water | 20 | 34.66 | 20.5 | 17 | 0.83 | S |
| Example A79 | Absorbing liquid A79 | (A7) | 30 | - | - | 1-propanol | 50 | 1-Methoxy-2-propanol | 20 | 23.41 | 20 | 17 | 0.85 | S |
| Example A80 | Absorbing liquid A80 | (A7) | 30 | - | - | 1-propanol | 50 | 3-Methoxy-1-butanol | 20 | 23.62 | 20.8 | 17.7 | 0.85 | S |
| Example A81 | Absorbing liquid A81 | (A7) | 30 | - | - | 1-propanol | 50 | Water | 20 | 31.23 | 20.2 | 16.6 | 0.82 | S |
| Example A82 | Absorbing liquid A82 | (A7) | 30 | - | - | Ethylene glycol monomethyl ether | 50 | Tetraethylene glycol dibutyl ether | 20 | 21.81 | 19.2 | 14.4 | 0.75 | A |
| Example A83 | Absorbing liquid A83 | (A7) | 30 | - | - | Tetraethylene glycol dibutyl ether | 50 | Sulfolane | 20 | 20.25 | 19.6 | 14.5 | 0.74 | A |

[Examples 124 to 246, Comparative Examples 3 to 4, Reference Example 2]

(Degree of change in absorption amount after repeated absorption/release evaluation of carbon dioxide gas)

Using the absorbing liquids described in Table 7-1 to Table 7-5 and Table 8-1 to Table 8-4, the aforementioned carbon dioxide gas absorption/release measurement was repeated 10 times. Subsequently, the 11th absorption amount was calculated in the same manner as the first test, and the degree of decrease compared to the first absorption amount was evaluated. The evaluation criteria were as follows, with S, A, and B considered as the usable range. The evaluation results are shown in Table 9-1 to Table 9-2.

S: The 11th absorption amount is 99.5% or more compared to the first absorption amount
B: The 11th absorption amount is 99% or more and less than 99.5% compared to the first absorption amount
B: The 11th absorption amount is 98% or more and less than 99% compared to the first absorption amount
C: The 11th absorption amount is less than 98% compared to the first absorption amount

[Table 9-1]

Table 9-1

| Example | Absorbing liquid | Change in absorption amount in the 11th absorption/release test (compared to the 1st absorption amount) | Example | Absorbing liquid | Change in absorption amount in the 11th absorption/release test (compared to the 1st absorption amount) |
|---|---|---|---|---|---|
| Example 124 | Absorbing liquid 1 | S | Example 187 | Absorbing liquid 64 | S |
| Example 125 | Absorbing liquid 2 | S | Example 188 | Absorbing liquid 65 | S |
| Example 126 | Absorbing liquid 3 | A | Example 189 | Absorbing liquid 66 | A |
| Example 127 | Absorbing liquid 4 | A | Example 190 | Absorbing liquid 67 | A |
| Example 128 | Absorbing liquid 5 | A | Example 191 | Absorbing liquid 68 | A |
| Example 129 | Absorbing liquid 6 | A | Example 192 | Absorbing liquid 69 | A |
| Example 130 | Absorbing liquid 7 | A | Example 193 | Absorbing liquid 70 | A |
| Example 131 | Absorbing liquid 8 | A | Example 194 | Absorbing liquid 71 | A |
| Example 132 | Absorbing liquid 9 | A | Example 195 | Absorbing liquid 72 | A |
| Example 133 | Absorbing liquid 10 | A | Example 196 | Absorbing liquid 73 | A |
| Example 134 | Absorbing liquid 11 | B | Example 197 | Absorbing liquid 74 | A |
| Example 135 | Absorbing liquid 12 | A | Example 198 | Absorbing liquid 75 | A |
| Example 136 | Absorbing liquid 13 | A | Example 199 | Absorbing liquid 76 | A |
| Example 137 | Absorbing liquid 14 | A | Example 200 | Absorbing liquid 77 | A |
| Example 138 | Absorbing liquid 15 | A | Example 201 | Absorbing liquid 78 | S |
| Example 139 | Absorbing liquid 16 | A | Example 202 | Absorbing liquid 79 | S |
| Example 140 | Absorbing liquid 17 | A | Example 203 | Absorbing liquid 80 | S |
| Example 141 | Absorbing liquid 18 | A | Example 204 | Absorbing liquid 81 | S |
| Example 142 | Absorbing liquid 19 | A | Example 205 | Absorbing liquid 82 | S |
| Example 143 | Absorbing liquid 20 | A | Example 206 | Absorbing liquid 83 | S |
| Example 144 | Absorbing liquid 21 | B | Example 207 | Absorbing liquid 84 | S |
| Example 145 | Absorbing liquid 22 | A | Example 208 | Absorbing liquid 85 | S |
| Example 146 | Absorbing liquid 23 | B | Example 209 | Absorbing liquid 86 | S |
| Example 147 | Absorbing liquid 24 | S | Example 210 | Absorbing liquid 87 | S |
| Example 148 | Absorbing liquid 25 | S | Example 211 | Absorbing liquid 88 | S |
| Example 149 | Absorbing liquid 26 | S | Example 212 | Absorbing liquid 89 | A |
| Example 150 | Absorbing liquid 27 | S | Example 213 | Absorbing liquid 90 | A |
| Example 151 | Absorbing liquid 28 | S | Example 214 | Absorbing liquid 91 | A |
| Example 152 | Absorbing liquid 29 | S | Example 215 | Absorbing liquid 92 | A |
| Example 153 | Absorbing liquid 30 | S | Example 216 | Absorbing liquid 93 | A |
| Example 154 | Absorbing liquid 31 | B | Example 217 | Absorbing liquid 94 | A |
| Example 155 | Absorbing liquid 32 | S | Example 218 | Absorbing liquid 95 | A |
| Example 156 | Absorbing liquid 33 | S | Example 219 | Absorbing liquid 96 | A |
| Example 157 | Absorbing liquid 34 | S | Example 220 | Absorbing liquid 97 | A |
| Example 158 | Absorbing liquid 35 | S | Example 221 | Absorbing liquid 98 | A |
| Example 159 | Absorbing liquid 36 | S | Example 222 | Absorbing liquid 99 | A |
| Example 160 | Absorbing liquid 37 | S | Example 223 | Absorbing liquid 100 | A |
| Example 161 | Absorbing liquid 38 | S | Example 224 | Absorbing liquid 101 | S |
| Example 162 | Absorbing liquid 39 | S | Example 225 | Absorbing liquid 102 | S |
| Example 163 | Absorbing liquid 40 | S | Example 226 | Absorbing liquid 103 | S |
| Example 164 | Absorbing liquid 41 | B | Example 227 | Absorbing liquid 104 | S |
| Example 165 | Absorbing liquid 42 | S | Example 228 | Absorbing liquid 105 | S |
| Example 166 | Absorbing liquid 43 | A | Example 229 | Absorbing liquid 106 | S |
| Example 167 | Absorbing liquid 44 | A | Example 230 | Absorbing liquid 107 | S |
| Example 168 | Absorbing liquid 45 | A | Example 231 | Absorbing liquid 108 | S |
| Example 169 | Absorbing liquid 46 | A | Example 232 | Absorbing liquid 109 | S |
| Example 170 | Absorbing liquid 47 | A | Example 233 | Absorbing liquid 110 | S |
| Example 171 | Absorbing liquid 48 | A | Example 234 | Absorbing liquid 111 | S |
| Example 172 | Absorbing liquid 49 | A | Example 235 | Absorbing liquid 112 | A |
| Example 173 | Absorbing liquid 50 | A | Example 236 | Absorbing liquid 113 | A |
| Example 174 | Absorbing liquid 51 | A | Example 237 | Absorbing liquid 114 | A |
| Example 175 | Absorbing liquid 52 | A | Example 238 | Absorbing liquid 115 | A |
| Example 176 | Absorbing liquid 53 | A | Example 239 | Absorbing liquid 116 | A |
| Example 177 | Absorbing liquid 54 | A | Example 240 | Absorbing liquid 117 | A |
| Example 178 | Absorbing liquid 55 | S | Example 241 | Absorbing liquid 118 | A |
| Example 179 | Absorbing liquid 56 | S | Example 242 | Absorbing liquid 119 | A |
| Example 180 | Absorbing liquid 57 | S | Example 243 | Absorbing liquid 120 | A |
| Example 181 | Absorbing liquid 58 | S | Example 244 | Absorbing liquid 121 | A |
| Example 182 | Absorbing liquid 59 | S | Example 245 | Absorbing liquid 122 | A |
| Example 183 | Absorbing liquid 60 | S | Example 246 | Absorbing liquid 123 | A |
| Example 184 | Absorbing liquid 61 | S | Comparative Example 3 | Absorbing liquid 124 | C |
| Example 185 | Absorbing liquid 62 | S | Comparative Example 4 | Absorbing liquid 125 | C |
| Example 186 | Absorbing liquid 63 | S | Reference Example 2 | Absorbing liquid 126 | B |

[Table 9-2]

Table 9-2

| Example | Absorbing liquid | Change in absorption amount in the 11th absorption/release test (compared to the 1st absorption amount) |
|---|---|---|
| Example F1 | Absorbing liquid E1 | S |
| Example F2 | Absorbing liquid E2 | S |
| Example F3 | Absorbing liquid E3 | S |
| Example F4 | Absorbing liquid E4 | A |
| Example F5 | Absorbing liquid E5 | A |
| Example F6 | Absorbing liquid E6 | A |
| Example F7 | Absorbing liquid E7 | A |
| Example F8 | Absorbing liquid E8 | A |
| Example F9 | Absorbing liquid E9 | A |
| Example F10 | Absorbing liquid E10 | A |
| Example F11 | Absorbing liquid E11 | S |
| Example F12 | Absorbing liquid E12 | S |
| Example F13 | Absorbing liquid E13 | S |
| Example F14 | Absorbing liquid E14 | S |
| Example F15 | Absorbing liquid E15 | S |
| Example F16 | Absorbing liquid E16 | S |
| Example F17 | Absorbing liquid E17 | S |
| Example F18 | Absorbing liquid E18 | S |
| Example F19 | Absorbing liquid E19 | S |
| Example F20 | Absorbing liquid E20 | S |
| Example F21 | Absorbing liquid A1 | A |
| Example F22 | Absorbing liquid A2 | A |
| Example F23 | Absorbing liquid A3 | A |
| Example F24 | Absorbing liquid A4 | A |
| Example F25 | Absorbing liquid A5 | A |
| Example F26 | Absorbing liquid A6 | A |
| Example F27 | Absorbing liquid A7 | A |
| Example F28 | Absorbing liquid A8 | A |
| Example F29 | Absorbing liquid A9 | A |
| Example F30 | Absorbing liquid A10 | A |
| Example F31 | Absorbing liquid A11 | A |
| Example F32 | Absorbing liquid A12 | A |
| Example F33 | Absorbing liquid A13 | A |
| Example F34 | Absorbing liquid A14 | A |
| Example F35 | Absorbing liquid A15 | A |
| Example F36 | Absorbing liquid A16 | A |
| Example F37 | Absorbing liquid A17 | A |
| Example F38 | Absorbing liquid A18 | A |
| Example F39 | Absorbing liquid A19 | A |
| Example F40 | Absorbing liquid A20 | S |
| Example F41 | Absorbing liquid A21 | S |
| Example F42 | Absorbing liquid A22 | S |
| Example F43 | Absorbing liquid A23 | S |
| Example F44 | Absorbing liquid A24 | S |
| Example F45 | Absorbing liquid A25 | S |
| Example F46 | Absorbing liquid A26 | A |
| Example F47 | Absorbing liquid A27 | A |
| Example F48 | Absorbing liquid A28 | S |
| Example F49 | Absorbing liquid A29 | S |
| Example F50 | Absorbing liquid A30 | S |
| Example F51 | Absorbing liquid A31 | S |
| Example F52 | Absorbing liquid A32 | S |

| Example | Absorbing liquid | Change in absorption amount in the 11th absorption/release test (compared to the 1st absorption amount) |
|---|---|---|
| Example F53 | Absorbing liquid A33 | A |
| Example F54 | Absorbing liquid A34 | A |
| Example F55 | Absorbing liquid A35 | A |
| Example F56 | Absorbing liquid A36 | S |
| Example F57 | Absorbing liquid A37 | S |
| Example F58 | Absorbing liquid A38 | S |
| Example F59 | Absorbing liquid A39 | S |
| Example F60 | Absorbing liquid A40 | S |
| Example F61 | Absorbing liquid A41 | S |
| Example F62 | Absorbing liquid A42 | A |
| Example F63 | Absorbing liquid A43 | A |
| Example F64 | Absorbing liquid A44 | S |
| Example F65 | Absorbing liquid A45 | S |
| Example F66 | Absorbing liquid A46 | S |
| Example F67 | Absorbing liquid A47 | S |
| Example F68 | Absorbing liquid A48 | S |
| Example F69 | Absorbing liquid A49 | A |
| Example F70 | Absorbing liquid A50 | A |
| Example F71 | Absorbing liquid A51 | A |
| Example F72 | Absorbing liquid A52 | S |
| Example F73 | Absorbing liquid A53 | S |
| Example F74 | Absorbing liquid A54 | S |
| Example F75 | Absorbing liquid A55 | S |
| Example F76 | Absorbing liquid A56 | S |
| Example F77 | Absorbing liquid A57 | S |
| Example F78 | Absorbing liquid A58 | A |
| Example F79 | Absorbing liquid A59 | A |
| Example F80 | Absorbing liquid A60 | S |
| Example F81 | Absorbing liquid A61 | S |
| Example F82 | Absorbing liquid A62 | S |
| Example F83 | Absorbing liquid A63 | S |
| Example F84 | Absorbing liquid A64 | S |
| Example F85 | Absorbing liquid A65 | A |
| Example F86 | Absorbing liquid A66 | A |
| Example F87 | Absorbing liquid A67 | A |
| Example F88 | Absorbing liquid A68 | S |
| Example F89 | Absorbing liquid A69 | S |
| Example F90 | Absorbing liquid A70 | S |
| Example F91 | Absorbing liquid A71 | S |
| Example F92 | Absorbing liquid A72 | S |
| Example F93 | Absorbing liquid A73 | S |
| Example F94 | Absorbing liquid A74 | A |
| Example F95 | Absorbing liquid A75 | A |
| Example F96 | Absorbing liquid A76 | S |
| Example F97 | Absorbing liquid A77 | S |
| Example F98 | Absorbing liquid A78 | S |
| Example F99 | Absorbing liquid A79 | S |
| Example F100 | Absorbing liquid A80 | S |
| Example F101 | Absorbing liquid A81 | A |
| Example F102 | Absorbing liquid A82 | A |
| Example F103 | Absorbing liquid A83 | A |

[Examples M1 to M78, Comparative Example 5, Reference Example 3]

(Absorption rate of carbon dioxide gas)

The absorption rate evaluation was performed using absorbing liquids 1, 12, 22, 32, 43 to 116, 125, and 126 described in Table 7-1 to Table 7-5 and Table 8-1. 100 g of the prepared carbon dioxide absorbing liquid (in a state placed in a 200 ml gas absorption bottle) was temperature-adjusted to 25°C in a water bath. A mixed gas (500 ml/min) of carbon dioxide gas at 100 ml/min and nitrogen gas at 400 ml/min was bubbled and blown into this carbon dioxide absorbing liquid. The absorption amount of carbon dioxide gas at this time (carbon dioxide absorption amount for 10 minutes (L)) was measured using a gas flow meter and a carbon dioxide concentration meter. The value obtained by dividing this absorption amount by 10 was used as the absorption rate (L/min).

The evaluation criteria were as follows, with S, A, and B considered as the usable range. The evaluation results

are shown in Table 10.

S: Absorption rate of 1.00 L/min or more
A: Absorption rate of 0.75 L/min or more and less than 1.00 L/min
B: Absorption rate of 0.50 L/min or more and less than 0.75 L/min
C: Absorption rate less than 0.50 L/min

[Table 10]

Table 10

| Example | Amine compound | Absorbing liquid | Absorption rate |
|---|---|---|---|
| Example M1 | (A1) | Absorbing liquid 1 | A |
| Example M2 | (A2) | Absorbing liquid 12 | A |
| Example M3 | (A3) | Absorbing liquid 43 | A |
| Example M4 | (A4) | Absorbing liquid 44 | A |
| Example M5 | (A5) | Absorbing liquid 45 | A |
| Example M6 | (A6) | Absorbing liquid 22 | A |
| Example M7 | (A7) | Absorbing liquid 32 | A |
| Example M8 | (A8) | Absorbing liquid 46 | A |
| Example M9 | (A9) | Absorbing liquid 47 | A |
| Example M10 | (A10) | Absorbing liquid 48 | A |
| Example M10 | (A11) | Absorbing liquid 49 | A |
| Example M12 | (A12) | Absorbing liquid 50 | A |
| Example M13 | (A13) | Absorbing liquid 51 | A |
| Example M14 | (A14) | Absorbing liquid 52 | A |
| Example M15 | (A15) | Absorbing liquid 53 | A |
| Example M16 | (A16) | Absorbing liquid 54 | A |
| Example M17 | (A17) | Absorbing liquid 55 | A |
| Example M18 | (A18) | Absorbing liquid 56 | A |
| Example M19 | (A19) | Absorbing liquid 57 | A |
| Example M20 | (A20) | Absorbing liquid 58 | A |
| Example M21 | (A21) | Absorbing liquid 59 | A |
| Example M22 | (A22) | Absorbing liquid 60 | A |
| Example M23 | (A23) | Absorbing liquid 61 | A |
| Example M24 | (A24) | Absorbing liquid 62 | A |
| Example M25 | (A25) | Absorbing liquid 63 | A |
| Example M26 | (A26) | Absorbing liquid 64 | A |
| Example M27 | (A27) | Absorbing liquid 65 | A |
| Example M28 | (A28) | Absorbing liquid 66 | A |
| Example M29 | (A29) | Absorbing liquid 67 | A |
| Example M30 | (A30) | Absorbing liquid 68 | A |
| Example M31 | (A31) | Absorbing liquid 69 | A |
| Example M32 | (A32) | Absorbing liquid 70 | A |
| Example M33 | (A33) | Absorbing liquid 71 | A |
| Example M34 | (A34) | Absorbing liquid 72 | A |
| Example M35 | (A35) | Absorbing liquid 73 | A |
| Example M36 | (A36) | Absorbing liquid 74 | A |
| Example M37 | (A37) | Absorbing liquid 75 | A |
| Example M38 | (A38) | Absorbing liquid 76 | A |
| Example M39 | (A39) | Absorbing liquid 77 | A |
| Example M40 | (A40) | Absorbing liquid 78 | A |
| Example M41 | (A41) | Absorbing liquid 79 | A |
| Example M42 | (A42) | Absorbing liquid 80 | A |
| Example M43 | (A43) | Absorbing liquid 81 | A |
| Example M44 | (A44) | Absorbing liquid 82 | A |
| Example M45 | (A45) | Absorbing liquid 83 | A |
| Example M46 | (A46) | Absorbing liquid 84 | A |
| Example M47 | (A47) | Absorbing liquid 85 | A |
| Example M48 | (A48) | Absorbing liquid 86 | A |
| Example M49 | (A49) | Absorbing liquid 87 | A |
| Example M50 | (A50) | Absorbing liquid 88 | A |
| Example M51 | (A51) | Absorbing liquid 89 | A |
| Example M52 | (A52) | Absorbing liquid 90 | A |
| Example M53 | (A53) | Absorbing liquid 91 | A |
| Example M54 | (A54) | Absorbing liquid 92 | A |
| Example M55 | (A55) | Absorbing liquid 93 | A |
| Example M56 | (A56) | Absorbing liquid 94 | A |
| Example M57 | (A57) | Absorbing liquid 95 | A |
| Example M58 | (A58) | Absorbing liquid 96 | A |
| Example M59 | (A59) | Absorbing liquid 97 | A |
| Example M60 | (A60) | Absorbing liquid 98 | A |
| Example M61 | (A61) | Absorbing liquid 99 | A |
| Example M62 | (A62) | Absorbing liquid 100 | A |
| Example M63 | (A63) | Absorbing liquid 101 | A |
| Example M64 | (A64) | Absorbing liquid 102 | A |
| Example M65 | (A65) | Absorbing liquid 103 | A |
| Example M66 | (A66) | Absorbing liquid 104 | A |
| Example M67 | (A67) | Absorbing liquid 105 | A |
| Example M68 | (A68) | Absorbing liquid 106 | A |
| Example M69 | (A69) | Absorbing liquid 107 | A |
| Example M70 | (A70) | Absorbing liquid 108 | A |
| Example M71 | (A71) | Absorbing liquid 109 | A |
| Example M72 | (A72) | Absorbing liquid 110 | A |
| Example M73 | (A73) | Absorbing liquid 111 | A |
| Example M74 | (A74) | Absorbing liquid 112 | A |
| Example M75 | (A75) | Absorbing liquid 113 | A |
| Example M76 | (A76) | Absorbing liquid 114 | A |
| Example M77 | (A77) | Absorbing liquid 115 | A |
| Example M78 | (A78) | Absorbing liquid 116 | A |
| Comparative Example 5 | MEA | Absorbing liquid 125 | C |
| Reference Example 3 | ATMP | Absorbing liquid 126 | C |

As described in the above examples, the carbon dioxide absorbing liquid of the disclosure exhibits superior effects in terms of carbon dioxide release rate and release efficiency (release amount/absorption amount) compared to conventional carbon carbon dioxide absorbing liquids. Moreover, in contrast to the generally known release temperature of 120°C for MEA aqueous solution, it was found that the absorbing liquid of the disclosure may efficiently release carbon dioxide at a release temperature of 60°C under the experimental conditions used in this study.

2. Example Group 2

[Synthesis Example 79]

Synthesis method of Compound (A79)

[Chemical formula 11]

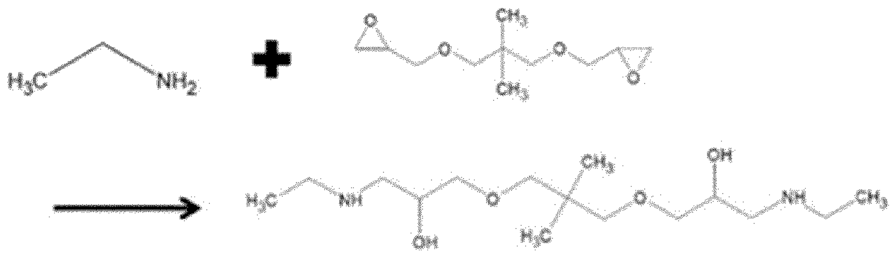

Under a nitrogen atmosphere, 30.0 g (665.5 mmol) of ethylamine (b1) mixed with 300 ml of methanol and 72.0 g (332.7 mmol) of neopentyl glycol glycidyl ether (c7) were added and stirred. After addition, the mixture was stirred at room temperature for 24 hours, and the progress of the reaction was confirmed by the absence of the reactant (b1) detected by TOF-MS. The solvent methanol was removed by reducing pressure at 40°C or below, and the target compound (A79) was obtained.

(TOF-MS Measurement Result)

Calculated molecular weight: C15H34N2 O4, Mol. Wt. 306.4;
Observed molecular weight: m/z 306.5

[Synthesis Examples 80 to 122]

Compounds (A80) to (A128) were synthesized by appropriately combining the amine compounds in the aforementioned Table 2 and the epoxy compounds in Table 3-1 to Table 3-5, in the same manner as in Synthesis Example 79. The obtained compounds were identified by TOF-MS in the same manner as in Synthesis Example 79. The yields of the synthesized compounds and the mass spectrum results are shown in Table 11. It is noted that the compound numbers are the same as those described in Table 1-6 to Table 1-8 of this specification.

[Table 11]

Table 11

| Synthesis example | Compound | TOF-MS measured value | Theoretical value | Synthesis example | Compound | TOF-MS measured value | Theoretical value |
|---|---|---|---|---|---|---|---|
| 79 | (A79) | 306.5 | 306.4 | 104 | (A104) | 204.4 | 204.3 |
| 80 | (A80) | 336.7 | 336.5 | 105 | (A105) | 218.4 | 218.3 |
| 81 | (A81) | 338.5 | 338.4 | 106 | (A106) | 218.3 | 218.3 |
| 82 | (A82) | 521.0 | 520.8 | 107 | (A107) | 191.3 | 191.3 |
| 83 | (A83) | 370.7 | 370.6 | 108 | (A108) | 203.4 | 203.3 |
| 84 | (A84) | 432.8 | 432.6 | 109 | (A109) | 259.4 | 259.4 |
| 85 | (A85) | 637.1 | 637.0 | 110 | (A110) | 320.4 | 320.5 |
| 86 | (A86) | 294.6 | 294.4 | 111 | (A111) | 147.2 | 147.2 |
| 87 | (A87) | 522.7 | 522.6 | 112 | (A112) | 234.4 | 234.3 |
| 88 | (A88) | 236.5 | 236.3 | 113 | (A113) | 238.3 | 238.3 |
| 89 | (A89) | 252.4 | 252.3 | 114 | (A114) | 274.4 | 274.5 |
| 90 | (A90) | 342.6 | 342.4 | 115 | (A115) | 259.5 | 259.4 |
| 91 | (A91) | 568.9 | 568.8 | 116 | (A116) | 204.4 | 204.3 |
| 92 | (A92) | 438.7 | 438.5 | 117 | (A117) | 315.6 | 315.5 |
| 93 | (A93) | 311.4 | 311.3 | 118 | (A118) | 260.4 | 260.4 |
| 94 | (A94) | 855.3 | 855.1 | 119 | (A119) | 234.3 | 234.3 |
| 95 | (A95) | 364.5 | 364.5 | 120 | (A120) | 477.6 | 477.6 |
| 96 | (A96) | 392.6 | 392.6 | 121 | (A121) | 233.4 | 233.4 |
| 97 | (A97) | 420.7 | 420.6 | 122 | (A122) | 274.4 | 274.4 |
| 98 | (A98) | 366.4 | 366.5 | 123 | (A123) | 205.1 | 205.3 |
| 99 | (A99) | 422.6 | 422.6 | 124 | (A124) | 147.9 | 148.2 |
| 100 | (A100) | 422.7 | 422.6 | 125 | (A125) | 177.0 | 177.2 |
| 101 | (A101) | 474.3 | 474.7 | 126 | (A126) | 219.5 | 219.3 |
| 102 | (A102) | 394.5 | 394.6 | 127 | (A127) | 290.1 | 290.4 |
| 103 | (A103) | 250.4 | 250.3 | 128 | (A128) | 232.6 | 232.4 |

(Measurement method for release efficiency of carbon dioxide gas)

The carbon dioxide absorbing liquid (100 g) (in a state placed in a 200 ml gas absorption bottle) adjusted in the later-described examples was temperature-adjusted to 25°C in a water bath. A mixed gas (500 ml/min) consisting of carbon dioxide gas at 100 ml/min and nitrogen gas at 400 ml/min was bubbled through this carbon dioxide absorbing liquid for 1 hour. The absorption amount of carbon dioxide gas (carbon dioxide absorption amount for 1 hour (L)) during this process was measured using a gas flow meter and a carbon dioxide concentration meter. Using this carbon dioxide absorption amount for 1 hour (L), the carbon dioxide absorption amount (L) per 1 kg of carbon dioxide absorbing liquid was calculated.

Next, this carbon dioxide absorbing liquid was temperature-adjusted to 60°C in a water bath. Nitrogen gas at 500 ml/min was bubbled through this carbon dioxide absorbing liquid for 2 hours. The release amount of carbon dioxide gas (carbon dioxide release amount for 2 hours (L)) during this process was measured using a gas flow meter and a carbon dioxide concentration meter. Using this carbon dioxide release amount for 2 hours (L), the carbon dioxide release amount (L) per 1 kg of carbon dioxide absorbing liquid was calculated.

From the aforementioned carbon dioxide release amount for 2 hours (L) and the aforementioned carbon dioxide absorption amount for 1 hour (L), the carbon dioxide gas release efficiency (= carbon dioxide release amount for 2 hours (L) ÷ carbon dioxide absorption amount for 1 hour (L)) was calculated.

Based on the measurement of the calculated carbon dioxide gas release efficiency, the following criteria were established for evaluation, with S, A, and B considered as the usable range. The evaluation results are shown in Table 12-1 to Table 12-7.

S: Release efficiency of 0.8 or higher
A: Release efficiency of 0.7 or higher and less than 0.8
B: Release efficiency of 0.6 or higher and less than 0.7
C: Release efficiency less than 0.6

[Materials and gas types used for evaluation]

The following abbreviations were used to simplify the notation.

ATMP: 4-amino-2,2,6,6-tetramethylpiperidine
MDEA: N-methyldiethanolamine
MBZA: methylbenzylamine
MEA: monoethanolamine
AB: 2-aminobutanol
DMAPA: dimethylaminopropylamine

The gas type used for the evaluation is the same as that in Table 6 described above.

[Example B1 (absorbing liquid B1)]

30 g of the compound (A79) obtained in Synthesis Example 79 was mixed and stirred with 70 g of dimethyl sulfoxide to prepare a carbon dioxide absorbing liquid (100 g). This liquid was placed in a 200 mL gas absorption bottle, and the release efficiency of carbon dioxide gas was measured.

The absorption amount of carbon dioxide for 1 hour (L) was 2.18 L when converted to standard state. In other words, the absorption amount of carbon dioxide per 1 kg of carbon dioxide absorbing liquid for 1 hour (L) was 21.8 L at standard state. (The carbon dioxide absorption amount per 1 kg of carbon dioxide absorbing liquid per minute (mL/min) was 363 mL/min (= 21.8 [L/hour] $\times$ 1000 [mL/L] $\div$ 60 [min/hour]).

The carbon dioxide release amount for 2 hours (L) was 1.80 L when converted to standard state. In other words, the carbon dioxide release amount per 1 kg of carbon dioxide absorbing liquid for 2 hours (L) was 18.0 L when converted to standard state.

(The carbon dioxide release amount per 1 kg of carbon dioxide absorbing liquid per minute (mL/min) was 150 mL/min (= 18.0 [L/2 hours] $\times$ 1000 [mL/L] $\div$ 120 [min/2 hours]).

From these results, the release efficiency of carbon dioxide gas was 0.83. The above results are shown in Table 12-1.

[Example B2 (absorbing liquid B2)]

The carbon dioxide absorbing liquid (100 g) was prepared in the same manner by changing 30 g of compound (1) to 15 g and changing dimethyl sulfoxide from 70 g to 85 g. This liquid was placed in a 200 mL gas absorption bottle, and the release efficiency of carbon dioxide gas was measured. The evaluation results are shown in Table 12-1.

[Examples B3 to B54, E21 to E40, J1 to J165 (absorbing liquids B3 to B54, E21 to E40, J1 to J165)]

The compound and liquid medium described in Example B1 were changed to the compounds and liquid media shown in Table 12-1 to Table 12-7, respectively, and similar experiments were conducted. The evaluation results are shown in Table 12-1 to Table 12-7.

[Table 12-1]

Table 12-1

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid medium | dT of liquid medium [MPa1/2] | Liquid medium /wt% | | | | |
| Example B1 | Absorbing liquid B1 | (A84) | 30 | Dimethyl sulfoxide | 26.68 | 70 | 21.8 | 17.6 | 0.81 | S |
| Example B2 | Absorbing liquid B2 | | 15 | Dimethyl sulfoxide | 26.68 | 85 | 12.0 | 9.6 | 0.80 | S |
| Example B3 | Absorbing liquid B3 | | 30 | N-Methyl-2-pyrrolidone | 22.96 | 70 | 18.5 | 13.4 | 0.72 | A |
| Example B4 | Absorbing liquid B4 | | 30 | Diethylene glycol dibutyl ether | 17.00 | 70 | 18.2 | 13.2 | 0.73 | A |
| Example B5 | Absorbing liquid B5 | | 30 | Triethylene glycol dibutyl ether | 17.20 | 70 | 18.5 | 13.4 | 0.72 | A |
| Example B6 | Absorbing liquid B6 | | 30 | Tetraethylene glycol dibutyl ether | 17.80 | 70 | 18.7 | 13.6 | 0.73 | A |
| Example B7 | Absorbing liquid B7 | | 30 | Propylene glycol monopropyl ether | 20.00 | 70 | 19.5 | 13.8 | 0.71 | A |
| Example B8 | Absorbing liquid B8 | | 30 | Ethylene glycol monobutyl ether | 20.82 | 70 | 17.2 | 12.7 | 0.74 | A |
| Example B9 | Absorbing liquid B9 | | 30 | Ethylene glycol monomethyl ether | 23.41 | 70 | 20.0 | 16.2 | 0.81 | S |
| Example B10 | Absorbing liquid B10 | | 30 | Sulfolane | 26.37 | 70 | 20.5 | 16.4 | 0.80 | S |
| Example B11 | Absorbing liquid B11 | | 30 | Water | 47.81 | 70 | 22.0 | 13.5 | 0.61 | B |
| Example E21 | Absorbing liquid E21 | | 30 | Methanol | 29.41 | 70 | 19.2 | 15.6 | 0.81 | S |
| Example E22 | Absorbing liquid E22 | | 30 | Ethanol | 26.52 | 70 | 20.3 | 16.8 | 0.83 | S |
| Example E23 | Absorbing liquid E23 | | 30 | 1-propanol | 24.60 | 70 | 19.5 | 15.6 | 0.80 | S |
| Example E24 | Absorbing liquid E24 | | 30 | 3-Methoxy-1-butanol | 21.17 | 70 | 18.9 | 14.0 | 0.74 | A |
| Example E25 | Absorbing liquid E25 | | 30 | 1-Methoxy-2-propanol | 20.44 | 70 | 20.7 | 15.5 | 0.75 | A |
| Example B12 | Absorbing liquid B12 | (A85) | 30 | Dimethyl sulfoxide | 26.68 | 70 | 21.6 | 17.7 | 0.82 | S |
| Example B13 | Absorbing liquid B13 | | 30 | N-Methyl-2-pyrrolidone | 22.96 | 70 | 19.2 | 14.9 | 0.78 | A |
| Example B14 | Absorbing liquid B14 | | 30 | Diethylene glycol dibutyl ether | 17.00 | 70 | 18.7 | 12.4 | 0.66 | B |
| Example B15 | Absorbing liquid B15 | | 30 | Triethylene glycol dibutyl ether | 17.20 | 70 | 18.4 | 13.2 | 0.72 | A |
| Example B16 | Absorbing liquid B16 | | 30 | Tetraethylene glycol dibutyl ether | 17.80 | 70 | 17.9 | 13.8 | 0.77 | A |
| Example B17 | Absorbing liquid B17 | | 30 | Propylene glycol monopropyl ether | 20.00 | 70 | 19.2 | 13.5 | 0.70 | A |
| Example B18 | Absorbing liquid B18 | | 30 | Ethylene glycol monobutyl ether | 20.82 | 70 | 19.3 | 13.8 | 0.72 | A |
| Example B19 | Absorbing liquid B19 | | 30 | Ethylene glycol monomethyl ether | 23.41 | 70 | 19.9 | 16.1 | 0.81 | S |
| Example B20 | Absorbing liquid B20 | | 30 | Sulfolane | 26.37 | 70 | 20.5 | 16.4 | 0.80 | S |
| Example B21 | Absorbing liquid B21 | | 30 | Water | 47.81 | 70 | 21.0 | 12.9 | 0.61 | B |
| Example E26 | Absorbing liquid E26 | | 30 | Methanol | 29.41 | 70 | 21.2 | 17.2 | 0.81 | S |
| Example E27 | Absorbing liquid E27 | | 30 | Ethanol | 26.52 | 70 | 20.4 | 16.3 | 0.80 | S |
| Example E28 | Absorbing liquid E28 | | 30 | 1-propanol | 24.60 | 70 | 20.3 | 16.8 | 0.83 | S |
| Example E29 | Absorbing liquid E29 | | 30 | 3-Methoxy-1-butanol | 21.17 | 70 | 19.0 | 14.1 | 0.74 | A |
| Example E30 | Absorbing liquid E30 | | 30 | 1-Methoxy-2-propanol | 20.44 | 70 | 19.4 | 14.6 | 0.75 | A |
| Example B22 | Absorbing liquid B22 | (A91) | 30 | Dimethyl sulfoxide | 26.68 | 70 | 20.5 | 16.6 | 0.81 | S |
| Example B23 | Absorbing liquid B23 | | 30 | Methylcyclohexane | 16.03 | 70 | 17.4 | 10.5 | 0.60 | B |
| Example B24 | Absorbing liquid B24 | | 30 | Diethylene glycol dibutyl ether | 17.00 | 70 | 15.4 | 11.2 | 0.73 | A |
| Example B25 | Absorbing liquid B25 | | 30 | Triethylene glycol dibutyl ether | 17.20 | 70 | 15.8 | 11.3 | 0.72 | A |
| Example B26 | Absorbing liquid B26 | | 30 | Tetraethylene glycol dibutyl ether | 17.80 | 70 | 16.1 | 11.5 | 0.71 | A |
| Example B27 | Absorbing liquid B27 | | 30 | Propylene glycol monopropyl ether | 20.00 | 70 | 16.9 | 12.4 | 0.73 | A |

[Table 12-2]

Table 12-2

| Example | Absorbing liquid | Composition of carbon dioxide absorbing liquid: Amine compound | Amine compound /wt% | Liquid medium | dT of liquid medium [MPa1/2] | Liquid medium /wt% | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| Example B28 | Absorbing liquid B28 | '(A91) | 30 | Ethylene glycol monobutyl ether | 20.82 | 70 | 18.7 | 13.2 | 0.71 | A |
| Example B29 | Absorbing liquid B29 | | 30 | Ethylene glycol monomethyl ether | 23.41 | 70 | 18.8 | 15.4 | 0.82 | S |
| Example B30 | Absorbing liquid B30 | | 30 | Sulfolane | 26.37 | 70 | 18.9 | 15.1 | 0.80 | S |
| Example B31 | Absorbing liquid B31 | | 30 | Water | 47.81 | 70 | 20.5 | 12.7 | 0.62 | B |
| Example E31 | Absorbing liquid E31 | | 30 | Methanol | 29.41 | 70 | 21.6 | 17.5 | 0.81 | S |
| Example E32 | Absorbing liquid E32 | | 30 | Ethanol | 26.52 | 70 | 21.3 | 17.5 | 0.82 | S |
| Example E33 | Absorbing liquid E33 | | 30 | 1-propanol | 24.60 | 70 | 20.0 | 16.4 | 0.82 | S |
| Example E34 | Absorbing liquid E34 | | 30 | 3-Methoxy-1-butanol | 21.17 | 70 | 20.1 | 15.5 | 0.77 | A |
| Example E35 | Absorbing liquid E35 | | 30 | 1-Methoxy-2-propanol | 20.44 | 70 | 19.0 | 15.0 | 0.79 | A |
| Example B32 | Absorbing liquid B32 | (A92) | 30 | Dimethyl sulfoxide | 26.68 | 70 | 21.7 | 17.6 | 0.81 | S |
| Example B33 | Absorbing liquid B33 | | 30 | N-Methyl-2-pyrrolidone | 22.96 | 70 | 18.2 | 13.5 | 0.74 | A |
| Example B34 | Absorbing liquid B34 | | 30 | Diethylene glycol dibutyl ether | 17.00 | 70 | 16.8 | 12.9 | 0.77 | A |
| Example B35 | Absorbing liquid B35 | | 30 | Triethylene glycol dibutyl ether | 17.20 | 70 | 16.6 | 12.1 | 0.73 | A |
| Example B36 | Absorbing liquid B36 | | 30 | Tetraethylene glycol dibutyl ether | 17.80 | 70 | 15.9 | 11.2 | 0.70 | A |
| Example B37 | Absorbing liquid B37 | | 30 | Propylene glycol monopropyl ether | 20.00 | 70 | 17.9 | 12.9 | 0.72 | A |
| Example B38 | Absorbing liquid B38 | | 30 | Ethylene glycol monobutyl ether | 20.82 | 70 | 18.4 | 13.8 | 0.75 | A |
| Example B39 | Absorbing liquid B39 | | 30 | Ethylene glycol monomethyl ether | 23.41 | 70 | 18.9 | 15.1 | 0.80 | S |
| Example B40 | Absorbing liquid B40 | | 30 | Sulfolane | 26.37 | 70 | 19.8 | 16.2 | 0.82 | S |
| Example B41 | Absorbing liquid B41 | | 30 | Water | 47.81 | 70 | 21.4 | 13.0 | 0.61 | B |
| Example B42 | Absorbing liquid B42 | | 30 | 1-Ethyl-3-methylimidazolium methyl phosphite | - | 70 | 20.1 | 16.1 | 0.80 | S |
| Example E36 | Absorbing liquid E36 | | 30 | Methanol | 29.41 | 70 | 21.3 | 17.3 | 0.81 | S |
| Example E37 | Absorbing liquid E37 | | 30 | Ethanol | 26.52 | 70 | 20.8 | 17.3 | 0.83 | S |
| Example E38 | Absorbing liquid E38 | | 30 | 1-propanol | 24.60 | 70 | 21.0 | 17.2 | 0.82 | S |
| Example E39 | Absorbing liquid E39 | | 30 | 3-Methoxy-1-butanol | 21.17 | 70 | 18.2 | 13.8 | 0.76 | A |
| Example E40 | Absorbing liquid E40 | | 30 | 1-Methoxy-2-propanol | 20.44 | 70 | 18.5 | 13.7 | 0.74 | A |
| Example B43 | Absorbing liquid B43 | (A79) | 30 | Dimethyl sulfoxide | 26.68 | 70 | 18.8 | 15.1 | 0.80 | S |
| Example B44 | Absorbing liquid B44 | (A80) | 30 | | | 70 | 19.1 | 15.2 | 0.80 | S |
| Example B45 | Absorbing liquid B45 | (A81) | 30 | | | 70 | 20.2 | 14.8 | 0.73 | A |
| Example B46 | Absorbing liquid B46 | (A82) | 30 | | | 70 | 20.7 | 15.7 | 0.76 | A |
| Example B47 | Absorbing liquid B47 | (A83) | 30 | | | 70 | 19.8 | 15.9 | 0.80 | S |
| Example B48 | Absorbing liquid B48 | (A86) | 30 | | | 70 | 19.4 | 13.7 | 0.71 | A |
| Example B49 | Absorbing liquid B49 | (A87) | 30 | | | 70 | 19.2 | 14.5 | 0.76 | A |
| Example B50 | Absorbing liquid B50 | (A88) | 30 | | | 70 | 19.4 | 13.9 | 0.72 | A |
| Example B51 | Absorbing liquid B51 | (A89) | 30 | | | 70 | 20.1 | 13.7 | 0.68 | B |
| Example B52 | Absorbing liquid B52 | (A90) | 30 | | | 70 | 19.8 | 15.2 | 0.77 | A |
| Example B53 | Absorbing liquid B53 | (A93) | 30 | | | 70 | 17.2 | 14.8 | 0.86 | S |
| Example B54 | Absorbing liquid B54 | (A94) | 30 | | | 70 | 17.7 | 15.0 | 0.85 | S |

[Table 12-3]

Table 12-3

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid medium | dT of liquid medium [MPa1/2] | Liquid medium /wt% | | | | |
| Example J1 | Absorbing liquid J1 | (A95) | 30 | Dimethyl sulfoxide | 26.68 | 70 | 21.5 | 17.6 | 0.82 | S |
| Example J2 | Absorbing liquid J2 | | 15 | Dimethyl sulfoxide | 26.68 | 85 | 11.8 | 9.6 | 0.81 | S |
| Example J3 | Absorbing liquid J3 | | 30 | N-Methyl-2-pyrrolidone | 22.96 | 70 | 18.7 | 13.3 | 0.71 | A |
| Example J4 | Absorbing liquid J4 | | 30 | Diethylene glycol dibutyl ether | 17.00 | 70 | 18.0 | 13.2 | 0.73 | A |
| Example J5 | Absorbing liquid J5 | | 30 | Triethylene glycol dibutyl ether | 17.20 | 70 | 18.6 | 13.2 | 0.71 | A |
| Example J6 | Absorbing liquid J6 | | 30 | Tetraethylene glycol dibutyl ether | 17.80 | 70 | 19.0 | 13.4 | 0.71 | A |
| Example J7 | Absorbing liquid J7 | | 30 | Propylene glycol monopropyl ether | 20.00 | 70 | 19.1 | 13.5 | 0.71 | A |
| Example J8 | Absorbing liquid J8 | | 30 | Ethylene glycol monobutyl ether | 20.82 | 70 | 17.2 | 12.5 | 0.73 | A |
| Example J9 | Absorbing liquid J9 | | 30 | Ethylene glycol monomethyl ether | 23.41 | 70 | 20.1 | 16.1 | 0.80 | S |
| Example J10 | Absorbing liquid J10 | | 30 | Sulfolane | 26.37 | 70 | 20.5 | 16.3 | 0.80 | S |
| Example J11 | Absorbing liquid J11 | | 30 | Water | 47.81 | 70 | 22.4 | 13.8 | 0.62 | B |
| Example J12 | Absorbing liquid J12 | | 30 | Methanol | 29.41 | 70 | 19.1 | 15.7 | 0.82 | S |
| Example J13 | Absorbing liquid J13 | | 30 | Ethanol | 26.52 | 70 | 20.2 | 17.1 | 0.84 | S |
| Example J14 | Absorbing liquid J14 | | 30 | 1-propanol | 24.60 | 70 | 19.2 | 15.4 | 0.80 | S |
| Example J15 | Absorbing liquid J15 | | 30 | 3-Methoxy-1-butanol | 21.17 | 70 | 18.7 | 14.2 | 0.76 | A |
| Example J16 | Absorbing liquid J16 | | 30 | 1-Methoxy-2-propanol | 20.44 | 70 | 21.0 | 15.4 | 0.73 | A |
| Example J17 | Absorbing liquid J17 | (A96) | 30 | Dimethyl sulfoxide | 26.68 | 70 | 21.7 | 17.7 | 0.81 | S |
| Example J18 | Absorbing liquid J18 | | 30 | N-Methyl-2-pyrrolidone | 22.96 | 70 | 19.3 | 14.6 | 0.76 | A |
| Example J19 | Absorbing liquid J19 | | 30 | Diethylene glycol dibutyl ether | 17.00 | 70 | 18.6 | 12.7 | 0.68 | B |
| Example J20 | Absorbing liquid J20 | | 30 | Triethylene glycol dibutyl ether | 17.20 | 70 | 18.2 | 13.3 | 0.73 | A |
| Example J21 | Absorbing liquid J21 | | 30 | Tetraethylene glycol dibutyl ether | 17.80 | 70 | 18.0 | 13.6 | 0.76 | A |
| Example J22 | Absorbing liquid J22 | | 30 | Propylene glycol monopropyl ether | 20.00 | 70 | 18.8 | 13.4 | 0.71 | A |
| Example J23 | Absorbing liquid J23 | | 30 | Ethylene glycol monobutyl ether | 20.82 | 70 | 19.6 | 14.1 | 0.72 | A |
| Example J24 | Absorbing liquid J24 | | 30 | Ethylene glycol monomethyl ether | 23.41 | 70 | 19.9 | 16.0 | 0.80 | S |
| Example J25 | Absorbing liquid J25 | | 30 | Sulfolane | 26.37 | 70 | 20.8 | 16.6 | 0.80 | S |
| Example J26 | Absorbing liquid J26 | | 30 | Water | 47.81 | 70 | 20.5 | 13.0 | 0.63 | B |
| Example J27 | Absorbing liquid J27 | | 30 | Methanol | 29.41 | 70 | 21.4 | 17.5 | 0.82 | S |
| Example J28 | Absorbing liquid J28 | | 30 | Ethanol | 26.52 | 70 | 20.3 | 16.4 | 0.81 | S |
| Example J29 | Absorbing liquid J29 | | 30 | 1-propanol | 24.60 | 70 | 20.0 | 16.5 | 0.82 | S |
| Example J30 | Absorbing liquid J30 | | 30 | 3-Methoxy-1-butanol | 21.17 | 70 | 19.1 | 14.3 | 0.75 | A |
| Example J31 | Absorbing liquid J31 | | 30 | 1-Methoxy-2-propanol | 20.44 | 70 | 19.4 | 14.6 | 0.75 | A |
| Example J32 | Absorbing liquid J32 | (A104) | 30 | Dimethyl sulfoxide | 26.68 | 70 | 20.3 | 16.4 | 0.80 | S |
| Example J33 | Absorbing liquid J33 | | 30 | Methylcyclohexane | 16.03 | 70 | 17.3 | 10.5 | 0.61 | B |
| Example J34 | Absorbing liquid J34 | | 30 | Diethylene glycol dibutyl ether | 17.00 | 70 | 15.1 | 11.0 | 0.73 | A |
| Example J35 | Absorbing liquid J35 | | 30 | Triethylene glycol dibutyl ether | 17.20 | 70 | 15.7 | 11.1 | 0.71 | A |
| Example J36 | Absorbing liquid J36 | | 30 | Tetraethylene glycol dibutyl ether | 17.80 | 70 | 16.2 | 11.5 | 0.71 | A |

[Table 12-4]

Table 12-4

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid medium | dT of liquid medium [MPa1/2] | Liquid medium /wt% | | | | |
| Example J37 | Absorbing liquid J37 | '(A104) | 30 | Propylene glycol monopropyl ether | 20.00 | 70 | 16.6 | 12.3 | 0.74 | A |
| Example J38 | Absorbing liquid J38 | | 30 | Ethylene glycol monobutyl ether | 20.82 | 70 | 19.1 | 14.0 | 0.73 | A |
| Example J39 | Absorbing liquid J39 | | 30 | Ethylene glycol monomethyl ether | 23.41 | 70 | 18.3 | 15.4 | 0.84 | S |
| Example J40 | Absorbing liquid J40 | | 30 | Sulfolane | 26.37 | 70 | 18.7 | 15.1 | 0.81 | S |
| Example J41 | Absorbing liquid J41 | | 30 | Water | 47.81 | 70 | 20.3 | 12.5 | 0.61 | B |
| Example J42 | Absorbing liquid J42 | | 30 | Methanol | 29.41 | 70 | 20.7 | 17.2 | 0.83 | S |
| Example J43 | Absorbing liquid J43 | | 30 | Ethanol | 26.52 | 70 | 21.4 | 17.7 | 0.83 | S |
| Example J44 | Absorbing liquid J44 | | 30 | 1-propanol | 24.60 | 70 | 19.7 | 16.4 | 0.83 | S |
| Example J45 | Absorbing liquid J45 | | 30 | 3-Methoxy-1-butanol | 21.17 | 70 | 19.7 | 15.2 | 0.77 | A |
| Example J46 | Absorbing liquid J46 | | 30 | 1-Methoxy-2-propanol | 20.44 | 70 | 18.7 | 14.1 | 0.75 | A |
| Example J47 | Absorbing liquid J47 | (A105) | 30 | Dimethyl sulfoxide | 26.68 | 70 | 21.4 | 17.3 | 0.81 | S |
| Example J48 | Absorbing liquid J48 | | 30 | N-Methyl-2-pyrrolidone | 22.96 | 70 | 18.5 | 13.8 | 0.74 | A |
| Example J49 | Absorbing liquid J49 | | 30 | Diethylene glycol dibutyl ether | 17.00 | 70 | 17.1 | 12.8 | 0.75 | A |
| Example J50 | Absorbing liquid J50 | | 30 | Triethylene glycol dibutyl ether | 17.20 | 70 | 16.4 | 12.3 | 0.75 | A |
| Example J51 | Absorbing liquid J51 | | 30 | Tetraethylene glycol dibutyl ether | 17.80 | 70 | 15.6 | 11.2 | 0.72 | A |
| Example J52 | Absorbing liquid J52 | | 30 | Propylene glycol monopropyl ether | 20.00 | 70 | 17.8 | 12.8 | 0.72 | A |
| Example J53 | Absorbing liquid J53 | | 30 | Ethylene glycol monobutyl ether | 20.82 | 70 | 18.8 | 13.6 | 0.73 | A |
| Example J54 | Absorbing liquid J54 | | 30 | Ethylene glycol monomethyl ether | 23.41 | 70 | 18.7 | 15.0 | 0.80 | S |
| Example J55 | Absorbing liquid J55 | | 30 | Sulfolane | 26.37 | 70 | 19.6 | 16.0 | 0.82 | S |
| Example J56 | Absorbing liquid J56 | | 30 | Water | 47.81 | 70 | 21.0 | 13.1 | 0.62 | B |
| Example J57 | Absorbing liquid J57 | | 30 | 1-Ethyl-3-methylimidazolium methyl phosphite | - | 70 | 20.0 | 16.5 | 0.82 | S |
| Example J58 | Absorbing liquid J58 | | 30 | Methanol | 29.41 | 70 | 21.1 | 16.9 | 0.80 | S |
| Example J59 | Absorbing liquid J59 | | 30 | Ethanol | 26.52 | 70 | 20.5 | 17.3 | 0.84 | S |
| Example J60 | Absorbing liquid J60 | | 30 | 1-propanol | 24.60 | 70 | 20.6 | 17.3 | 0.84 | S |
| Example J61 | Absorbing liquid J61 | | 30 | 3-Methoxy-1-butanol | 21.17 | 70 | 18.3 | 13.8 | 0.75 | A |
| Example J62 | Absorbing liquid J62 | | 30 | 1-Methoxy-2-propanol | 20.44 | 70 | 18.7 | 14.0 | 0.75 | A |
| Example J63 | Absorbing liquid J63 | (A97) | 30 | Dimethyl sulfoxide | 26.68 | 70 | 18.4 | 14.9 | 0.81 | S |
| Example J64 | Absorbing liquid J64 | (A98) | 30 | | | 70 | 19.0 | 15.4 | 0.81 | S |
| Example J65 | Absorbing liquid J65 | (A99) | 30 | | | 70 | 20.6 | 14.5 | 0.70 | A |
| Example J66 | Absorbing liquid J66 | (A100) | 30 | | | 70 | 20.6 | 16.1 | 0.78 | A |
| Example J67 | Absorbing liquid J67 | (A101) | 30 | | | 70 | 19.5 | 15.6 | 0.80 | S |
| Example J68 | Absorbing liquid J68 | (A102) | 30 | | | 70 | 19.8 | 14.4 | 0.73 | A |
| Example J69 | Absorbing liquid J69 | (A103) | 30 | | | 70 | 18.8 | 14.2 | 0.76 | A |
| Example J70 | Absorbing liquid J70 | (A106) | 30 | | | 70 | 19.5 | 14.1 | 0.72 | A |
| Example J71 | Absorbing liquid J71 | (A107) | 30 | | | 70 | 19.6 | 13.5 | 0.69 | B |
| Example J72 | Absorbing liquid J72 | (A108) | 30 | | | 70 | 19.6 | 15.2 | 0.78 | A |
| Example J73 | Absorbing liquid J73 | (A109) | 30 | | | 70 | 17.5 | 14.9 | 0.85 | S |
| Example J74 | Absorbing liquid J74 | (A110) | 30 | | | 70 | 17.9 | 14.8 | 0.83 | S |
| Example J75 | Absorbing liquid J75 | (A111) | 30 | | | 70 | 17.4 | 14.6 | 0.84 | S |
| Example J76 | Absorbing liquid J76 | (A112) | 30 | | | 70 | 17.6 | 14.9 | 0.85 | S |
| Example J77 | Absorbing liquid J77 | (A113) | 30 | | | 70 | 17.5 | 14.9 | 0.85 | S |
| Example J78 | Absorbing liquid J78 | (A114) | 30 | | | 70 | 17.5 | 14.7 | 0.84 | S |

[Table 12-5]

Table 12-5

| Example | Absorbing liquid | Composition of carbon dioxide absorbing liquid | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| | | Amine compound | Amine compound /wt% | Liquid medium | dT of liquid medium [MPa1/2] | Liquid medium /wt% | | | | |
| Example J79 | Absorbing liquid J79 | (A117) | 30 | Dimethyl sulfoxide | 26.68 | 70 | 19.0 | 15.7 | 0.83 | S |
| Example J80 | Absorbing liquid J80 | | 15 | Dimethyl sulfoxide | 26.68 | 85 | 18.8 | 16.5 | 0.88 | S |
| Example J81 | Absorbing liquid J81 | | 30 | N-Methyl-2-pyrrolidone | 22.96 | 70 | 20.3 | 14.7 | 0.72 | A |
| Example J82 | Absorbing liquid J82 | | 30 | Diethylene glycol dibutyl ether | 17.00 | 70 | 18.4 | 13.3 | 0.72 | A |
| Example J83 | Absorbing liquid J83 | | 30 | Triethylene glycol dibutyl ether | 17.20 | 70 | 19.7 | 14.9 | 0.75 | A |
| Example J84 | Absorbing liquid J84 | | 30 | Tetraethylene glycol dibutyl ether | 17.80 | 70 | 18.8 | 13.4 | 0.71 | A |
| Example J85 | Absorbing liquid J85 | | 30 | Propylene glycol monopropyl ether | 20.00 | 70 | 20.4 | 15.0 | 0.73 | A |
| Example J86 | Absorbing liquid J86 | | 30 | Ethylene glycol monobutyl ether | 20.82 | 70 | 19.3 | 14.6 | 0.76 | A |
| Example J87 | Absorbing liquid J87 | | 30 | Ethylene glycol monomethyl ether | 23.41 | 70 | 19.8 | 17.2 | 0.87 | S |
| Example J88 | Absorbing liquid J88 | | 30 | Sulfolane | 26.37 | 70 | 19.1 | 15.6 | 0.81 | S |
| Example J89 | Absorbing liquid J89 | | 30 | Water | 47.81 | 70 | 19.7 | 12.9 | 0.65 | B |
| Example J90 | Absorbing liquid J90 | | 30 | Methanol | 29.41 | 70 | 18.2 | 15.7 | 0.86 | S |
| Example J91 | Absorbing liquid J91 | | 30 | Ethanol | 26.52 | 70 | 20.3 | 16.3 | 0.80 | S |
| Example J92 | Absorbing liquid J92 | | 30 | 1-propanol | 24.60 | 70 | 19.8 | 17.1 | 0.87 | S |
| Example J93 | Absorbing liquid J93 | | 30 | 3-Methoxy-1-butanol | 21.17 | 70 | 19.9 | 15.1 | 0.76 | A |
| Example J94 | Absorbing liquid J94 | | 30 | 1-Methoxy-2-propanol | 20.44 | 70 | 18.8 | 14.3 | 0.76 | A |
| Example J95 | Absorbing liquid J95 | (A118) | 30 | Dimethyl sulfoxide | 26.68 | 70 | 20.9 | 18.1 | 0.87 | S |
| Example J96 | Absorbing liquid J96 | | 30 | N-Methyl-2-pyrrolidone | 22.96 | 70 | 18.6 | 13.6 | 0.73 | A |
| Example J97 | Absorbing liquid J97 | | 30 | Diethylene glycol dibutyl ether | 17.00 | 70 | 20.3 | 12.8 | 0.63 | B |
| Example J98 | Absorbing liquid J98 | | 30 | Triethylene glycol dibutyl ether | 17.20 | 70 | 18.2 | 13.0 | 0.71 | A |
| Example J99 | Absorbing liquid J99 | | 30 | Tetraethylene glycol dibutyl ether | 17.80 | 70 | 18.1 | 13.0 | 0.72 | A |
| Example J100 | Absorbing liquid J100 | | 30 | Propylene glycol monopropyl ether | 20.00 | 70 | 18.5 | 14.1 | 0.76 | A |
| Example J101 | Absorbing liquid J101 | | 30 | Ethylene glycol monobutyl ether | 20.82 | 70 | 20.7 | 14.9 | 0.72 | A |
| Example J102 | Absorbing liquid J102 | | 30 | Ethylene glycol monomethyl ether | 23.41 | 70 | 19.6 | 17.3 | 0.88 | S |
| Example J103 | Absorbing liquid J103 | | 30 | Sulfolane | 26.37 | 70 | 19.3 | 15.6 | 0.81 | S |
| Example J104 | Absorbing liquid J104 | | 30 | Water | 47.81 | 70 | 19.5 | 13.0 | 0.67 | B |
| Example J105 | Absorbing liquid J105 | | 30 | Methanol | 29.41 | 70 | 19.3 | 16.9 | 0.88 | S |
| Example J106 | Absorbing liquid J106 | | 30 | Ethanol | 26.52 | 70 | 18.2 | 15.2 | 0.83 | S |
| Example J107 | Absorbing liquid J107 | | 30 | 1-propanol | 24.60 | 70 | 19.5 | 16.2 | 0.83 | S |
| Example J108 | Absorbing liquid J108 | | 30 | 3-Methoxy-1-butanol | 21.17 | 70 | 18.5 | 13.4 | 0.72 | A |
| Example J109 | Absorbing liquid J109 | | 30 | 1-Methoxy-2-propanol | 20.44 | 70 | 19.0 | 13.9 | 0.73 | A |

[Table 12-6]

Table 12-6

| Example | Absorbing liquid | Composition of carbon dioxide absorbing liquid: Amine compound | Amine compound /wt% | Liquid medium | dT of liquid medium [MPa1/2] | Liquid medium /wt% | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| Example J110 | Absorbing liquid J110 | (A121) | 30 | Dimethyl sulfoxide | 26.68 | 70 | 18.9 | 16.4 | 0.87 | S |
| Example J111 | Absorbing liquid J111 | | 30 | Methylcyclohexane | 16.03 | 70 | 18.6 | 12.2 | 0.66 | B |
| Example J112 | Absorbing liquid J112 | | 30 | Diethylene glycol dibutyl ether | 17.00 | 70 | 17.5 | 12.3 | 0.70 | A |
| Example J113 | Absorbing liquid J113 | | 30 | Triethylene glycol dibutyl ether | 17.20 | 70 | 19.3 | 14.2 | 0.73 | A |
| Example J114 | Absorbing liquid J114 | | 30 | Tetraethylene glycol dibutyl ether | 17.80 | 70 | 18.4 | 14.0 | 0.76 | A |
| Example J115 | Absorbing liquid J115 | | 30 | Propylene glycol monopropyl ether | 20.00 | 70 | 18.8 | 14.3 | 0.76 | A |
| Example J116 | Absorbing liquid J116 | | 30 | Ethylene glycol monobutyl ether | 20.82 | 70 | 19.9 | 14.1 | 0.71 | A |
| Example J117 | Absorbing liquid J117 | | 30 | Ethylene glycol monomethyl ether | 23.41 | 70 | 20.7 | 17.6 | 0.85 | S |
| Example J118 | Absorbing liquid J118 | | 30 | Sulfolane | 26.37 | 70 | 17.8 | 15.7 | 0.88 | S |
| Example J119 | Absorbing liquid J119 | | 30 | Water | 47.81 | 70 | 19.7 | 13.1 | 0.66 | B |
| Example J120 | Absorbing liquid J120 | | 30 | Methanol | 29.41 | 70 | 19.2 | 15.4 | 0.80 | S |
| Example J121 | Absorbing liquid J121 | | 30 | Ethanol | 26.52 | 70 | 18.8 | 15.5 | 0.82 | S |
| Example J122 | Absorbing liquid J122 | | 30 | 1-propanol | 24.60 | 70 | 19.6 | 16.2 | 0.82 | S |
| Example J123 | Absorbing liquid J123 | | 30 | 3-Methoxy-1-butanol | 21.17 | 70 | 17.7 | 13.6 | 0.77 | A |
| Example J124 | Absorbing liquid J124 | | 30 | 1-Methoxy-2-propanol | 20.44 | 70 | 18.5 | 13.3 | 0.72 | A |
| Example J125 | Absorbing liquid J125 | (A122) | 30 | Dimethyl sulfoxide | 26.68 | 70 | 19.7 | 16.3 | 0.83 | S |
| Example J126 | Absorbing liquid J126 | | 30 | N-Methyl-2-pyrrolidone | 22.96 | 70 | 18.6 | 14.4 | 0.77 | A |
| Example J127 | Absorbing liquid J127 | | 30 | Diethylene glycol dibutyl ether | 17.00 | 70 | 19.6 | 14.4 | 0.74 | A |
| Example J128 | Absorbing liquid J128 | | 30 | Triethylene glycol dibutyl ether | 17.20 | 70 | 19.7 | 14.9 | 0.76 | A |
| Example J129 | Absorbing liquid J129 | | 30 | Tetraethylene glycol dibutyl ether | 17.80 | 70 | 20.5 | 14.4 | 0.70 | A |
| Example J130 | Absorbing liquid J130 | | 30 | Propylene glycol monopropyl ether | 20.00 | 70 | 19.0 | 13.5 | 0.71 | A |
| Example J131 | Absorbing liquid J131 | | 30 | Ethylene glycol monobutyl ether | 20.82 | 70 | 19.1 | 14.6 | 0.77 | A |
| Example J132 | Absorbing liquid J132 | | 30 | Ethylene glycol monomethyl ether | 23.41 | 70 | 19.4 | 17.0 | 0.87 | S |
| Example J133 | Absorbing liquid J133 | | 30 | Sulfolane | 26.37 | 70 | 19.8 | 17.1 | 0.87 | S |
| Example J134 | Absorbing liquid J134 | | 30 | Water | 47.81 | 70 | 18.8 | 12.2 | 0.65 | B |
| Example J135 | Absorbing liquid J135 | | 30 | 1-Ethyl-3-methylimidazolium methyl phosphite | - | 70 | 19.5 | 16.2 | 0.83 | S |
| Example J136 | Absorbing liquid J136 | | 30 | Methanol | 29.41 | 70 | 17.8 | 15.4 | 0.87 | S |
| Example J137 | Absorbing liquid J137 | | 30 | Ethanol | 26.52 | 70 | 20.1 | 16.5 | 0.82 | S |
| Example J138 | Absorbing liquid J138 | | 30 | 1-propanol | 24.60 | 70 | 19.3 | 15.8 | 0.82 | S |
| Example J139 | Absorbing liquid J139 | | 30 | 3-Methoxy-1-butanol | 21.17 | 70 | 17.8 | 13.3 | 0.75 | A |
| Example J140 | Absorbing liquid J140 | | 30 | 1-Methoxy-2-propanol | 20.44 | 70 | 19.1 | 14.1 | 0.74 | A |

[Table 12-7]

Table 12-7

| Example | Absorbing liquid | Composition of carbon dioxide absorbing liquid: Amine compound | Amine compound /wt% | Liquid medium | dT of liquid medium [MPa1/2] | Liquid medium /wt% | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|
| Example J141 | Absorbing liquid J141 | (A115) | 30 | Dimethyl sulfoxide | 26.68 | 70 | 17.5 | 14.9 | 0.85 | S |
| Example J142 | Absorbing liquid J142 | (A116) | 30 | | | 70 | 20.4 | 16.3 | 0.80 | S |
| Example J143 | Absorbing liquid J143 | (A119) | 30 | | | 70 | 18.2 | 13.9 | 0.77 | A |
| Example J144 | Absorbing liquid J144 | (A120) | 30 | | | 100 | 19.6 | 14.2 | 0.73 | A |
| Example J145 | Absorbing liquid J145 | (A124) | 30 | Dimethyl sulfoxide | 26.68 | 70 | 19.7 | 16.3 | 0.83 | S |
| Example J146 | Absorbing liquid J146 | | 30 | N-Methyl-2-pyrrolidone | 22.96 | 70 | 18.6 | 16.8 | 0.90 | A |
| Example J147 | Absorbing liquid J147 | | 30 | Diethylene glycol dibutyl ether | 17.00 | 70 | 19.6 | 14.4 | 0.74 | A |
| Example J148 | Absorbing liquid J148 | | 30 | Triethylene glycol dibutyl ether | 17.20 | 70 | 19.7 | 14.9 | 0.76 | A |
| Example J149 | Absorbing liquid J149 | | 30 | Tetraethylene glycol dibutyl ether | 17.80 | 70 | 20.5 | 14.4 | 0.70 | A |
| Example J150 | Absorbing liquid J150 | | 30 | Propylene glycol monopropyl ether | 20.00 | 70 | 19.0 | 13.5 | 0.71 | A |
| Example J151 | Absorbing liquid J151 | | 30 | Ethylene glycol monobutyl ether | 20.82 | 70 | 19.1 | 14.6 | 0.77 | A |
| Example J152 | Absorbing liquid J152 | | 30 | Ethylene glycol monomethyl ether | 23.41 | 70 | 19.4 | 17.0 | 0.87 | S |
| Example J153 | Absorbing liquid J153 | | 30 | Sulfolane | 26.37 | 70 | 19.8 | 17.1 | 0.87 | S |
| Example J154 | Absorbing liquid J154 | | 30 | Water | 47.81 | 70 | 18.8 | 14.3 | 0.76 | A |
| Example J155 | Absorbing liquid J155 | | 30 | 1-Ethyl-3-methylimidazolium methyl phosphite | - | 70 | 19.5 | 16.2 | 0.83 | S |
| Example J156 | Absorbing liquid J156 | | 30 | Methanol | 29.41 | 70 | 17.8 | 15.4 | 0.87 | S |
| Example J157 | Absorbing liquid J157 | | 30 | Ethanol | 26.52 | 70 | 20.1 | 16.5 | 0.82 | S |
| Example J158 | Absorbing liquid J158 | | 30 | 1-propanol | 24.60 | 70 | 19.3 | 15.8 | 0.82 | S |
| Example J159 | Absorbing liquid J159 | | 30 | 3-Methoxy-1-butanol | 21.17 | 70 | 17.8 | 13.3 | 0.75 | A |
| Example J160 | Absorbing liquid J160 | | 30 | 1-Methoxy-2-propanol | 20.44 | 70 | 19.1 | 14.1 | 0.74 | A |
| Example J161 | Absorbing liquid J161 | (A123) | 30 | Dimethyl sulfoxide | 26.68 | 70 | 17.5 | 14.9 | 0.85 | S |
| Example J162 | Absorbing liquid J162 | (A125) | 30 | | | 70 | 20.4 | 16.3 | 0.80 | S |
| Example J163 | Absorbing liquid J163 | (A126) | 30 | | | 70 | 18.2 | 15.8 | 0.87 | S |
| Example J164 | Absorbing liquid J164 | (A127) | 30 | | | 70 | 19.6 | 16.2 | 0.83 | S |
| Example J165 | Absorbing liquid J165 | (A128) | 30 | | | 70 | 19.3 | 16.4 | 0.85 | S |

[Example D1 (absorbing liquid B55)]

25 g of compound (A84) and 5 g of MDEA were mixed with 70 g of dimethyl sulfoxide and stirred to prepare a carbon dioxide absorbing liquid (100 g). This liquid was placed in a 200 mL gas absorption bottle, and the experiment was performed in the same manner. The evaluation results are shown in Table 13-1.

[Example D2 (absorbing liquid B56)]

25 g of compound (A85) and 5 g of MBZA were mixed with 60 g of sulfolane and 10 g of water, and stirred to prepare a carbon dioxide absorbing liquid (100 g). This liquid was placed in a 200 mL gas absorption bottle, and the experiment was performed in the same manner. The evaluation results are shown in Table 13-1.

[Example D3 (absorbing liquid B57)]

25 g of compound (A89) and 5 g of MBZA were mixed with 60 g of dimethyl sulfoxide and 10 g of water, and stirred to prepare a carbon dioxide absorbing liquid (100 g). This liquid was placed in a 200 mL gas absorption bottle, and the experiment was performed in the same manner. The evaluation results are shown in Table 13-1.

[Example D4 (absorbing liquid B58)]

25 g of compound (A84) and 5 g of compound (A93) were mixed with 70 g of dimethyl sulfoxide and stirred to prepare a carbon dioxide absorbing liquid (100 g). This liquid was placed in a 200 mL gas absorption bottle, and the experiment was performed in the same manner. The evaluation results are shown in Table 13-1.

[Example D5 (absorbing liquid B59)]

25 g of compound (A85) and 5 g of compound (A94) were mixed with 70 g of dimethyl sulfoxide and stirred to prepare a carbon dioxide absorbing liquid (100 g). This liquid was placed in a 200 mL gas absorption bottle, and the experiment was performed in the same manner. The evaluation results are shown in Table 13-1.

[Example D6 (absorbing liquid B60)]

25 g of compound (A85) and 2 g of ATMP were mixed with 60 g of dimethyl sulfoxide and 10 g of N-methyl-2-pyrrolidone, and stirred to prepare a carbon dioxide absorbing liquid (100 g). This liquid was placed in a 200 mL gas absorption bottle, and the experiment was performed in the same manner. The evaluation results are shown in Table 13-1.

[Example A124 (absorbing liquid A124)]

25 g of compound (A127) was mixed with 70 g of dimethyl sulfoxide and 5 g of 2-aminobutanol, and stirred to prepare a carbon dioxide absorbing liquid (100 g). This liquid was placed in a 200 mL gas absorption bottle, and the experiment was performed in the same manner. The evaluation results are shown in Table 13-1.

[Example A125 (absorbing liquid A125)]

25 g of compound (A128) was mixed with 70 g of dimethyl sulfoxide and 5 g of dimethylaminopropylamine, and stirred to prepare a carbon dioxide absorbing liquid (100 g). This liquid was placed in a 200 mL gas absorption bottle, and the experiment was performed in the same manner. The evaluation results are shown in Table 13-1.

[Example D7 (absorbing liquid B61)]

30 g of compound (A84) was mixed with 60 g of dimethyl sulfoxide and 10 g of N-methyl-2-pyrrolidone, and stirred to prepare a carbon dioxide absorbing liquid (100 g). This liquid was placed in a 200 mL gas absorption bottle, and the experiment was performed in the same manner. The evaluation results are shown in Table 13-1.

[Examples C1 to C83, K1 to K128 (absorbing liquids C1 to C83, K1 to K128)]

The compound and liquid medium described in Example D1 were changed to the compounds and liquid media described in Table 13-1 to Table 13-7, respectively. The carbon dioxide gas absorption amount was measured according to

the method described in the aforementioned [Measurement method for release efficiency of carbon dioxide gas]. The carbon dioxide absorption amount (L) per 1 kg of carbon dioxide absorbing liquid and the carbon dioxide gas release efficiency were calculated. It is noted that the gas types used for the evaluation are as shown in Table 6 above.

Based on the measurement of the calculated carbon dioxide gas release efficiency, the following criteria were established for evaluation, with S, A, and B considered as the usable range. The evaluation results are shown in Table 13-1 to Table 13-7.

S: Release efficiency of 0.8 or higher
A: Release efficiency of 0.7 or higher and less than 0.8
B: Release efficiency of 0.6 or higher and less than 0.7
C: Release efficiency less than 0.6

[Table 13-1]

Table 13-1

| Example | Absorbing liquid | Composition of carbon dioxide absorbing liquid | | | | | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | dT [MPa1/2] of the entire liquid medium | | | | |
| Example D1 | Absorbing liquid B55 | (A84) | 25 | MDEA | 5 | Dimethyl sulfoxide | 70 | - | - | 26.68 | 19.6 | 16.4 | 0.84 | S |
| Example D2 | Absorbing liquid B56 | (A85) | 25 | MBZA | 5 | Sulfolane | 60 | Water | 10 | 29.43 | 19.1 | 16.0 | 0.84 | S |
| Example D3 | Absorbing liquid B57 | (A89) | 25 | MBZA | 5 | Dimethyl sulfoxide | 60 | Water | 10 | 29.70 | 19.4 | 15.2 | 0.78 | A |
| Example D4 | Absorbing liquid B58 | (A84) | 25 | (A93) | 5 | Dimethyl sulfoxide | 70 | - | - | 26.68 | 19.8 | 16.8 | 0.85 | S |
| Example D5 | Absorbing liquid B59 | (A85) | 25 | (A94) | 5 | Dimethyl sulfoxide | 70 | - | - | 26.68 | 18.6 | 15.4 | 0.83 | S |
| Example D6 | Absorbing liquid B60 | (A85) | 28 | ATMP | 2 | Dimethyl sulfoxide | 60 | N-Methyl-2-pyrrolidone | 10 | 26.15 | 19.3 | 15.9 | 0.82 | S |
| Example A124 | Absorbing liquid A124 | (A127) | 25 | AB | 5 | Dimethyl sulfoxide | 70 | - | - | 26.68 | 20.5 | 17.4 | 0.85 | S |
| Example A125 | Absorbing liquid A125 | (A128) | 25 | DMAPA | 5 | Dimethyl sulfoxide | 70 | - | - | 26.68 | 21.2 | 18.2 | 0.86 | S |
| Example D7 | Absorbing liquid B61 | (A84) | 30 | - | - | Dimethyl sulfoxide | 60 | N-Methyl-2-pyrrolidone | 10 | 26.15 | 19.3 | 16.7 | 0.87 | S |
| Example C1 | Absorbing liquid C1 | (A84) | 30 | - | - | Dimethyl sulfoxide | 50 | N-Methyl-2-pyrrolidone | 20 | 25.61 | 20.7 | 17.4 | 0.84 | S |
| Example C2 | Absorbing liquid C2 | (A84) | 30 | - | - | Dimethyl sulfoxide | 35 | N-Methyl-2-pyrrolidone | 35 | 24.82 | 19.4 | 16.3 | 0.84 | S |
| Example C3 | Absorbing liquid C3 | (A84) | 30 | - | - | Dimethyl sulfoxide | 20 | N-Methyl-2-pyrrolidone | 50 | 24.02 | 20.6 | 16.9 | 0.82 | S |
| Example C4 | Absorbing liquid C4 | (A84) | 30 | - | - | Dimethyl sulfoxide | 10 | N-Methyl-2-pyrrolidone | 60 | 23.49 | 20.8 | 16.6 | 0.80 | S |
| Example C5 | Absorbing liquid C5 | (A85) | 30 | - | - | Dimethyl sulfoxide | 60 | N-Methyl-2-pyrrolidone | 10 | 26.14 | 20.7 | 17.2 | 0.83 | S |
| Example C6 | Absorbing liquid C6 | (A85) | 30 | - | - | Dimethyl sulfoxide | 50 | N-Methyl-2-pyrrolidone | 20 | 25.61 | 20.7 | 17.2 | 0.83 | S |
| Example C7 | Absorbing liquid C7 | (A85) | 30 | - | - | Dimethyl sulfoxide | 35 | N-Methyl-2-pyrrolidone | 35 | 24.82 | 20.6 | 17.1 | 0.83 | S |
| Example C8 | Absorbing liquid C8 | (A85) | 30 | - | - | Dimethyl sulfoxide | 20 | N-Methyl-2-pyrrolidone | 50 | 24.02 | 20.4 | 17.5 | 0.86 | S |
| Example C9 | Absorbing liquid C9 | (A85) | 30 | - | - | Dimethyl sulfoxide | 10 | N-Methyl-2-pyrrolidone | 60 | 23.49 | 20.6 | 17.1 | 0.83 | S |
| Example C10 | Absorbing liquid C10 | (A91) | 30 | - | - | Dimethyl sulfoxide | 60 | N-Methyl-2-pyrrolidone | 10 | 26.14 | 20.1 | 17.1 | 0.85 | S |
| Example C11 | Absorbing liquid C11 | (A91) | 30 | - | - | Dimethyl sulfoxide | 50 | N-Methyl-2-pyrrolidone | 20 | 25.61 | 19.5 | 16.2 | 0.83 | S |
| Example C12 | Absorbing liquid C12 | (A91) | 30 | - | - | Dimethyl sulfoxide | 35 | N-Methyl-2-pyrrolidone | 35 | 24.82 | 21.1 | 17.7 | 0.84 | S |
| Example C13 | Absorbing liquid C13 | (A91) | 30 | - | - | Dimethyl sulfoxide | 20 | N-Methyl-2-pyrrolidone | 50 | 24.02 | 20.3 | 17.3 | 0.85 | S |
| Example C14 | Absorbing liquid C14 | (A91) | 30 | - | - | Dimethyl sulfoxide | 10 | N-Methyl-2-pyrrolidone | 60 | 23.49 | 20 | 17.2 | 0.86 | S |
| Example C15 | Absorbing liquid C15 | (A92) | 30 | - | - | Dimethyl sulfoxide | 60 | N-Methyl-2-pyrrolidone | 10 | 26.14 | 20.7 | 17.4 | 0.84 | S |
| Example C16 | Absorbing liquid C16 | (A92) | 30 | - | - | Dimethyl sulfoxide | 50 | N-Methyl-2-pyrrolidone | 20 | 25.61 | 20.2 | 16.6 | 0.82 | S |
| Example C17 | Absorbing liquid C17 | (A92) | 30 | - | - | Dimethyl sulfoxide | 35 | N-Methyl-2-pyrrolidone | 35 | 24.82 | 20.5 | 17.2 | 0.84 | S |
| Example C18 | Absorbing liquid C18 | (A92) | 30 | - | - | Dimethyl sulfoxide | 20 | N-Methyl-2-pyrrolidone | 50 | 24.02 | 21.1 | 17.7 | 0.84 | S |
| Example C19 | Absorbing liquid C19 | (A92) | 30 | - | - | Dimethyl sulfoxide | 10 | N-Methyl-2-pyrrolidone | 60 | 23.49 | 19.8 | 16.2 | 0.82 | S |
| Example C20 | Absorbing liquid C20 | (A84) | 30 | - | - | Dimethyl sulfoxide | 50 | Methanol | 20 | 27.46 | 20.2 | 16.6 | 0.82 | S |
| Example C21 | Absorbing liquid C21 | (A84) | 30 | - | - | Dimethyl sulfoxide | 50 | Ethanol | 20 | 26.63 | 19.5 | 16 | 0.82 | S |
| Example C22 | Absorbing liquid C22 | (A84) | 30 | - | - | Dimethyl sulfoxide | 50 | 1-propanol | 20 | 26.08 | 20.9 | 17.3 | 0.83 | S |
| Example C23 | Absorbing liquid C23 | (A84) | 30 | - | - | Dimethyl sulfoxide | 50 | 3-Methoxy-1-butanol | 20 | 25.10 | 21.3 | 17.5 | 0.82 | S |
| Example C24 | Absorbing liquid C24 | (A84) | 30 | - | - | Dimethyl sulfoxide | 50 | 1-Methoxy-2-propanol | 20 | 24.89 | 20.5 | 16.8 | 0.82 | S |
| Example C25 | Absorbing liquid C25 | (A84) | 30 | - | - | Dimethyl sulfoxide | 50 | Water | 20 | 32.71 | 21 | 17.4 | 0.83 | S |
| Example C26 | Absorbing liquid C26 | (A84) | 30 | - | - | Dimethyl sulfoxide | 50 | Sulfolane | 20 | 26.59 | 20.9 | 16.9 | 0.81 | S |
| Example C27 | Absorbing liquid C27 | (A84) | 30 | - | - | Dimethyl sulfoxide | 50 | Ethylene glycol monomethyl ether | 20 | 25.74 | 20.9 | 17.8 | 0.85 | S |

[Table 13-2]

Table 13-2

| Example | Absorbing liquid | Composition of carbon dioxide absorbing liquid | | | | | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | dT [MPa1/2] of the entire liquid medium | | | | |
| Example C28 | Absorbing liquid C28 | (A84) | 30 | - | - | Methanol | 50 | Ethanol | 20 | 28.58 | 20.8 | 17.1 | 0.82 | S |
| Example C29 | Absorbing liquid C29 | (A84) | 30 | - | - | Methanol | 50 | 1-propanol | 20 | 28.03 | 20.8 | 16.8 | 0.81 | S |
| Example C30 | Absorbing liquid C30 | (A84) | 30 | - | - | Methanol | 50 | Water | 20 | 34.66 | 20.7 | 17 | 0.82 | S |
| Example C31 | Absorbing liquid C31 | (A84) | 30 | - | - | 1-propanol | 50 | 1-Methoxy-2-propanol | 20 | 23.41 | 19.6 | 16.3 | 0.83 | S |
| Example C32 | Absorbing liquid C32 | (A84) | 30 | - | - | 1-propanol | 50 | 3-Methoxy-1-butanol | 20 | 23.62 | 20.2 | 17.2 | 0.85 | S |
| Example C33 | Absorbing liquid C33 | (A84) | 30 | - | - | 1-propanol | 50 | Water | 20 | 31.23 | 20.7 | 16.8 | 0.81 | S |
| Example C34 | Absorbing liquid C34 | (A84) | 30 | - | - | Ethylene glycol monomethyl ether | 50 | Tetraethylene glycol dibutyl ether | 20 | 21.81 | 19.4 | 14.2 | 0.73 | A |
| Example C35 | Absorbing liquid C35 | (A84) | 30 | - | - | Tetraethylene glycol dibutyl ether | 50 | Sulfolane | 20 | 20.25 | 19 | 13.7 | 0.72 | A |
| Example C36 | Absorbing liquid C36 | (A85) | 30 | - | - | Dimethyl sulfoxide | 50 | Methanol | 20 | 27.46 | 20.1 | 16.9 | 0.84 | S |
| Example C37 | Absorbing liquid C37 | (A85) | 30 | - | - | Dimethyl sulfoxide | 50 | Ethanol | 20 | 26.63 | 21 | 17.4 | 0.83 | S |
| Example C38 | Absorbing liquid C38 | (A85) | 30 | - | - | Dimethyl sulfoxide | 50 | 1-propanol | 20 | 26.08 | 20.7 | 17.4 | 0.84 | S |
| Example C39 | Absorbing liquid C39 | (A85) | 30 | - | - | Dimethyl sulfoxide | 50 | 3-Methoxy-1-butanol | 20 | 25.10 | 20.2 | 16.6 | 0.82 | S |
| Example C40 | Absorbing liquid C40 | (A85) | 30 | - | - | Dimethyl sulfoxide | 50 | 1-Methoxy-2-propanol | 20 | 24.89 | 21.7 | 18.2 | 0.84 | S |
| Example C41 | Absorbing liquid C41 | (A85) | 30 | - | - | Dimethyl sulfoxide | 50 | Water | 20 | 32.71 | 20.3 | 16.8 | 0.83 | S |
| Example C42 | Absorbing liquid C42 | (A85) | 30 | - | - | Dimethyl sulfoxide | 50 | Sulfolane | 20 | 26.59 | 20.4 | 17.5 | 0.86 | S |
| Example C43 | Absorbing liquid C43 | (A85) | 30 | - | - | Dimethyl sulfoxide | 50 | Ethylene glycol monomethyl ether | 20 | 25.74 | 20.8 | 17.5 | 0.84 | S |
| Example C44 | Absorbing liquid C44 | (A85) | 30 | - | - | Methanol | 50 | Ethanol | 20 | 28.58 | 21.1 | 18.1 | 0.86 | S |
| Example C45 | Absorbing liquid C45 | (A85) | 30 | - | - | Methanol | 50 | 1-propanol | 20 | 28.03 | 20.3 | 17.1 | 0.84 | S |
| Example C46 | Absorbing liquid C46 | (A85) | 30 | - | - | Methanol | 50 | Water | 20 | 34.66 | 20.5 | 16.8 | 0.82 | S |
| Example C47 | Absorbing liquid C47 | (A85) | 30 | - | - | 1-propanol | 50 | 1-Methoxy-2-propanol | 20 | 23.41 | 20.6 | 17.3 | 0.84 | S |
| Example C48 | Absorbing liquid C48 | (A85) | 30 | - | - | 1-propanol | 50 | 3-Methoxy-1-butanol | 20 | 23.62 | 19.8 | 16.4 | 0.83 | S |
| Example C49 | Absorbing liquid C49 | (A85) | 30 | - | - | 1-propanol | 50 | Water | 20 | 31.23 | 20.4 | 16.7 | 0.82 | S |
| Example C50 | Absorbing liquid C50 | (A85) | 30 | - | - | Ethylene glycol monomethyl ether | 50 | Tetraethylene glycol dibutyl ether | 20 | 21.81 | 18.9 | 14.2 | 0.75 | A |
| Example C51 | Absorbing liquid C51 | (A85) | 30 | - | - | Tetraethylene glycol dibutyl ether | 50 | Sulfolane | 20 | 20.25 | 17.8 | 13.7 | 0.77 | A |
| Example C52 | Absorbing liquid C52 | (A91) | 30 | - | - | Dimethyl sulfoxide | 50 | Methanol | 20 | 27.46 | 20.3 | 16.8 | 0.83 | S |
| Example C53 | Absorbing liquid C53 | (A91) | 30 | - | - | Dimethyl sulfoxide | 50 | Ethanol | 20 | 26.63 | 20.9 | 17.1 | 0.82 | S |
| Example C54 | Absorbing liquid C54 | (A91) | 30 | - | - | Dimethyl sulfoxide | 50 | 1-propanol | 20 | 26.08 | 21.5 | 17.8 | 0.83 | S |
| Example C55 | Absorbing liquid C55 | (A91) | 30 | - | - | Dimethyl sulfoxide | 50 | 3-Methoxy-1-butanol | 20 | 25.10 | 20.7 | 17.2 | 0.83 | S |
| Example C56 | Absorbing liquid C56 | (A91) | 30 | - | - | Dimethyl sulfoxide | 50 | 1-Methoxy-2-propanol | 20 | 24.89 | 20.7 | 17 | 0.82 | S |
| Example C57 | Absorbing liquid C57 | (A91) | 30 | - | - | Dimethyl sulfoxide | 50 | Water | 20 | 32.71 | 21.5 | 17.6 | 0.82 | S |
| Example C58 | Absorbing liquid C58 | (A91) | 30 | - | - | Dimethyl sulfoxide | 50 | Sulfolane | 20 | 26.59 | 20 | 16.2 | 0.81 | S |
| Example C59 | Absorbing liquid C59 | (A91) | 30 | - | - | Dimethyl sulfoxide | 50 | Ethylene glycol monomethyl ether | 20 | 25.74 | 20.9 | 17.8 | 0.85 | S |
| Example C60 | Absorbing liquid C60 | (A91) | 30 | - | - | Methanol | 50 | Ethanol | 20 | 28.58 | 20.9 | 17.1 | 0.82 | S |
| Example C61 | Absorbing liquid C61 | (A91) | 30 | - | - | Methanol | 50 | 1-propanol | 20 | 28.03 | 21.4 | 18 | 0.84 | S |
| Example C62 | Absorbing liquid C62 | (A91) | 30 | - | - | Methanol | 50 | Water | 20 | 34.66 | 20.1 | 17.3 | 0.86 | S |

[Table 13-3]

Table 13-3

| Example | Absorbing liquid | Composition of carbon dioxide absorbing liquid | | | | | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | dT [MPa1/2] of the entire liquid medium | | | | |
| Example C63 | Absorbing liquid C63 | (A91) | 30 | - | - | 1-propanol | 50 | 1-Methoxy-2-propanol | 20 | 23.41 | 20.4 | 16.9 | 0.83 | S |
| Example C64 | Absorbing liquid C64 | (A91) | 30 | - | - | 1-propanol | 50 | 3-Methoxy-1-butanol | 20 | 23.62 | 20.1 | 16.9 | 0.84 | S |
| Example C65 | Absorbing liquid C65 | (A91) | 30 | - | - | 1-propanol | 50 | Water | 20 | 31.23 | 20.6 | 17.5 | 0.85 | S |
| Example C66 | Absorbing liquid C66 | (A91) | 30 | - | - | Ethylene glycol monomethyl ether | 50 | Tetraethylene glycol dibutyl ether | 20 | 21.81 | 20.2 | 14.5 | 0.72 | A |
| Example C67 | Absorbing liquid C67 | (A91) | 30 | - | - | Tetraethylene glycol dibutyl ether | 50 | Sulfolane | 20 | 20.25 | 18.6 | 13.6 | 0.73 | A |
| Example C68 | Absorbing liquid C68 | (A92) | 30 | - | - | Dimethyl sulfoxide | 50 | Methanol | 20 | 27.46 | 20.3 | 17.1 | 0.84 | S |
| Example C69 | Absorbing liquid C69 | (A92) | 30 | - | - | Dimethyl sulfoxide | 50 | Ethanol | 20 | 26.63 | 21.3 | 17.7 | 0.83 | S |
| Example C70 | Absorbing liquid C70 | (A92) | 30 | - | - | Dimethyl sulfoxide | 50 | 1-propanol | 20 | 26.08 | 19.8 | 16.4 | 0.83 | S |
| Example C71 | Absorbing liquid C71 | (A92) | 30 | - | - | Dimethyl sulfoxide | 50 | 3-Methoxy-1-butanol | 20 | 25.10 | 20.8 | 17.5 | 0.84 | S |
| Example C72 | Absorbing liquid C72 | (A92) | 30 | - | - | Dimethyl sulfoxide | 50 | 1-Methoxy-2-propanol | 20 | 24.89 | 19.7 | 16.2 | 0.82 | S |
| Example C73 | Absorbing liquid C73 | (A92) | 30 | - | - | Dimethyl sulfoxide | 50 | Water | 20 | 32.71 | 19.8 | 16.6 | 0.84 | S |
| Example C74 | Absorbing liquid C74 | (A92) | 30 | - | - | Dimethyl sulfoxide | 50 | Sulfolane | 20 | 26.59 | 20.4 | 16.9 | 0.83 | S |
| Example C75 | Absorbing liquid C75 | (A92) | 30 | - | - | Dimethyl sulfoxide | 50 | Ethylene glycol monomethyl ether | 20 | 25.74 | 20.2 | 17 | 0.84 | S |
| Example C76 | Absorbing liquid C76 | (A92) | 30 | - | - | Methanol | 50 | Ethanol | 20 | 28.58 | 21.3 | 17.5 | 0.82 | S |
| Example C77 | Absorbing liquid C77 | (A92) | 30 | - | - | Methanol | 50 | 1-propanol | 20 | 28.03 | 20.8 | 17.9 | 0.86 | S |
| Example C78 | Absorbing liquid C78 | (A92) | 30 | - | - | Methanol | 50 | Water | 20 | 34.66 | 20.5 | 17 | 0.83 | S |
| Example C79 | Absorbing liquid C79 | (A92) | 30 | - | - | 1-propanol | 50 | 1-Methoxy-2-propanol | 20 | 23.41 | 20 | 17 | 0.85 | S |
| Example C80 | Absorbing liquid C80 | (A92) | 30 | - | - | 1-propanol | 50 | 3-Methoxy-1-butanol | 20 | 23.62 | 20.8 | 17.7 | 0.85 | S |
| Example C81 | Absorbing liquid C81 | (A92) | 30 | - | - | 1-propanol | 50 | Water | 20 | 31.23 | 20.2 | 16.6 | 0.82 | S |
| Example C82 | Absorbing liquid C82 | (A92) | 30 | - | - | Ethylene glycol monomethyl ether | 50 | Tetraethylene glycol dibutyl ether | 20 | 21.81 | 19.5 | 14.6 | 0.75 | A |
| Example C83 | Absorbing liquid C83 | (A92) | 30 | - | - | Tetraethylene glycol dibutyl ether | 50 | Sulfolane | 20 | 20.25 | 18 | 13.7 | 0.76 | A |
| Example K1 | Absorbing liquid K1 | (A95) | 30 | - | - | Dimethyl sulfoxide | 50 | Methanol | 20 | 27.46 | 19.4 | 15.9 | 0.82 | S |
| Example K2 | Absorbing liquid K2 | (A95) | 30 | - | - | Dimethyl sulfoxide | 50 | Ethanol | 20 | 26.63 | 18.7 | 16.0 | 0.86 | S |
| Example K3 | Absorbing liquid K3 | (A95) | 30 | - | - | Dimethyl sulfoxide | 50 | 1-propanol | 20 | 26.08 | 20.0 | 16.5 | 0.83 | S |
| Example K4 | Absorbing liquid K4 | (A95) | 30 | - | - | Dimethyl sulfoxide | 50 | 3-Methoxy-1-butanol | 20 | 25.10 | 21.2 | 16.9 | 0.80 | S |
| Example K5 | Absorbing liquid K5 | (A95) | 30 | - | - | Dimethyl sulfoxide | 50 | 1-Methoxy-2-propanol | 20 | 24.89 | 20.1 | 16.7 | 0.83 | S |
| Example K6 | Absorbing liquid K6 | (A95) | 30 | - | - | Dimethyl sulfoxide | 50 | Water | 20 | 32.71 | 20.2 | 17.0 | 0.84 | S |
| Example K7 | Absorbing liquid K7 | (A95) | 30 | - | - | Dimethyl sulfoxide | 50 | Sulfolane | 20 | 26.59 | 20.7 | 16.5 | 0.80 | S |
| Example K8 | Absorbing liquid K8 | (A95) | 30 | - | - | Dimethyl sulfoxide | 50 | Ethylene glycol monomethyl ether | 20 | 25.74 | 20.6 | 17.6 | 0.86 | S |
| Example K9 | Absorbing liquid K9 | (A95) | 30 | - | - | Methanol | 50 | Ethanol | 20 | 28.58 | 20.6 | 16.7 | 0.81 | S |
| Example K10 | Absorbing liquid K10 | (A95) | 30 | - | - | Methanol | 50 | 1-propanol | 20 | 28.03 | 20.3 | 16.2 | 0.80 | S |
| Example K11 | Absorbing liquid K11 | (A95) | 30 | - | - | Methanol | 50 | Water | 20 | 34.66 | 19.7 | 16.4 | 0.83 | S |
| Example K12 | Absorbing liquid K12 | (A95) | 30 | - | - | 1-propanol | 50 | 1-Methoxy-2-propanol | 20 | 23.41 | 19.5 | 16.2 | 0.83 | S |
| Example K13 | Absorbing liquid K13 | (A95) | 30 | - | - | 1-propanol | 50 | 3-Methoxy-1-butanol | 20 | 23.62 | 19.7 | 16.4 | 0.83 | S |
| Example K14 | Absorbing liquid K14 | (A95) | 30 | - | - | 1-propanol | 50 | Water | 20 | 31.23 | 20.0 | 16.1 | 0.80 | S |

[Table 13-4]

Table 13-4

| Example | Absorbing liquid | Composition of carbon dioxide absorbing liquid | | | | | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | dT [MPa1/2] of the entire liquid medium | | | | |
| Example K15 | Absorbing liquid K15 | (A95) | 30 | - | - | Ethylene glycol monomethyl ether | 50 | Tetraethylene glycol dibutyl ether | 20 | 21.81 | 20.6 | 16.0 | 0.78 | A |
| Example K16 | Absorbing liquid K16 | (A95) | 30 | - | - | Tetraethylene glycol dibutyl ether | 50 | Sulfolane | 20 | 20.25 | 19.3 | 15.1 | 0.78 | A |
| Example K17 | Absorbing liquid K17 | (A96) | 30 | - | - | Dimethyl sulfoxide | 50 | Methanol | 20 | 27.46 | 21.3 | 17.4 | 0.82 | S |
| Example K18 | Absorbing liquid K18 | (A96) | 30 | - | - | Dimethyl sulfoxide | 50 | Ethanol | 20 | 26.63 | 19.7 | 16.0 | 0.81 | S |
| Example K19 | Absorbing liquid K19 | (A96) | 30 | - | - | Dimethyl sulfoxide | 50 | 1-propanol | 20 | 26.08 | 19.6 | 17.3 | 0.88 | S |
| Example K20 | Absorbing liquid K20 | (A96) | 30 | - | - | Dimethyl sulfoxide | 50 | 3-Methoxy-1-butanol | 20 | 25.10 | 19.8 | 17.3 | 0.87 | S |
| Example K21 | Absorbing liquid K21 | (A96) | 30 | - | - | Dimethyl sulfoxide | 50 | 1-Methoxy-2-propanol | 20 | 24.89 | 21.0 | 17.4 | 0.83 | S |
| Example K22 | Absorbing liquid K22 | (A96) | 30 | - | - | Dimethyl sulfoxide | 50 | Water | 20 | 32.71 | 20.3 | 16.9 | 0.83 | S |
| Example K23 | Absorbing liquid K23 | (A96) | 30 | - | - | Dimethyl sulfoxide | 50 | Sulfolane | 20 | 26.59 | 19.9 | 16.5 | 0.83 | S |
| Example K24 | Absorbing liquid K24 | (A96) | 30 | - | - | Dimethyl sulfoxide | 50 | Ethylene glycol monomethyl ether | 20 | 25.74 | 19.7 | 16.5 | 0.84 | S |
| Example K25 | Absorbing liquid K25 | (A96) | 30 | - | - | Methanol | 50 | Ethanol | 20 | 28.58 | 19.8 | 16.2 | 0.82 | S |
| Example K26 | Absorbing liquid K26 | (A96) | 30 | - | - | Methanol | 50 | 1-propanol | 20 | 28.03 | 20.4 | 16.2 | 0.80 | S |
| Example K27 | Absorbing liquid K27 | (A96) | 30 | - | - | Methanol | 50 | Water | 20 | 34.66 | 20.1 | 16.1 | 0.80 | S |
| Example K28 | Absorbing liquid K28 | (A96) | 30 | - | - | 1-propanol | 50 | 1-Methoxy-2-propanol | 20 | 23.41 | 20.5 | 17.0 | 0.83 | S |
| Example K29 | Absorbing liquid K29 | (A96) | 30 | - | - | 1-propanol | 50 | 3-Methoxy-1-butanol | 20 | 23.62 | 19.8 | 16.9 | 0.85 | S |
| Example K30 | Absorbing liquid K30 | (A96) | 30 | - | - | 1-propanol | 50 | Water | 20 | 31.23 | 20.8 | 17.3 | 0.83 | S |
| Example K31 | Absorbing liquid K31 | (A96) | 30 | - | - | Ethylene glycol monomethyl ether | 50 | Tetraethylene glycol dibutyl ether | 20 | 21.81 | 19.7 | 15.5 | 0.79 | A |
| Example K32 | Absorbing liquid K32 | (A96) | 30 | - | - | Tetraethylene glycol dibutyl ether | 50 | Sulfolane | 20 | 20.25 | 20.0 | 15.6 | 0.78 | A |
| Example K33 | Absorbing liquid K33 | (A104) | 30 | - | - | Dimethyl sulfoxide | 50 | Methanol | 20 | 27.46 | 19.9 | 16.7 | 0.84 | S |
| Example K34 | Absorbing liquid K34 | (A104) | 30 | - | - | Dimethyl sulfoxide | 50 | Ethanol | 20 | 26.63 | 20.9 | 17.6 | 0.84 | S |
| Example K35 | Absorbing liquid K35 | (A104) | 30 | - | - | Dimethyl sulfoxide | 50 | 1-propanol | 20 | 26.08 | 19.1 | 16.0 | 0.84 | S |
| Example K36 | Absorbing liquid K36 | (A104) | 30 | - | - | Dimethyl sulfoxide | 50 | 3-Methoxy-1-butanol | 20 | 25.10 | 19.9 | 16.1 | 0.81 | S |
| Example K37 | Absorbing liquid K37 | (A104) | 30 | - | - | Dimethyl sulfoxide | 50 | 1-Methoxy-2-propanol | 20 | 24.89 | 19.4 | 16.1 | 0.83 | S |
| Example K38 | Absorbing liquid K38 | (A104) | 30 | - | - | Dimethyl sulfoxide | 50 | Water | 20 | 32.71 | 20.5 | 17.4 | 0.85 | S |
| Example K39 | Absorbing liquid K39 | (A104) | 30 | - | - | Dimethyl sulfoxide | 50 | Sulfolane | 20 | 26.59 | 20.3 | 17.1 | 0.84 | S |
| Example K40 | Absorbing liquid K40 | (A104) | 30 | - | - | Dimethyl sulfoxide | 50 | Ethylene glycol monomethyl ether | 20 | 25.74 | 20.7 | 17.1 | 0.83 | S |
| Example K41 | Absorbing liquid K41 | (A104) | 30 | - | - | Methanol | 50 | Ethanol | 20 | 28.58 | 19.1 | 16.1 | 0.84 | S |
| Example K42 | Absorbing liquid K42 | (A104) | 30 | - | - | Methanol | 50 | 1-propanol | 20 | 28.03 | 20.0 | 17.5 | 0.87 | S |
| Example K43 | Absorbing liquid K43 | (A104) | 30 | - | - | Methanol | 50 | Water | 20 | 34.66 | 19.8 | 16.6 | 0.84 | S |
| Example K44 | Absorbing liquid K44 | (A104) | 30 | - | - | 1-propanol | 50 | 1-Methoxy-2-propanol | 20 | 23.41 | 19.2 | 16.2 | 0.84 | S |
| Example K45 | Absorbing liquid K45 | (A104) | 30 | - | - | 1-propanol | 50 | 3-Methoxy-1-butanol | 20 | 23.62 | 20.8 | 16.7 | 0.80 | S |
| Example K46 | Absorbing liquid K46 | (A104) | 30 | - | - | 1-propanol | 50 | Water | 20 | 31.23 | 20.6 | 17.4 | 0.84 | S |
| Example K47 | Absorbing liquid K47 | (A104) | 30 | - | - | Ethylene glycol monomethyl ether | 50 | Tetraethylene glycol dibutyl ether | 20 | 21.81 | 19.7 | 15.5 | 0.79 | A |
| Example K48 | Absorbing liquid K48 | (A104) | 30 | - | - | Tetraethylene glycol dibutyl ether | 50 | Sulfolane | 20 | 20.25 | 19.4 | 14.4 | 0.74 | A |

[Table 13-5]

Table 13-5

| Example | Absorbing liquid | Composition of carbon dioxide absorbing liquid | | | | | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | dT [MPa1/2] of the entire liquid medium | | | | |
| Example K49 | Absorbing liquid K49 | (A105) | 30 | - | - | Dimethyl sulfoxide | 50 | Methanol | 20 | 27.46 | 20.8 | 17.5 | 0.84 | S |
| Example K50 | Absorbing liquid K50 | (A105) | 30 | - | - | Dimethyl sulfoxide | 50 | Ethanol | 20 | 26.63 | 19.7 | 16.1 | 0.82 | S |
| Example K51 | Absorbing liquid K51 | (A105) | 30 | - | - | Dimethyl sulfoxide | 50 | 1-propanol | 20 | 26.08 | 19.6 | 16.1 | 0.82 | S |
| Example K52 | Absorbing liquid K52 | (A105) | 30 | - | - | Dimethyl sulfoxide | 50 | 3-Methoxy-1-butanol | 20 | 25.10 | 20.6 | 16.9 | 0.82 | S |
| Example K53 | Absorbing liquid K53 | (A105) | 30 | - | - | Dimethyl sulfoxide | 50 | 1-Methoxy-2-propanol | 20 | 24.89 | 18.9 | 15.9 | 0.84 | S |
| Example K54 | Absorbing liquid K54 | (A105) | 30 | - | - | Dimethyl sulfoxide | 50 | Water | 20 | 32.71 | 19.8 | 16.0 | 0.81 | S |
| Example K55 | Absorbing liquid K55 | (A105) | 30 | - | - | Dimethyl sulfoxide | 50 | Sulfolane | 20 | 26.59 | 19.6 | 16.4 | 0.84 | S |
| Example K56 | Absorbing liquid K56 | (A105) | 30 | - | - | Dimethyl sulfoxide | 50 | Ethylene glycol monomethyl ether | 20 | 25.74 | 19.7 | 16.6 | 0.85 | S |
| Example K57 | Absorbing liquid K57 | (A105) | 30 | - | - | Methanol | 50 | Ethanol | 20 | 28.58 | 20.3 | 17.2 | 0.84 | S |
| Example K58 | Absorbing liquid K58 | (A105) | 30 | - | - | Methanol | 50 | 1-propanol | 20 | 28.03 | 20.7 | 17.1 | 0.83 | S |
| Example K59 | Absorbing liquid K59 | (A105) | 30 | - | - | Methanol | 50 | Water | 20 | 34.66 | 20.5 | 16.7 | 0.82 | S |
| Example K60 | Absorbing liquid K60 | (A105) | 30 | - | - | 1-propanol | 50 | 1-Methoxy-2-propanol | 20 | 23.41 | 19.7 | 16.9 | 0.86 | S |
| Example K61 | Absorbing liquid K61 | (A105) | 30 | - | - | 1-propanol | 50 | 3-Methoxy-1-butanol | 20 | 23.62 | 20.1 | 17.1 | 0.85 | S |
| Example K62 | Absorbing liquid K62 | (A105) | 30 | - | - | 1-propanol | 50 | Water | 20 | 31.23 | 19.6 | 16.5 | 0.84 | S |
| Example K63 | Absorbing liquid K63 | (A105) | 30 | - | - | Ethylene glycol monomethyl ether | 50 | Tetraethylene glycol dibutyl ether | 20 | 21.81 | 19.0 | 14.1 | 0.74 | A |
| Example K64 | Absorbing liquid K64 | (A105) | 30 | - | - | Tetraethylene glycol dibutyl ether | 50 | Sulfolane | 20 | 20.25 | 17.3 | 13.3 | 0.77 | A |
| Example K65 | Absorbing liquid K65 | (A115) | 30 | - | - | Dimethyl sulfoxide | 50 | Methanol | 20 | 27.46 | 19.5 | 16.9 | 0.87 | S |
| Example K66 | Absorbing liquid K66 | (A115) | 30 | - | - | Dimethyl sulfoxide | 50 | Ethanol | 20 | 26.63 | 18.0 | 15.8 | 0.88 | S |
| Example K67 | Absorbing liquid K67 | (A115) | 30 | - | - | Dimethyl sulfoxide | 50 | 1-propanol | 20 | 26.08 | 18.0 | 14.8 | 0.82 | S |
| Example K68 | Absorbing liquid K68 | (A115) | 30 | - | - | Dimethyl sulfoxide | 50 | 3-Methoxy-1-butanol | 20 | 25.10 | 17.7 | 14.6 | 0.82 | S |
| Example K69 | Absorbing liquid K69 | (A115) | 30 | - | - | Dimethyl sulfoxide | 50 | 1-Methoxy-2-propanol | 20 | 24.89 | 18.9 | 16.4 | 0.87 | S |
| Example K70 | Absorbing liquid K70 | (A115) | 30 | - | - | Dimethyl sulfoxide | 50 | Water | 20 | 32.71 | 20.9 | 17.1 | 0.82 | S |
| Example K71 | Absorbing liquid K71 | (A115) | 30 | - | - | Dimethyl sulfoxide | 50 | Sulfolane | 20 | 26.59 | 17.9 | 15.5 | 0.86 | S |
| Example K72 | Absorbing liquid K72 | (A115) | 30 | - | - | Dimethyl sulfoxide | 50 | Ethylene glycol monomethyl ether | 20 | 25.74 | 18.1 | 15.6 | 0.86 | S |
| Example K73 | Absorbing liquid K73 | (A115) | 30 | - | - | Methanol | 50 | Ethanol | 20 | 28.58 | 20.2 | 17.5 | 0.86 | S |
| Example K74 | Absorbing liquid K74 | (A115) | 30 | - | - | Methanol | 50 | 1-propanol | 20 | 28.03 | 21.4 | 18.8 | 0.88 | S |
| Example K75 | Absorbing liquid K75 | (A115) | 30 | - | - | Methanol | 50 | Water | 20 | 34.66 | 19.1 | 16.3 | 0.86 | S |
| Example K76 | Absorbing liquid K76 | (A115) | 30 | - | - | 1-propanol | 50 | 1-Methoxy-2-propanol | 20 | 23.41 | 19.5 | 16.7 | 0.85 | S |
| Example K77 | Absorbing liquid K77 | (A115) | 30 | - | - | 1-propanol | 50 | 3-Methoxy-1-butanol | 20 | 23.62 | 19.7 | 16.7 | 0.85 | S |
| Example K78 | Absorbing liquid K78 | (A115) | 30 | - | - | 1-propanol | 50 | Water | 20 | 31.23 | 19.0 | 16.1 | 0.85 | S |
| Example K79 | Absorbing liquid K79 | (A115) | 30 | - | - | Ethylene glycol monomethyl ether | 50 | Tetraethylene glycol dibutyl ether | 20 | 21.81 | 19.7 | 14.7 | 0.75 | A |
| Example K80 | Absorbing liquid K80 | (A115) | 30 | - | - | Tetraethylene glycol dibutyl ether | 50 | Sulfolane | 20 | 20.25 | 20.6 | 14.6 | 0.71 | A |

[Table 13-6]

Table 13-6

| Example | Absorbing liquid | Composition of carbon dioxide absorbing liquid | | | | | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | dT [MPa1/2] of the entire liquid medium | | | | |
| Example K81 | Absorbing liquid K81 | (A116) | 30 | - | - | Dimethyl sulfoxide | 50 | Methanol | 20 | 27.46 | 19.0 | 15.5 | 0.82 | S |
| Example K82 | Absorbing liquid K82 | (A116) | 30 | - | - | Dimethyl sulfoxide | 50 | Ethanol | 20 | 26.63 | 17.9 | 15.1 | 0.84 | S |
| Example K83 | Absorbing liquid K83 | (A116) | 30 | - | - | Dimethyl sulfoxide | 50 | 1-propanol | 20 | 26.08 | 18.0 | 14.9 | 0.83 | S |
| Example K84 | Absorbing liquid K84 | (A116) | 30 | - | - | Dimethyl sulfoxide | 50 | 3-Methoxy-1-butanol | 20 | 25.10 | 18.5 | 15.5 | 0.84 | S |
| Example K85 | Absorbing liquid K85 | (A116) | 30 | - | - | Dimethyl sulfoxide | 50 | 1-Methoxy-2-propanol | 20 | 24.89 | 17.3 | 13.9 | 0.80 | S |
| Example K86 | Absorbing liquid K86 | (A116) | 30 | - | - | Dimethyl sulfoxide | 50 | Water | 20 | 32.71 | 18.2 | 15.0 | 0.82 | S |
| Example K87 | Absorbing liquid K87 | (A116) | 30 | - | - | Dimethyl sulfoxide | 50 | Sulfolane | 20 | 26.59 | 18.1 | 15.5 | 0.86 | S |
| Example K88 | Absorbing liquid K88 | (A116) | 30 | - | - | Dimethyl sulfoxide | 50 | Ethylene glycol monomethyl ether | 20 | 25.74 | 17.4 | 15.0 | 0.86 | S |
| Example K89 | Absorbing liquid K89 | (A116) | 30 | - | - | Methanol | 50 | Ethanol | 20 | 28.58 | 19.3 | 16.0 | 0.83 | S |
| Example K90 | Absorbing liquid K90 | (A116) | 30 | - | - | Methanol | 50 | 1-propanol | 20 | 28.03 | 19.3 | 16.3 | 0.85 | S |
| Example K91 | Absorbing liquid K91 | (A116) | 30 | - | - | Methanol | 50 | Water | 20 | 34.66 | 18.2 | 16.0 | 0.88 | S |
| Example K92 | Absorbing liquid K92 | (A116) | 30 | - | - | 1-propanol | 50 | 1-Methoxy-2-propanol | 20 | 23.41 | 20.1 | 17.6 | 0.88 | S |
| Example K93 | Absorbing liquid K93 | (A116) | 30 | - | - | 1-propanol | 50 | 3-Methoxy-1-butanol | 20 | 23.62 | 19.3 | 15.7 | 0.82 | S |
| Example K94 | Absorbing liquid K94 | (A116) | 30 | - | - | 1-propanol | 50 | Water | 20 | 31.23 | 19.9 | 16.6 | 0.83 | S |
| Example K95 | Absorbing liquid K95 | (A116) | 30 | - | - | Ethylene glycol monomethyl ether | 50 | Tetraethylene glycol dibutyl ether | 20 | 21.81 | 19.9 | 14.2 | 0.72 | A |
| Example K96 | Absorbing liquid K96 | (A116) | 30 | - | - | Tetraethylene glycol dibutyl ether | 50 | Sulfolane | 20 | 20.25 | 20.4 | 15.6 | 0.76 | A |
| Example K97 | Absorbing liquid K97 | (A119) | 30 | - | - | Dimethyl sulfoxide | 50 | Methanol | 20 | 27.46 | 20.5 | 17.9 | 0.87 | S |
| Example K98 | Absorbing liquid K98 | (A119) | 30 | - | - | Dimethyl sulfoxide | 50 | Ethanol | 20 | 26.63 | 18.2 | 14.8 | 0.81 | S |
| Example K99 | Absorbing liquid K99 | (A119) | 30 | - | - | Dimethyl sulfoxide | 50 | 1-propanol | 20 | 26.08 | 18.0 | 14.6 | 0.81 | S |
| Example K100 | Absorbing liquid K100 | (A119) | 30 | - | - | Dimethyl sulfoxide | 50 | 3-Methoxy-1-butanol | 20 | 25.10 | 18.7 | 16.3 | 0.87 | S |
| Example K101 | Absorbing liquid K101 | (A119) | 30 | - | - | Dimethyl sulfoxide | 50 | 1-Methoxy-2-propanol | 20 | 24.89 | 18.2 | 15.6 | 0.86 | S |
| Example K102 | Absorbing liquid K102 | (A119) | 30 | - | - | Dimethyl sulfoxide | 50 | Water | 20 | 32.71 | 17.7 | 14.9 | 0.84 | S |
| Example K103 | Absorbing liquid K103 | (A119) | 30 | - | - | Dimethyl sulfoxide | 50 | Sulfolane | 20 | 26.59 | 17.3 | 15.0 | 0.87 | S |
| Example K104 | Absorbing liquid K104 | (A119) | 30 | - | - | Dimethyl sulfoxide | 50 | Ethylene glycol monomethyl ether | 20 | 25.74 | 19.7 | 15.9 | 0.81 | S |
| Example K105 | Absorbing liquid K105 | (A119) | 30 | - | - | Methanol | 50 | Ethanol | 20 | 28.58 | 19.9 | 16.3 | 0.82 | S |
| Example K106 | Absorbing liquid K106 | (A119) | 30 | - | - | Methanol | 50 | 1-propanol | 20 | 28.03 | 19.1 | 15.5 | 0.81 | S |
| Example K107 | Absorbing liquid K107 | (A119) | 30 | - | - | Methanol | 50 | Water | 20 | 34.66 | 17.9 | 14.4 | 0.80 | S |
| Example K108 | Absorbing liquid K108 | (A119) | 30 | - | - | 1-propanol | 50 | 1-Methoxy-2-propanol | 20 | 23.41 | 18.9 | 16.5 | 0.87 | S |
| Example K109 | Absorbing liquid K109 | (A119) | 30 | - | - | 1-propanol | 50 | 3-Methoxy-1-butanol | 20 | 23.62 | 19.0 | 15.3 | 0.81 | S |
| Example K110 | Absorbing liquid K110 | (A119) | 30 | - | - | 1-propanol | 50 | Water | 20 | 31.23 | 20.2 | 17.2 | 0.85 | S |
| Example K111 | Absorbing liquid K111 | (A119) | 30 | - | - | Ethylene glycol monomethyl ether | 50 | Tetraethylene glycol dibutyl ether | 20 | 21.81 | 18.1 | 13.2 | 0.73 | A |
| Example K112 | Absorbing liquid K112 | (A119) | 30 | - | - | Tetraethylene glycol dibutyl ether | 50 | Sulfolane | 20 | 20.25 | 17.9 | 13.8 | 0.77 | A |

[Table 13-7]

Table 13-7

| Example | Absorbing liquid | Composition of carbon dioxide absorbing liquid | | | | | | | | | | Absorption amount per 1 kg of carbon dioxide absorbing liquid for 1 hour [L] | Release amount per 1 kg of carbon dioxide absorbing liquid for 2 hours [L] | Release efficiency | Evaluation |
|---|---|---|---|---|---|---|---|---|---|---|---|---|---|---|---|
| | | Amine compound 1 | Amine compound 1 /wt% | Amine compound 2 | Amine compound 2 /wt% | Liquid medium 1 | Liquid medium 1/wt% | Liquid medium 2 | Liquid medium 2/wt% | dT [MPa1/2] of the entire liquid medium | | | | | |
| Example K113 | Absorbing liquid K113 | (A120) | 30 | - | - | Dimethyl sulfoxide | 50 | Methanol | 20 | 27.46 | 19.7 | 17.3 | 0.88 | S |
| Example K114 | Absorbing liquid K114 | (A120) | 30 | - | - | Dimethyl sulfoxide | 50 | Ethanol | 20 | 26.63 | 18.0 | 15.3 | 0.85 | S |
| Example K115 | Absorbing liquid K115 | (A120) | 30 | - | - | Dimethyl sulfoxide | 50 | 1-propanol | 20 | 26.08 | 17.4 | 14.1 | 0.81 | S |
| Example K116 | Absorbing liquid K116 | (A120) | 30 | - | - | Dimethyl sulfoxide | 50 | 3-Methoxy-1-butanol | 20 | 25.10 | 19.3 | 17.1 | 0.88 | S |
| Example K117 | Absorbing liquid K117 | (A120) | 30 | - | - | Dimethyl sulfoxide | 50 | 1-Methoxy-2-propanol | 20 | 24.89 | 19.2 | 16.5 | 0.86 | S |
| Example K118 | Absorbing liquid K118 | (A120) | 30 | - | - | Dimethyl sulfoxide | 50 | Water | 20 | 32.71 | 19.9 | 17.4 | 0.87 | S |
| Example K119 | Absorbing liquid K119 | (A120) | 30 | - | - | Dimethyl sulfoxide | 50 | Sulfolane | 20 | 26.59 | 17.9 | 14.7 | 0.82 | S |
| Example K120 | Absorbing liquid K120 | (A120) | 30 | - | - | Dimethyl sulfoxide | 50 | Ethylene glycol monomethyl ether | 20 | 25.74 | 18.9 | 15.5 | 0.82 | S |
| Example K121 | Absorbing liquid K121 | (A120) | 30 | - | - | Methanol | 50 | Ethanol | 20 | 28.58 | 18.9 | 15.5 | 0.82 | S |
| Example K122 | Absorbing liquid K122 | (A120) | 30 | - | - | Methanol | 50 | 1-propanol | 20 | 28.03 | 19.7 | 17.4 | 0.88 | S |
| Example K123 | Absorbing liquid K123 | (A120) | 30 | - | - | Methanol | 50 | Water | 20 | 34.66 | 20.1 | 16.6 | 0.83 | S |
| Example K124 | Absorbing liquid K124 | (A120) | 30 | - | - | 1-propanol | 50 | 1-Methoxy-2-propanol | 20 | 23.41 | 20.2 | 17.0 | 0.84 | S |
| Example K125 | Absorbing liquid K125 | (A120) | 30 | - | - | 1-propanol | 50 | 3-Methoxy-1-butanol | 20 | 23.62 | 19.2 | 16.3 | 0.85 | S |
| Example K126 | Absorbing liquid K126 | (A120) | 30 | - | - | 1-propanol | 50 | Water | 20 | 31.23 | 17.5 | 15.0 | 0.86 | S |
| Example K127 | Absorbing liquid K127 | (A120) | 30 | - | - | Ethylene glycol monomethyl ether | 50 | Tetraethylene glycol dibutyl ether | 20 | 21.81 | 18.2 | 13.7 | 0.75 | A |
| Example K128 | Absorbing liquid K128 | (A120) | 30 | - | - | Tetraethylene glycol dibutyl ether | 50 | Sulfolane | 20 | 20.25 | 18.8 | 13.5 | 0.72 | A |

[Examples G1 to G61, H1 to H202, L1 to L128]

(Degree of change in absorption amount after repeated absorption/release evaluation of carbon dioxide gas)

Using the absorbing liquids B1 to B61, C1 to C83, E21 to E35, K1 to K128, and G1 to G99 described in Table 12-1 to Table 12-7, the aforementioned carbon dioxide gas absorption/release measurement was repeated 10 times. Subsequently, the 11th absorption amount was calculated in the same manner as the first test, and the degree of decrease compared to the first absorption amount was evaluated. The evaluation criteria were as follows, with S, A, and B considered as the usable range. The evaluation results are shown in Table 14-1 to Table 14-4.

S: The 11th absorption amount is 99.5% or more compared to the first absorption amount
B: The 11th absorption amount is 99% or more and less than 99.5% compared to the first absorption amount
B: The 11th absorption amount is 98% or more and less than 99% compared to the first absorption amount
C: The 11th absorption amount is less than 98% compared to the first absorption amount

[Table 14-1]

Table 14-1

| Example | Absorbing liquid | Change in absorption amount in the 11th absorption/release test (compared to the 1st absorption amount) |
|---|---|---|
| Example G1 | Absorbing liquid B1 | S |
| Example G2 | Absorbing liquid B2 | A |
| Example G3 | Absorbing liquid B3 | S |
| Example G4 | Absorbing liquid B4 | S |
| Example G5 | Absorbing liquid B5 | S |
| Example G6 | Absorbing liquid B6 | S |
| Example G7 | Absorbing liquid B7 | S |
| Example G8 | Absorbing liquid B8 | S |
| Example G9 | Absorbing liquid B9 | S |
| Example G10 | Absorbing liquid B10 | S |
| Example G11 | Absorbing liquid B11 | B |
| Example G12 | Absorbing liquid B12 | S |
| Example G13 | Absorbing liquid B13 | S |
| Example G14 | Absorbing liquid B14 | S |
| Example G15 | Absorbing liquid B15 | S |
| Example G16 | Absorbing liquid B16 | S |
| Example G17 | Absorbing liquid B17 | S |
| Example G18 | Absorbing liquid B18 | S |
| Example G19 | Absorbing liquid B19 | S |
| Example G20 | Absorbing liquid B20 | S |
| Example G21 | Absorbing liquid B21 | B |
| Example G22 | Absorbing liquid B22 | S |
| Example G23 | Absorbing liquid B23 | B |
| Example G24 | Absorbing liquid B24 | A |
| Example G25 | Absorbing liquid B25 | A |
| Example G26 | Absorbing liquid B26 | A |
| Example G27 | Absorbing liquid B27 | A |
| Example G28 | Absorbing liquid B28 | A |
| Example G29 | Absorbing liquid B29 | A |
| Example G30 | Absorbing liquid B30 | A |

| Example | Absorbing liquid | Change in absorption amount in the 11th absorption/release test (compared to the 1st absorption amount) |
|---|---|---|
| Example G31 | Absorbing liquid B31 | B |
| Example G32 | Absorbing liquid B32 | S |
| Example G33 | Absorbing liquid B33 | A |
| Example G34 | Absorbing liquid B34 | A |
| Example G35 | Absorbing liquid B35 | A |
| Example G36 | Absorbing liquid B36 | A |
| Example G37 | Absorbing liquid B37 | A |
| Example G38 | Absorbing liquid B38 | A |
| Example G39 | Absorbing liquid B39 | A |
| Example G40 | Absorbing liquid B40 | S |
| Example G41 | Absorbing liquid B41 | B |
| Example G42 | Absorbing liquid B42 | S |
| Example G43 | Absorbing liquid B43 | A |
| Example G44 | Absorbing liquid B44 | A |
| Example G45 | Absorbing liquid B45 | A |
| Example G46 | Absorbing liquid B46 | A |
| Example G47 | Absorbing liquid B47 | A |
| Example G48 | Absorbing liquid B48 | A |
| Example G49 | Absorbing liquid B49 | A |
| Example G50 | Absorbing liquid B50 | A |
| Example G51 | Absorbing liquid B51 | A |
| Example G52 | Absorbing liquid B52 | A |
| Example G53 | Absorbing liquid B53 | A |
| Example G54 | Absorbing liquid B54 | A |
| Example G55 | Absorbing liquid B55 | S |
| Example G56 | Absorbing liquid B56 | S |
| Example G57 | Absorbing liquid B57 | S |
| Example G58 | Absorbing liquid B58 | S |
| Example G59 | Absorbing liquid B59 | S |
| Example G60 | Absorbing liquid B60 | S |
| Example G61 | Absorbing liquid B61 | S |

[Table 14-2]

Table 14-2

| Example | Absorbing liquid | Change in absorption amount in the 11th absorption/release test (compared to the 1st absorption amount) | Example | Absorbing liquid | Change in absorption amount in the 11th absorption/release test (compared to the 1st absorption amount) |
|---|---|---|---|---|---|
| Example H1 | Absorbing liquid E21 | S | Example H51 | Absorbing liquid C31 | S |
| Example H2 | Absorbing liquid E22 | S | Example H52 | Absorbing liquid C32 | S |
| Example H3 | Absorbing liquid E23 | S | Example H53 | Absorbing liquid C33 | A |
| Example H4 | Absorbing liquid E24 | A | Example H54 | Absorbing liquid C34 | A |
| Example H5 | Absorbing liquid E25 | A | Example H55 | Absorbing liquid C35 | A |
| Example H6 | Absorbing liquid E26 | S | Example H56 | Absorbing liquid C36 | S |
| Example H7 | Absorbing liquid E27 | S | Example H57 | Absorbing liquid C37 | S |
| Example H8 | Absorbing liquid E28 | S | Example H58 | Absorbing liquid C38 | S |
| Example H9 | Absorbing liquid E29 | A | Example H59 | Absorbing liquid C39 | S |
| Example H10 | Absorbing liquid E30 | A | Example H60 | Absorbing liquid C40 | S |
| Example H11 | Absorbing liquid E31 | A | Example H61 | Absorbing liquid C41 | S |
| Example H12 | Absorbing liquid E32 | A | Example H62 | Absorbing liquid C42 | A |
| Example H13 | Absorbing liquid E33 | A | Example H63 | Absorbing liquid C43 | A |
| Example H14 | Absorbing liquid E34 | A | Example H64 | Absorbing liquid C44 | S |
| Example H15 | Absorbing liquid E35 | A | Example H65 | Absorbing liquid C45 | S |
| Example H16 | Absorbing liquid E36 | S | Example H66 | Absorbing liquid C46 | S |
| Example H17 | Absorbing liquid E37 | S | Example H67 | Absorbing liquid C47 | S |
| Example H18 | Absorbing liquid E38 | S | Example H68 | Absorbing liquid C48 | S |
| Example H19 | Absorbing liquid E39 | A | Example H69 | Absorbing liquid C49 | A |
| Example H20 | Absorbing liquid E40 | A | Example H70 | Absorbing liquid C50 | A |
| Example H21 | Absorbing liquid C1 | S | Example H71 | Absorbing liquid C51 | A |
| Example H22 | Absorbing liquid C2 | S | Example H72 | Absorbing liquid C52 | S |
| Example H23 | Absorbing liquid C3 | S | Example H73 | Absorbing liquid C53 | S |
| Example H24 | Absorbing liquid C4 | S | Example H74 | Absorbing liquid C54 | S |
| Example H25 | Absorbing liquid C5 | S | Example H75 | Absorbing liquid C55 | S |
| Example H26 | Absorbing liquid C6 | S | Example H76 | Absorbing liquid C56 | S |
| Example H27 | Absorbing liquid C7 | S | Example H77 | Absorbing liquid C57 | S |
| Example H28 | Absorbing liquid C8 | S | Example H78 | Absorbing liquid C58 | A |
| Example H29 | Absorbing liquid C9 | S | Example H79 | Absorbing liquid C59 | A |
| Example H30 | Absorbing liquid C10 | S | Example H80 | Absorbing liquid C60 | S |
| Example H31 | Absorbing liquid C11 | S | Example H81 | Absorbing liquid C61 | S |
| Example H32 | Absorbing liquid C12 | S | Example H82 | Absorbing liquid C62 | S |
| Example H33 | Absorbing liquid C13 | S | Example H83 | Absorbing liquid C63 | S |
| Example H34 | Absorbing liquid C14 | S | Example H84 | Absorbing liquid C64 | S |
| Example H35 | Absorbing liquid C15 | S | Example H85 | Absorbing liquid C65 | A |
| Example H36 | Absorbing liquid C16 | S | Example H86 | Absorbing liquid C66 | A |
| Example H37 | Absorbing liquid C17 | S | Example H87 | Absorbing liquid C67 | A |
| Example H38 | Absorbing liquid C18 | S | Example H88 | Absorbing liquid C68 | S |
| Example H39 | Absorbing liquid C19 | S | Example H89 | Absorbing liquid C69 | S |
| Example H40 | Absorbing liquid C20 | S | Example H90 | Absorbing liquid C70 | S |
| Example H41 | Absorbing liquid C21 | S | Example H91 | Absorbing liquid C71 | S |
| Example H42 | Absorbing liquid C22 | S | Example H92 | Absorbing liquid C72 | S |
| Example H43 | Absorbing liquid C23 | S | Example H93 | Absorbing liquid C73 | S |
| Example H44 | Absorbing liquid C24 | S | Example H94 | Absorbing liquid C74 | A |
| Example H45 | Absorbing liquid C25 | S | Example H95 | Absorbing liquid C75 | A |
| Example H46 | Absorbing liquid C26 | A | Example H96 | Absorbing liquid C76 | S |
| Example H47 | Absorbing liquid C27 | A | Example H97 | Absorbing liquid C77 | S |
| Example H48 | Absorbing liquid C28 | S | Example H98 | Absorbing liquid C78 | S |
| Example H49 | Absorbing liquid C29 | S | Example H99 | Absorbing liquid C79 | S |
| Example H50 | Absorbing liquid C30 | S | Example H100 | Absorbing liquid C80 | S |

[Table 14-3]

Table 14-3

| Example | Absorbing liquid | Change in absorption amount in the 11th absorption/release test (compared to the 1st absorption amount) | Example | Absorbing liquid | Change in absorption amount in the 11th absorption/release test (compared to the 1st absorption amount) |
|---|---|---|---|---|---|
| Example H101 | Absorbing liquid C81 | A | Example H152 | Absorbing liquid G49 | A |
| Example H102 | Absorbing liquid C82 | A | Example H153 | Absorbing liquid G50 | A |
| Example H103 | Absorbing liquid C83 | A | Example H154 | Absorbing liquid G51 | A |
| Example H104 | Absorbing liquid G1 | B | Example H155 | Absorbing liquid G52 | A |
| Example H105 | Absorbing liquid G2 | B | Example H156 | Absorbing liquid G53 | A |
| Example H106 | Absorbing liquid G3 | B | Example H157 | Absorbing liquid G54 | A |
| Example H107 | Absorbing liquid G4 | B | Example H158 | Absorbing liquid G55 | A |
| Example H108 | Absorbing liquid G5 | A | Example H159 | Absorbing liquid G56 | A |
| Example H109 | Absorbing liquid G6 | A | Example H160 | Absorbing liquid G57 | A |
| Example H110 | Absorbing liquid G7 | B | Example H161 | Absorbing liquid G58 | A |
| Example H111 | Absorbing liquid G8 | A | Example H162 | Absorbing liquid G59 | A |
| Example H112 | Absorbing liquid G9 | A | Example H163 | Absorbing liquid G60 | A |
| Example H113 | Absorbing liquid G10 | A | Example H164 | Absorbing liquid G61 | A |
| Example H114 | Absorbing liquid G11 | A | Example H165 | Absorbing liquid G62 | A |
| Example H115 | Absorbing liquid G12 | A | Example H166 | Absorbing liquid G63 | A |
| Example H116 | Absorbing liquid G13 | A | Example H167 | Absorbing liquid G64 | A |
| Example H117 | Absorbing liquid G14 | A | Example H168 | Absorbing liquid G65 | A |
| Example H118 | Absorbing liquid G15 | A | Example H169 | Absorbing liquid G66 | A |
| Example H119 | Absorbing liquid G16 | A | Example H170 | Absorbing liquid G67 | A |
| Example H120 | Absorbing liquid G17 | A | Example H171 | Absorbing liquid G68 | A |
| Example H121 | Absorbing liquid G18 | A | Example H172 | Absorbing liquid G69 | A |
| Example H122 | Absorbing liquid G19 | A | Example H173 | Absorbing liquid G70 | A |
| Example H123 | Absorbing liquid G20 | A | Example H174 | Absorbing liquid G71 | A |
| Example H124 | Absorbing liquid G21 | A | Example H175 | Absorbing liquid G72 | A |
| Example H125 | Absorbing liquid G22 | A | Example H176 | Absorbing liquid G73 | A |
| Example H126 | Absorbing liquid G23 | A | Example H177 | Absorbing liquid G74 | A |
| Example H127 | Absorbing liquid G24 | A | Example H178 | Absorbing liquid G75 | S |
| Example H128 | Absorbing liquid G25 | A | Example H179 | Absorbing liquid G76 | S |
| Example H129 | Absorbing liquid G26 | A | Example H180 | Absorbing liquid G77 | A |
| Example H130 | Absorbing liquid G27 | A | Example H181 | Absorbing liquid G78 | A |
| Example H131 | Absorbing liquid G28 | A | Example H182 | Absorbing liquid G79 | S |
| Example H132 | Absorbing liquid G29 | A | Example H183 | Absorbing liquid G80 | S |
| Example H133 | Absorbing liquid G30 | A | Example H184 | Absorbing liquid G81 | S |
| Example H134 | Absorbing liquid G31 | A | Example H185 | Absorbing liquid G82 | S |
| Example H135 | Absorbing liquid G32 | A | Example H186 | Absorbing liquid G83 | S |
| Example H136 | Absorbing liquid G33 | A | Example H187 | Absorbing liquid G84 | A |
| Example H137 | Absorbing liquid G34 | A | Example H188 | Absorbing liquid G85 | S |
| Example H138 | Absorbing liquid G35 | A | Example H189 | Absorbing liquid G86 | S |
| Example H139 | Absorbing liquid G36 | A | Example H190 | Absorbing liquid G87 | S |
| Example H140 | Absorbing liquid G37 | A | Example H191 | Absorbing liquid G88 | S |
| Example H141 | Absorbing liquid G38 | A | Example H192 | Absorbing liquid G89 | S |
| Example H142 | Absorbing liquid G39 | A | Example H193 | Absorbing liquid G90 | S |
| Example H143 | Absorbing liquid G40 | A | Example H194 | Absorbing liquid G91 | A |
| Example H144 | Absorbing liquid G41 | A | Example H195 | Absorbing liquid G92 | S |
| Example H145 | Absorbing liquid G42 | A | Example H196 | Absorbing liquid G93 | S |
| Example H146 | Absorbing liquid G43 | A | Example H197 | Absorbing liquid G94 | S |
| Example H147 | Absorbing liquid G44 | A | Example H198 | Absorbing liquid G95 | S |
| Example H148 | Absorbing liquid G45 | A | Example H199 | Absorbing liquid G96 | S |
| Example H149 | Absorbing liquid G46 | A | Example H200 | Absorbing liquid G97 | S |
| Example H150 | Absorbing liquid G47 | A | Example H201 | Absorbing liquid G98 | S |
| Example H151 | Absorbing liquid G48 | A | Example H202 | Absorbing liquid G99 | B |

[Table 14-4]

Table 14-4

| Example | Absorbing liquid | Change in absorption amount in the 11th absorption/release test (compared to the 1st absorption amount) | Example | Absorbing liquid | Change in absorption amount in the 11th absorption/release test (compared to the 1st absorption amount) |
|---|---|---|---|---|---|
| Example L1 | Absorbing liquid K1 | S | Example L65 | Absorbing liquid K65 | S |
| Example L2 | Absorbing liquid K2 | S | Example L66 | Absorbing liquid K66 | S |
| Example L3 | Absorbing liquid K3 | S | Example L67 | Absorbing liquid K67 | S |
| Example L4 | Absorbing liquid K4 | S | Example L68 | Absorbing liquid K68 | S |
| Example L5 | Absorbing liquid K5 | S | Example L69 | Absorbing liquid K69 | S |
| Example L6 | Absorbing liquid K6 | S | Example L70 | Absorbing liquid K70 | S |
| Example L7 | Absorbing liquid K7 | A | Example L71 | Absorbing liquid K71 | A |
| Example L8 | Absorbing liquid K8 | A | Example L72 | Absorbing liquid K72 | A |
| Example L9 | Absorbing liquid K9 | S | Example L73 | Absorbing liquid K73 | S |
| Example L10 | Absorbing liquid K10 | S | Example L74 | Absorbing liquid K74 | S |
| Example L11 | Absorbing liquid K11 | S | Example L75 | Absorbing liquid K75 | S |
| Example L12 | Absorbing liquid K12 | S | Example L76 | Absorbing liquid K76 | S |
| Example L13 | Absorbing liquid K13 | S | Example L77 | Absorbing liquid K77 | S |
| Example L14 | Absorbing liquid K14 | A | Example L78 | Absorbing liquid K78 | A |
| Example L15 | Absorbing liquid K15 | A | Example L79 | Absorbing liquid K79 | A |
| Example L16 | Absorbing liquid K16 | A | Example L80 | Absorbing liquid K80 | A |
| Example L17 | Absorbing liquid K17 | S | Example L81 | Absorbing liquid K81 | S |
| Example L18 | Absorbing liquid K18 | S | Example L82 | Absorbing liquid K82 | S |
| Example L19 | Absorbing liquid K19 | S | Example L83 | Absorbing liquid K83 | S |
| Example L20 | Absorbing liquid K20 | S | Example L84 | Absorbing liquid K84 | S |
| Example L21 | Absorbing liquid K21 | S | Example L85 | Absorbing liquid K85 | S |
| Example L22 | Absorbing liquid K22 | S | Example L86 | Absorbing liquid K86 | S |
| Example L23 | Absorbing liquid K23 | A | Example L87 | Absorbing liquid K87 | A |
| Example L24 | Absorbing liquid K24 | A | Example L88 | Absorbing liquid K88 | A |
| Example L25 | Absorbing liquid K25 | S | Example L89 | Absorbing liquid K89 | S |
| Example L26 | Absorbing liquid K26 | S | Example L90 | Absorbing liquid K90 | S |
| Example L27 | Absorbing liquid K27 | S | Example L91 | Absorbing liquid K91 | S |
| Example L28 | Absorbing liquid K28 | S | Example L92 | Absorbing liquid K92 | S |
| Example L29 | Absorbing liquid K29 | S | Example L93 | Absorbing liquid K93 | S |
| Example L30 | Absorbing liquid K30 | A | Example L94 | Absorbing liquid K94 | A |
| Example L31 | Absorbing liquid K31 | A | Example L95 | Absorbing liquid K95 | A |
| Example L32 | Absorbing liquid K32 | A | Example L96 | Absorbing liquid K96 | A |
| Example L33 | Absorbing liquid K33 | S | Example L97 | Absorbing liquid K97 | S |
| Example L34 | Absorbing liquid K34 | S | Example L98 | Absorbing liquid K98 | S |
| Example L35 | Absorbing liquid K35 | S | Example L99 | Absorbing liquid K99 | S |
| Example L36 | Absorbing liquid K36 | S | Example L100 | Absorbing liquid K100 | S |
| Example L37 | Absorbing liquid K37 | S | Example L101 | Absorbing liquid K101 | S |
| Example L38 | Absorbing liquid K38 | S | Example L102 | Absorbing liquid K102 | S |
| Example L39 | Absorbing liquid K39 | A | Example L103 | Absorbing liquid K103 | A |
| Example L40 | Absorbing liquid K40 | A | Example L104 | Absorbing liquid K104 | A |
| Example L41 | Absorbing liquid K41 | S | Example L105 | Absorbing liquid K105 | S |
| Example L42 | Absorbing liquid K42 | S | Example L106 | Absorbing liquid K106 | S |
| Example L43 | Absorbing liquid K43 | S | Example L107 | Absorbing liquid K107 | S |
| Example L44 | Absorbing liquid K44 | S | Example L108 | Absorbing liquid K108 | S |
| Example L45 | Absorbing liquid K45 | S | Example L109 | Absorbing liquid K109 | S |
| Example L46 | Absorbing liquid K46 | A | Example L110 | Absorbing liquid K110 | A |
| Example L47 | Absorbing liquid K47 | A | Example L111 | Absorbing liquid K111 | A |
| Example L48 | Absorbing liquid K48 | A | Example L112 | Absorbing liquid K112 | A |
| Example L49 | Absorbing liquid K49 | S | Example L113 | Absorbing liquid K113 | S |
| Example L50 | Absorbing liquid K50 | S | Example L114 | Absorbing liquid K114 | S |
| Example L51 | Absorbing liquid K51 | S | Example L115 | Absorbing liquid K115 | S |
| Example L52 | Absorbing liquid K52 | S | Example L116 | Absorbing liquid K116 | S |
| Example L53 | Absorbing liquid K53 | S | Example L117 | Absorbing liquid K117 | S |
| Example L54 | Absorbing liquid K54 | S | Example L118 | Absorbing liquid K118 | S |
| Example L55 | Absorbing liquid K55 | A | Example L119 | Absorbing liquid K119 | A |
| Example L56 | Absorbing liquid K56 | A | Example L120 | Absorbing liquid K120 | A |
| Example L57 | Absorbing liquid K57 | S | Example L121 | Absorbing liquid K121 | S |
| Example L58 | Absorbing liquid K58 | S | Example L122 | Absorbing liquid K122 | S |
| Example L59 | Absorbing liquid K59 | S | Example L123 | Absorbing liquid K123 | S |
| Example L60 | Absorbing liquid K60 | S | Example L124 | Absorbing liquid K124 | S |
| Example L61 | Absorbing liquid K61 | S | Example L125 | Absorbing liquid K125 | S |
| Example L62 | Absorbing liquid K62 | A | Example L126 | Absorbing liquid K126 | A |
| Example L63 | Absorbing liquid K63 | A | Example L127 | Absorbing liquid K127 | A |
| Example L64 | Absorbing liquid K64 | A | Example L128 | Absorbing liquid K128 | A |

[Examples M79 to M122]

(Absorption rate of carbon dioxide gas)

The absorption rate evaluation was performed using absorbing liquids B1, B12, B22, B32, B43 to B54, J1, J110, J125, J141 to J144, J17, J32, J47, J63 to 79, and J95 described in Table 12-1 to Table 12-7. 100 g of the prepared carbon dioxide absorbing liquid (in a state placed in a 200 ml gas absorption bottle) was temperature-adjusted to 25°C in a water bath. A mixed gas (500 ml/min) of carbon dioxide gas at 100 ml/min and nitrogen gas at 400 ml/min was bubbled and blown into this carbon dioxide absorbing liquid. The absorption amount of carbon dioxide gas at this time (carbon dioxide absorption amount for 10 minutes (L)) was measured using a gas flow meter and a carbon dioxide concentration meter. The value obtained by dividing this absorption amount by 10 was used as the absorption rate (L/min).

The evaluation criteria were as follows, with S, A, and B considered as the usable range. The evaluation results are shown in Table 15. It is noted that the results of Comparative Example 5 and Reference Example 3 using absorbing liquids 125 to 126 described in the aforementioned Example Group 1 are also shown for comparison.

S: Absorption rate of 1.00 L/min or more
A: Absorption rate of 0.75 L/min or more and less than 1.00 L/min
B: Absorption rate of 0.50 L/min or more and less than 0.75 L/min
C: Absorption rate less than 0.50 L/min

[Table 15]

Table 15

| Example | Amine compound | Absorbing liquid | Absorption rate |
|---|---|---|---|
| Example M79 | (A79) | Absorbing liquid B43 | B |
| Example M80 | (A80) | Absorbing liquid B44 | S |
| Example M81 | (A81) | Absorbing liquid B45 | B |
| Example M82 | (A82) | Absorbing liquid B46 | B |
| Example M83 | (A83) | Absorbing liquid B47 | B |
| Example M84 | (A84) | Absorbing liquid B1 | B |
| Example M85 | (A85) | Absorbing liquid B12 | B |
| Example M86 | (A86) | Absorbing liquid B48 | B |
| Example M87 | (A87) | Absorbing liquid B49 | B |
| Example M88 | (A88) | Absorbing liquid B50 | B |
| Example M89 | (A89) | Absorbing liquid B51 | B |
| Example M90 | (A90) | Absorbing liquid B52 | B |
| Example M91 | (A91) | Absorbing liquid B22 | B |
| Example M92 | (A92) | Absorbing liquid B32 | B |
| Example M93 | (A93) | Absorbing liquid B53 | B |
| Example M94 | (A94) | Absorbing liquid B54 | B |
| Example M95 | (A95) | Absorbing liquid J1 | S |
| Example M96 | (A96) | Absorbing liquid J17 | A |
| Example M97 | (A97) | Absorbing liquid J63 | B |
| Example M98 | (A98) | Absorbing liquid J64 | B |
| Example M99 | (A99) | Absorbing liquid J65 | B |
| Example M100 | (A100) | Absorbing liquid J66 | S |
| Example M101 | (A101) | Absorbing liquid J67 | A |
| Example M102 | (A102) | Absorbing liquid J68 | B |
| Example M103 | (A103) | Absorbing liquid J69 | B |
| Example M104 | (A104) | Absorbing liquid J32 | S |
| Example M105 | (A105) | Absorbing liquid J47 | S |
| Example M106 | (A106) | Absorbing liquid J70 | A |
| Example M107 | (A107) | Absorbing liquid J71 | B |
| Example M108 | (A108) | Absorbing liquid J72 | B |
| Example M109 | (A109) | Absorbing liquid J73 | B |
| Example M110 | (A110) | Absorbing liquid J74 | A |
| Example M111 | (A111) | Absorbing liquid J75 | B |
| Example M112 | (A112) | Absorbing liquid J76 | B |
| Example M113 | (A113) | Absorbing liquid J77 | A |
| Example M114 | (A114) | Absorbing liquid J78 | A |
| Example M115 | (A115) | Absorbing liquid J141 | A |
| Example M116 | (A116) | Absorbing liquid J142 | B |
| Example M117 | (A117) | Absorbing liquid J79 | S |
| Example M118 | (A118) | Absorbing liquid J95 | S |
| Example M119 | (A119) | Absorbing liquid J143 | A |
| Example M120 | (A120) | Absorbing liquid J144 | B |
| Example M121 | (A121) | Absorbing liquid J110 | B |
| Example M122 | (A122) | Absorbing liquid J125 | B |
| Comparative Example 5 | MEA | Absorbing liquid 125 | C |
| Reference Example 3 | ATMP | Absorbing liquid 126 | C |

As described in the above examples, the carbon dioxide absorbing liquid of the present invention exhibits superior effects in terms of carbon dioxide release rate and release efficiency (release amount/absorption amount) compared to conventional carbon carbon dioxide absorbing liquids. Moreover, in contrast to the generally known release temperature of 120°C for MEA aqueous solution, it was found that the absorbing liquid of the disclosure may efficiently release carbon dioxide at a release temperature of 60°C under the experimental conditions used in this study.

This application claims priority based on Japanese Patent Application No. 2022-175922 filed on November 2, 2022, Japanese Patent Application No. 2023-393 filed on January 5, 2023, and Japanese Patent Application No. 2023-91519 filed on June 2, 2023, the entire present inventions of which are incorporated herein by reference.

## Claims

1. An absorbing liquid for separating and recovering carbon dioxide from a gas containing carbon dioxide, the absorbing liquid comprising:

   an amine compound (A) represented by general formula (1) and a liquid medium (B),

   [Chemical Formula 1]

   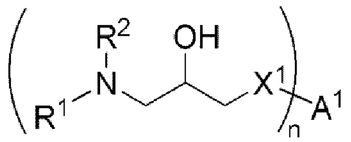
   (1)

   wherein:

   $R^1$ is a hydrogen atom or a hydrocarbon group which may have a substituent and may have a heteroatom in the carbon chain, and a carbon atom adjacent to N is a primary carbon atom or a carbon atom constituting a ring,
   $R^2$ is a hydrogen atom, an alkyl group having 1 to 8 carbon atoms in which a carbon atom adjacent to N is a primary carbon atom, or $-CH_2CH(OH)CH_2X^2A^2$,
   $X^1$ is a direct bonding, -O-, -OC(=O)-, -CO(=O)-, or $-NA^3-$,
   $A^1$ is a hydrogen atom or an n-valent organic residue (excluding the case where $X^1$ is a direct bonding and $A^1$ is a hydrogen atom),
   n is an integer of 1 to 6,
   $X^2$ is a direct bonding, -O-, -OC(=O)-, -CO(=O)-, or $-NA^3-$,
   $A^2$ is a hydrogen atom or a monovalent organic residue (excluding the case where $X^2$ is a direct bonding and $A^2$ is a hydrogen atom), and
   $A^3$ is a hydrogen atom or a monovalent organic residue.

2. The absorbing liquid according to claim 1, wherein the $R^1$ contains a nitrogen atom.

3. The absorbing liquid according to claim 1, wherein the $R^1$ is a group expressed by general formula (2), general formula (3), general formula (4), or general formula (5).

   [Chemical Formula 2]

   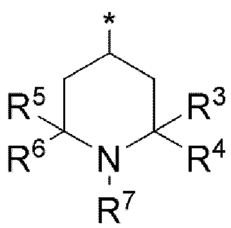
   (2)

   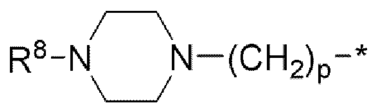
   (3)

   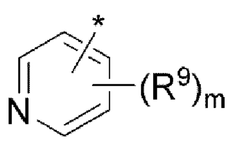
   (4)

   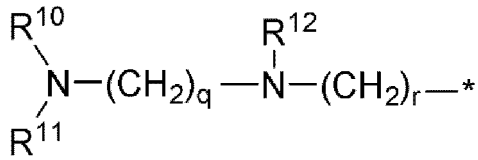
   (5)

   wherein:

   $R^3$, $R^4$, $R^5$, and $R^6$ are each independently a hydrogen atom or a methyl group,
   $R^7$ is a hydrogen atom or a methyl group,
   $R^8$ is a hydrogen atom or a methyl group,
   p is an integer of 0 to 4,

$R^9$ is an alkyl group having 1 to 8 carbon atoms,
m is an integer of 0 to 4,
$R^{10}$ and $R^{12}$ are each independently a hydrogen atom, an alkyl group having 1 to 8 carbon atoms, or a hydroxyalkyl group,
$R^{11}$ is a hydrogen atom, an alkyl group having 1 to 8 carbon atoms, a hydroxyalkyl group, or $-(CH_2)_s-R^{13}$,
$R^{13}$ is a hydroxyl group or $-N(R^{14})R^{15}$, and $R^{14}$ and $R^{15}$ are each independently a hydrogen atom, a methyl group, or a hydroxyalkyl group,
q is 2 or 3,
r is 2 or 3, and
s is 2 or 3.

**4.** The absorbing liquid according to claim 1, wherein $A^1$ is an n-valent organic residue.

**5.** The absorbing liquid according to claim 1, wherein $A^1$, $A^2$, and $A^3$ are each independently a straight-chain or branched aliphatic hydrocarbon residue, which may have a substituent and may have a heteroatom in the carbon chain; a (meth) acryloyl residue which may have a substituent; an alicyclic hydrocarbon residue which may have a substituent and may have a heteroatom in the carbon chain; an aromatic hydrocarbon residue which may have a substituent; or an aromatic heterocyclic residue which may have a substituent.

**6.** The absorbing liquid according to claim 1, wherein $R^2$ is a hydrogen atom.

**7.** The absorbing liquid according to claim 1, further comprising at least one amine compound (C) selected from a group consisting of amino alcohols, cyclic polyamines, and chain polyamines.

**8.** The absorbing liquid according to claim 1, wherein the liquid medium (B) has a total Hansen solubility parameter ($\delta T$) of 17 $MPa^{1/2}$ or more.

**9.** The absorbing liquid according to claim 1, wherein a proportion of water in the liquid medium (B) is 50% by mass or less.

**10.** The absorbing liquid according to claim 1, comprising 5% by mass or more of the amine compound (A) represented by the formula (1).

**11.** The absorbing liquid according to claim 1, wherein the gas further comprises hydrogen sulfide, and the absorbing liquid absorbs the hydrogen sulfide.

**12.** A method for separating and recovering carbon dioxide from a gas containing carbon dioxide, the method comprising:

process A: bringing the absorbing liquid according to any one of claims 1 to 11 into contact with a gas containing carbon dioxide to obtain an absorbing liquid that has absorbed carbon dioxide from the gas containing carbon dioxide, and
process B: heating the absorbing liquid that has absorbed carbon dioxide obtained in process A to desorb and release carbon dioxide from the absorbing liquid and recovering the released carbon dioxide.

**13.** The method according to claim 12, wherein a heating temperature in the process B is 50°C or higher and 160°C or lower.

## INTERNATIONAL SEARCH REPORT

International application No. **PCT/JP2023/039696**

**A. CLASSIFICATION OF SUBJECT MATTER**

***B01D 53/14***(2006.01)i; ***B01D 53/52***(2006.01)i; ***B01D 53/62***(2006.01)i; ***B01D 53/78***(2006.01)i; ***C01B 32/50***(2017.01)i; ***C07C 217/28***(2006.01)i; ***C07D 211/58***(2006.01)i; ***C07D 401/12***(2006.01)i; ***C07D 405/12***(2006.01)i
FI: B01D53/14 210; B01D53/14 220; B01D53/52 220; B01D53/62 ZAB; B01D53/78; C01B32/50; C07C217/28; C07D211/58; C07D401/12; C07D405/12

According to International Patent Classification (IPC) or to both national classification and IPC

**B. FIELDS SEARCHED**

Minimum documentation searched (classification system followed by classification symbols)

B01D53/14-18; B01D53/34-85; B01D53/96; C01B32/50; C07C217/28; C07D211/58; C07D401/12; C07D405/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2013-13854 A (ASAHI KASEI CORP) 24 January 2013 (2013-01-24) claims, paragraphs [0024]-[0028], [0036]-[0039], examples | 1-2, 4-10, 12-13 |
| Y | claims, paragraphs [0024]-[0028], [0036]-[0039], examples | 11 |
| X | JP 2021-194567 A (TOSOH CORP) 27 December 2021 (2021-12-27) claims, paragraphs [0034]-[0038], [0053]-[0055] | 1, 4-10, 12-13 |
| Y | claims, paragraphs [0034]-[0038], [0053]-[0055] | 11 |
| X | JP 2020-507463 A (BASF SE) 12 March 2020 (2020-03-12) claims, paragraphs [0012]-[0032], [0046], [0058], [0067] | 1, 4-13 |
| X | JP 2011-525422 A (BASF SE) 22 September 2011 (2011-09-22) claims, paragraphs [0031], [0035] | 1-2, 4-13 |
| Y | claims, paragraphs [0031], [0035] | 11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 January 2024** | **16 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2023/039696

C. DOCUMENTS CONSIDERED TO BE RELEVANT

| Category* | Citation of document, with indication, where appropriate, of the relevant passages | Relevant to claim No. |
|---|---|---|
| X | JP 2022-7403 A (TOSOH CORP) 13 January 2022 (2022-01-13) claims, paragraphs [0011]-[0031] | 1-10, 12-13 |
| Y | claims, paragraphs [0011]-[0031] | 11 |

Form PCT/ISA/210 (second sheet) (July 2022)

**INTERNATIONAL SEARCH REPORT**
**Information on patent family members**

International application No.
**PCT/JP2023/039696**

| Patent document cited in search report | Publication date (day/month/year) | Patent family member(s) | Publication date (day/month/year) |
|---|---|---|---|
| JP 2013-13854 A | 24 January 2013 | (Family: none) | |
| JP 2021-194567 A | 27 December 2021 | (Family: none) | |
| JP 2020-507463 A | 12 March 2020 | US 2019/0381448 A1<br>paragraphs [0012]-[0050], [0079], [0106], [0115], claims<br>WO 2018/146233 A1<br>CA 3052064 A1 | |
| JP 2011-525422 A | 22 September 2011 | US 2011/0135549 A1<br>paragraphs [0055], [0062], claims<br>WO 2009/156271 A1<br>CA 2726922 A1 | |
| JP 2022-7403 A | 13 January 2022 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H05301023 A
- JP 2009529420 A
- US 2006104877
- JP 2012223766 A
- JP 2012516761 A
- JP 2008247749 A
- JP 2022175922 A
- JP 2023000393 A
- JP 2023091519 A